# EUROPEAN PATENT SPECIFICATION

(11) **EP 2 403 853 B1**
(45) Date of publication and mention of the grant of the patent: **14.08.2013**
(21) Application number: 10707338.9
(22) Date of filing: 04.03.2010
(51) Int. Cl.: C07D 487/04, A61K 31/519, A61P 19/00, A61P 19/02, A61P 25/28, A61P 27/06, A61P 29/00, A61P 35/00

(54) **PYRROLOPYRIMIDINES USED AS KINASE INHIBITORS**
ALS KINASEINHIBITOREN VERWENDETE PYRROLOPYRIMIDINE
PYRROLOPYRIMIDINES UTILISÉES EN TANT QU'INHIBITEURS DE KINASES

(30) Priority: 04.03.2009 GB 0903759; 20.03.2009 US 162032 P
(43) Date of publication of application: 11.01.2012
(73) Proprietor: Medical Research Council Technology, London WC1H 9LT (GB)
(72) Inventor: MCIVER, Edward, Giles, London NW7 1AD (GB); HOUGH, Joane, LondonNW7 1AD (GB)
(74) Representative: Clyde-Watson, Zöe
(86) International application number: PCT/GB2010/000394
(87) International publication number: WO 2010/100431

(56) References cited:
- EP-A1- 0 795 556
- WO-A1-2005/080393
- WO-A1-2005/107760
- WO-A2-2007/140222

## Description

The present invention relates to pyrrolopyrimidine compounds that are capable of inhibiting one or more kinases. The compounds find applications in the treatment of a variety of disorders, including cancer, septic shock, Primary open Angle Glaucoma (POAG), hyperplasia, rheumatoid arthritis, psoriasis, artherosclerosis, retinopathy, osteoarthritis, endometriosis, chronic inflammation, and/or neurodegenerative diseases such as Alzheimers disease.

### BACKGROUND TO THE INVENTION

Pyrrolopyrimidines and analogues thereof are already described as active ingredients, such as, for example, proline transporter inhibitors for the (treatment of cognitive impairment (WO 07/024789); protein kinase inhibitors for the treatment of cancer WO 05/080393) and (WO 07/140222). WO 2005/107760 and EP 0795556 also describe pyrrolopyrimidine compounds as protein kinase inhibitors.

It is amongst the objects of the present invention to provide compounds which display a high degree of activity and/or specificity to particular kinases and may therefore serve as drug candidates or as starting points for further derivatisation and kinase inhibition studies.

It is a further object of the present invention to provide compounds for potential use as drug candidates for treating cancer, septic shock, inflammatory disease, primary open angle glaucoma (POAG) and/or Alzheimer's disease.

It is a further object to provide compounds which display a significant inhibitory effect on TBK1.

### STATEMENT OF INVENTION

A first aspect of the invention relates to a compound of formula (I), or a pharmaceutically acceptable salt or ester thereof, 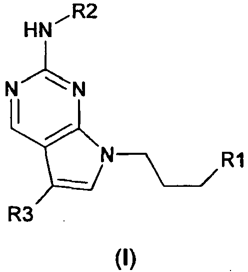 wherein:
R¹ is -NR⁷(CO)R¹¹;
R² is aryl, heteroaryl, fused aryl-C₃₋₆-heterocycloalkyl or fused heteroaryl-C₃₋₆-heterocycloalkyl, each of which is optionally substituted by one or more substitutents selected from aryl, heteroaryl, C₁₋₆-alkyl, C₃₋₆-heterocycloalkyl and a group A, wherein said C₁₋₆-alkyl group is in turn optionally substituted by one or more substituents selected from aryl, heteroaryl, C₃₋₆-heterocycloalkyl and a group A, said heteroaryl group is optionally substituted by one or more R¹⁰ groups; and wherein each C₃₋₆-heterocycloalkyl group is optionally substituted by one or more groups selected from C₁₋₆-alkyl, C₁₋₆-haloalkyl, and A, and optionally contains one or more groups selected from oxygen, sulphur, nitrogen and CO;
R³ is H, halogen, cyano or C₁₋₆-alkyl;
A is selected from halogen, hydroxyl, cyano, trifluoromethyl, alkoxy, -NO₂ -NH₂, -NR⁴R⁵ -OR⁶, -NR⁷(CO)R⁶, -NR⁷(CO)NR⁴R⁵, -NR⁷COOR⁷, -NR⁷(SO 2)R⁶, -CO₂H, -NR⁷(SO₂)NR⁴R⁵, -COOR⁷, -CONR⁴R⁵, COR⁶ and -SO₂CH₃;
each R⁴ and R⁵ is independently selected from hydrogen, C₃₋₇-cycloalkyl, aryl, heteroaryl, C₁₋₆-alkyl and a C₃₋₆-heterocycloalkyl ring optionally further containing one or more groups selected from oxygen, sulfur, nitrogen and CO, and optionally substituted by one or more R¹⁰ groups, wherein said C₁₋₆-alkyl is optionally substituted by one or more substituents selected from halogen, cyano, hydroxyl, aryl, heteroaryl, -NR⁸R⁹, - , NR⁷(CO)R⁶, -NR⁷COOR⁶, -NR⁷(SO₂)R⁶, -COOR⁶, -CONR⁸R⁹, OR¹⁰, -SO₂R⁶ and a C₃₋₆-heterocycloalkyl ring optionally further containing one or more groups selected from oxygen, sulfur, nitrogen and CO and optionally substituted by one or more or R¹⁰ groups; or
R⁴ and R⁵ together with the N to which they are attached form a C₃₋₆-heterocycloalkyl ring optionally further containing one or more groups selected from oxygen, sulfur, nitrogen and CO, wherein said C₃₋₅-heterocycloalkyl ring may be saturated or unsaturated and is optionally substituted with one or more groups selected from NR⁸R⁹ and R¹⁰;
each R⁶ is independently selected from C₁₋₅-alkyl, C₃₋₇ cycloalkyl, C₄₋₇heterocycloalkyl, aryl and heteroaryl, each of which may be optionally substituted by one or more substituents selected from halogen, R¹⁰ and -NR⁸R⁹;
each R⁷ is selected from hydrogen, C₁₋₆-alkyl and C₃₋₇-cycloalkyl, wherein said C₁₋₆-alkyl is optionally substituted by one or more halogens;
each of R⁸ and R⁹ is independently selected from hydrogen and C₁₋₆-alkyl, wherein said C₁₋₆-alkyl group is optionally substituted by one or more halogens; or
R⁸ and R⁹ together with the N to which they are attached form a C₄₋₆-heterocycloalkyl ring optionally further containing one or more heteroatoms selected from oxygen and sulfur, wherein said C₄₋₆-heterocycloalkyl ring is optionally substituted by one or more R¹⁰ groups; and
each R¹⁰ is selected from C₃₋₇-cycloalkyl and C₁₋₆-alkyl optionally substituted by one or more halogens, wherein where R¹⁰ is C₁₋₆-alkyl and two or more R¹⁰ groups are attached to the same carbon atom, the R¹⁰ groups may be linked to form a spiroalkyl group; and
each R¹¹ is independently selected from C₁₋₆-alkyl, C₃₋₇-cycloalkyl, C₁₋₆-alkyl-C₃₋₇-cycloalkyl, C₄₋₇-heterocycloalkyl, aryl and heteroaryl, each of which may be optionally - substituted by one or more substituents selected from A.

As is demonstrated in Examples section that follows, representative compounds of the present invention were tested for their kinase inhibition activity and showed significant potency to TBK1. These compounds therefore have applications in the treatment of diseases or disorders in which inhibiting the activity of TBK1 is beneficial.

A second aspect of the invention relates to a pharmaceutical composition comprising at least one compound as described above and a pharmaceutically acceptable carrier, diluent or excipient.

A third aspect of the invention relates to a compound as described above for use in medicine.

A fourth aspect of the invention relates to a compound as described above for treating a disorder selected from cancer, septic shock, Primary open Angle Glaucoma (POAG), hyperplasia, rheumatoid arthritis, psoriasis, artherosclerosis, retinopathy, osteoarthritis, endometriosis, chronic inflammation and Afzheimer's disease.

A fifth aspect of the invention relates to the use of a compound as described above in the preparation of a medicament for treating or preventing a disorder selected from cancer, septic shock, Primary open Angle Glaucoma (POAG), hyperplasia, rheumatoid arthritis, psoriasis, artherosclerosis, retinopathy, osteoarthritis, endometriosis, chronic inflammation and Alzheimer's disease.

A sixth aspect of the invention relates to the use of a compound as described above in the preparation of a medicament for the prevention or treatment of a disorder caused by, associated with or accompanied by any abnormal kinase activity, wherein the kinase is TBK1.

A seventh aspect of the invention relates to the use of a compound as described above in an assay for identifying further candidate compounds capable of inhibiting TBK1.

An eighth aspect of the invention relates to a process for preparing a compound of formula VII, wherein R¹ and R² are as defined above, said process comprising the steps of: 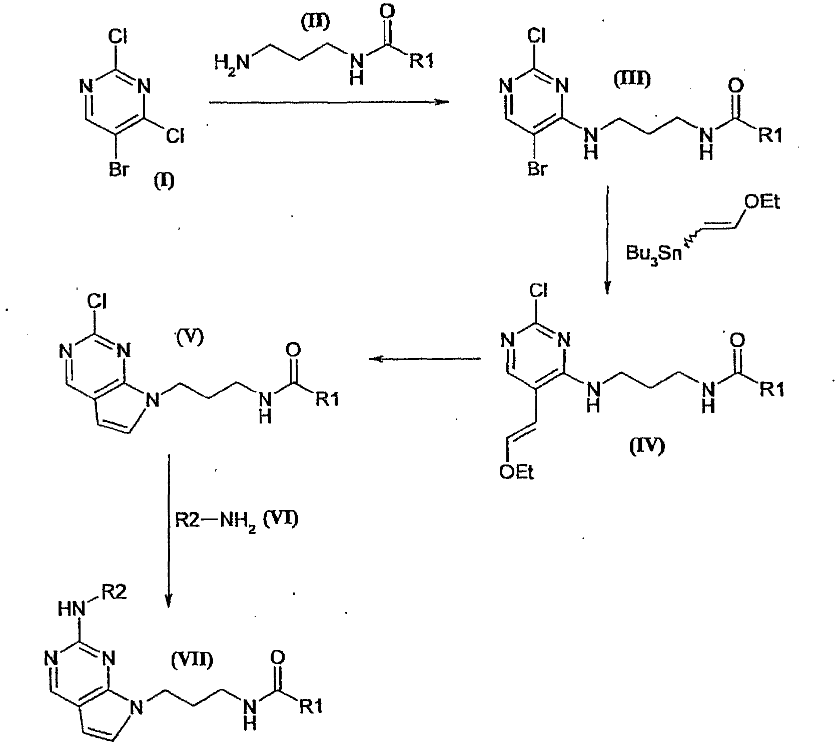
(i) reacting 5-bromo-2,4-dichloropyrimidine (I) with an amine of formula II to give a compound of formula III;
(ii) reacting said compound of formula III with ethoxyvinyltin to give a compound of formula IV;
(iii) cyclising said compound of formula IV to form a compound of formula V;
(iv) reacting said compound of formula V with an amine of formula VI to give a compound of formula VII.

### DETAILED DESCRIPTION

"alkyl" is defined herein as a straight-chain or branched alkyl radical, for example, methyl, ethyl, propyl, isopropyl, butyl, isobutyl, tert-butyl, pentyl, hexyl.

"Cycloalkyl° is defined herein as a monocyclic alkyl ring, such as, cyclopropyl, cyclobutyl, cyclopentyl, cyclohexyl or cycloheptyl.

"Halogen" is defined herein as chloro, fluoro, bromo or iodo.

As used herein, the term "aryl" refers to a C₆₋₁₂ aromatic group, which may be benzocondensed, for example, phenyl or naphthyl.

"Heteroaryl" is defined herein as a monocyclic or bicyclic C₂₋₁₂ aromatic ring comprising one or more heteroatoms (that may be the same or different), such as oxygen, nitrogen or sulphur. Examples of suitable heteroaryl groups include thienyl, furanyl, pyrrolyl, pyridinyl, oxazolyl, thiazolyl, imidazolyl, pyrazolyl, isoxazolyl, isothiazolyl, oxadiazolyl, triazolyl, thiadiazolyl etc. and benzo derivatives thereof, such as benzofuranyl, benzothienyl, benzimidazolyl, indolyl, isoindolyl, indazolyl etc.; or pyridyl, pyrazinyl, pyrimidinyl, pyridazinyl, triazinyl etc. and benzo derivatives thereof, such as quinolinyl, isoquinolinyl, cinnolinyl, phthalazinyl, quinazolinyl, quinoxalinyl, naphthyridinyl etc.

"Heterocycloalkyl" refers to a cyclic aliphatic group containing one or more heteroatoms selected from nitrogen, oxygen and sulphur, which is optionally interrupted by one or more -(CO)- groups in the ring and/or which optionally contains one or more double bonds in the ring. Preferably, the heterocycloalkyl group is a C₃₋₇-heterocycloalkyl, more preferably a C₃₋₆-heterocycloalkyl. Alternatively, the heterocycloalkyl group is a C₄₋₇-heterocycloalkyl, more preferably a C₄₋₆-heterocycloalkyl.

As mentioned above, for compounds of formula I, R¹ is NR⁷(CO)R¹¹.

In one preferred embodiment, R¹ is selected from NHCO-C₁₋₆-alkyl, NHCO-C₃₋₇-cycloalkyl, NHCO-C₁₋₆-alkyl-C₃₋₇-cycloalkyl, NHCO-heteroaryl, NHCO-C₃₋₆-heterocycloalkyl.

In a more preferred embodiment, R¹ is NHCO-C₃₋₇cycloalkyl.

In one preferred embodiment, R¹ is selected from NHCO-cyclobutyl, NHCO-cyclopentyl, NHCO-cyclohexyl and NHCO-thienyl.

In one preferred embodiment, R¹ is selected from NHCO-cyclobutyl, NHCO-thienyl (more preferably, NHCO-thien-2-yl), NHCO-cyclopentyl, NHCO-pyrazinyl, NHCOCH₂-cyclopropyl, NHCO-sec-butyl, NHCO-tetrahydrofuranyl (more preferably, NHCO-tetrahydrofuran-2-yl), NHCO-thiazolyl (more preferably, NHCO-thiazol-5-yl), NHCO-cyclopropyl, NHCO-isopropyl, NHCO-cyclohexyl, NHCOCH₂-cyclopentyl and NHCO-n-propyl.

R² is aryl, heteroaryl, fused aryl-C₃₋₆-heterocycloalkyl or fused heteroaryl-C₃₋₆-heterocycloalkyl, each of which is optionally substituted by one or more substitutents selected from aryl, heteroaryl, C₁₋₆-alkyl, C₃₋₆-heterocycloalkyl and a group A, wherein said C₁₋₆-alkyl group is in turn optionally substituted by one or more substituents selected from aryl, heteroaryl, C₃₋₆-heterocycloalkyl and a group A, said heteroaryl group is optionally substituted by one or more R¹⁰ groups; and wherein said C₃₋₆-heterocycloalkyl group is optionally substituted by one or more groups selected from alkyl and A, and optionally contains one or more groups selected from oxygen, sulphur, nitrogen and CO. Preferably, the C₃₋₆-heterocycloalkyl group is optionally substituted by one or more alkyl or COR⁶ groups

In one preferred embodiment, R² is aryl or heteroaryl, each of which is optionally substituted by one or more substitutents selected from aryl, heteroaryl, C₁₋₆-alkyl, C₃₋₆-heterocycloalkyl and a group A, wherein said C₁₋₆-alkyl group is in turn optionally substituted by one or more substituents selected from aryl, heteroaryl, C₃₋₆-heterocycloalkyl group and a group A, said heteroaryl group is optionally substituted by one or more R¹⁰ groups; and wherein said C₃₋₆-heterocycloalkyl group optionally contains one or more groups selected from oxygen, sulphur, nitrogen and CO, and is optionally substituted by one or more alkyl or A groups.

.In a more preferred embodiment, R² is selected from aryl or heteroaryl, each of which is optionally substituted by one or more substitutents selected from halo, optionally substituted C₃₋₇-heterocycloalkyl, optionally substituted C₁₋₆-alkyl, heteroaryl, C₁₋₆-alkyl-C₃₋₇-heterocycloalkyl, CN, NHCO-C₃₋₇-heterocycloalkyl, CO-C₃₋₇-heterocycloalkyl and NHCO-C₁₋₆-alkyl, wherein said C₃₋₇-heterocycloalkyl is optionally substituted by one or more C₁₋₆-alkyl or A groups, and said C₁₋₆-alkyl is optionally substituted by one or more halo or NR⁴R⁵ groups.

In one preferred embodiment, R² is selected from phenyl, pyridin-3-yl, pyrazol-4-yl, indazol-5-yl, indazol-6-yl, quinolinyl, quinoxalinyl, pyrazolopyridinyl, imidazopyridinyl and tetrahydroisoquinolinyl, each of which may be optionally substituted. Preferably, R² is selected from phenyl, pyridin-3-yl, pyrazol-4-yl, indazol-5-yl and indazol-6-yl.

In one particularly preferred embodiment, R² is selected from:
(i) phenyl optionally substituted by:
   halo, optionally substituted C₃₋₇-heterocycloalkyl, optionally substituted C₁₋₆-alkyl, heteroaryl, C₁₋₆-alkyl-C₃₋₇-heterocycloalkyl, CN, NHCO-C₃₋₇-heterocycloalkyl, CO-C₃₋₇-heterocycloalkyl or NHCO-C₁₋₆-alkyl, wherein said C₃₋₇-heterocycloalkyl is optionally substituted by one or more C₁₋₆-alkyl,
      CN, OH, alkoxy, haloalkyl, COR⁶ groups, and said C₁₋₆-alkyl is optionally substituted by one or more halo or NR⁴R⁵ groups;
(ii) pyridinyl optionally substituted by C₃₋₇-heterocycloalkyl, wherein said C₃₋₇-heterocycloalkyl is optionally further substituted by one or more C₁₋₆-alkyl groups;
(iii) pyrazolyl substituted by C₁₋₆-alkyl, C₁₋₆-alkyl-C₃₋₇-heterocycloalkyl or C₃₋₇-heterocycloalkyl,
   wherein said C₃₋₇-heterocycloalkyl is optionally further substituted by one or more C₁₋₆-alkyl groups;
(iv) indazolyl optionally substituted by C₁₋₆-alkyl;
(v) quinolinyl;
(vi) quinoxalinyl;
(vii) pyrazolopyridinyl;
(viii) imidazopyridinyl; and
(ix) tetrahydroisoquinolinyl.

In one particularly preferred embodiment, R² is selected from:
(i) phenyl optionally substituted by:
   halo, optionally substituted C₃₋₇-heterocycloalkyl, optionally substituted C₁₋₆-alkyl, heteroaryl, C₁₋₆-alkyl-C₃₋₇-heterocycloalkyl, CN, NHCO-C₃₋₇-heterocycloalkyl, CO-C₃₋₇-heterocydoalkyl or NHCO-C₁₋₆-alkyl, wherein said C₃₋₇-heterocycloalkyl is optionally substituted by one or more C₁₋₆-alkyl or COR⁶ groups, and said C₁₋₆-alkyl is optionally substituted by one or more halo or NR⁴R⁵ groups;
(ii) pyridinyl optionally substituted by C₃₋₇-heterocycloalkyl;
(iii) pyrazolyl substituted by C₁₋₆-alkyl, C₁₋₆-alkyl-C₃₋₇-heterocycloalkyl or C₃₋₇-heterocycloalkyl;
(iv) indazolyl optionally substituted by C₁₋₆-alkyl.

In one preferred embodiment, R² is selected from:
(i) phenyl optionally substituted by F, N-morpholinyl, N-methylpiperazinyl, CH₂-NMe₂, CH₂-pyrrolidinyl, oxazolyl, CN, CF₃, NHCO-pyrrolidinyl, CO-morpholinyl, NHCOMe, 2-oxopyrrolidin-1-yl, 1,2,4-triazol-1-yl, 4-hydroxy-1-methylpiperidin-4-yl, 1-methyl-piperidin-4-yl, 4-methoxy-1-methylpiperidin-4-yl, morpholin-4-yl-methyl, 4-cyano-1-methytpiperidin-4-yl, piperidin-1-yl-methyl or 1-(2-fluoroethyl)-piperidin-4-yl,
(ii) pyridinyl optionally substituted by morpholinyl or 4-methyl-perhydro-1,4-diazepin-1-yl;
(iii) pyrazolyl optionally substituted by Me, Et, CH₂CH₂-morpholinyl, or 1-isopropyl-piperidin-4-yl;
(iv) indazolyl optionally substituted by Me.

In one preferred embodiment, R² is selected from:
(i) phenyl optionally substituted by F, N-morpholinyl, N-methylpiperazinyl, CH₂-NMe₂, CH₂-pyrrolidinyl, oxazolyl, CN, CF₃, NHCO-pyrrolidinyl, CO-morpholinyl, NHCOMe, 2-oxopyrrolidin-1-yl;
(ii) pyridinyl optionally substituted by morpholinyl;
(iii) pyrazolyl optionally substituted by Me or CH₂CH₂-morpholinyl;
(iv) indazolyl optionally substituted by Me.

In one particularly preferred embodiment, R² is selected from pyridin-3-yl, 6-(morpholin-4-yl)-pyridin-3-yl and 6-(4-methylpiperazin-1-yl)-pyridin-3-yl.

In another preferred embodiment, R² is selected from 1-Me-1*H*-pyrazol-4-yl and 1-(2-morpholin-4-yl-ethyl)-1*H*-pyrazol-4-yl.

In another preferred embodiment, R² is selected from 1-Me-1*H*-indazol-5-yl and 1-Me-1*H*-indazol-6-yl.

In one preferred embodiment, R² is selected from: pyridin-3-yl, 6-(morpholin-4-yl)-pyridin-3-yl, 6-(4-methylpiperazin-1-yl)-pyridin-3-yl, 1-Me-1*H*-pyrazol-4-yl, 1-(2-morpholin-4-yl-ethyl)-1*H*-pyrazol-4-yl, 1-Me-1*H*-indazol-5-yl, 1-Me-1*H*-indazol-6-yl, 3-fluorophenyl, 3-trifluoromethylphenyl, 3-cyanophenyl, 3-oxazol-5-yl-phenyl, 3-acetylaminophenyl, 4-dimethylaminomethylphenyl, 4-(4-methyl-piperazin-1-yl)-phenyl, 3-pyrrolidin-1-yl-methylphenyl, 4-(morpholin-4-yl)-phenyl, 4-(morpholine-4-carbonyl)-phenyl, 3-(2-oxo-pyrrolidin-1-yl)-phenyl, 3-(pyrrolidin-1-yl-carboxyamino)-phenyl, 4-(2-oxo-pyrrolidin-1-yl)-phenyl,1*H*-indazol-5-yl, 3-(1,2,4-triazol-1-yl-phenyl), 4-(1,2,4-triazol-1-yl-phenyl), quinoxalin-6-yl, quinolin-6-yl, imidazo[1,2-a]pyridine-6-yl, 1-methyl-1H-pyrazolo[3,4b]pyridine-5-yl, 1-ethyl-1*H*-pyrazol-4-yl, piperidin-1-yl-methylphenyl, (1-isopropyl-piperidin-4-yl)-1H-pyrazol-4-yl, 6-(4-methyl-pefiydro-1,4-diazepin-1-yl)-pyridin-3-yl, morpholin-4-yl-methyl-phenyl, 2-methyl-1,2,3,4-tetrahydroisoquinolin-7-yl, 4-oxazol-5-yl-phenyl, 4-(l-methylpiperidin-4-yl)-phenyl, 4-(4-hydroxy-1-methyl-piperidin-4-yl)-phenyl, 4-(4-methoxy-1-methyl-piperidin-4-yl)-phenyl, 4-(4-cyano-1-methyl-piperidin-4-yl)-phenyl and 4-(1-(2-fluoroethyl)-piperidin-4-yl)-phenyl.

In another preferred embodiment, R² is selected from 3-fluorophenyl, 3-trifluoromethylphenyl, 3-cyanophenyl, 3-oxazol-5-yl-phenyl, 3-acetylaminophenyl, 4-dimethylaminomethylphenyl, 4-(4-methyl-piperazin-1-yl)-phenyl, 3-pyrrolidin-1-yl-methylphenyl, 4-(morpholin-4-yl)-phenyl, 4-(morpholine-4-carbonyl)-phenyl, 3-(2-oxopyrrolidin-1-yl)-phenyl and 3-(pyrrolidin-1-yl-carboxyamino)-phenyl.

In preferred embodiment, R³ is selected from H, halo and CN. More preferably, R³ is selected from H, Cl, Br and CN.

In preferred embodiment, R⁷ is selected from H and C₁₋₆-alkyl. More preferably R⁷ is H.

In one highly preferred embodiment, the compound of the invention is selected from the following:
Cyclobutanecarboxylic acid {3-[2-(1-methyl-1H-indazol-5-ylamino)-pyrrolo[2,3-d]pyrimidin-7-yl]-propyl}-amide [1];
Cyclobutanecarboxylic acid {3-[2-(1-methyl-1H-indazol-6-ylamino)-pyrrolo[2,3-d]pyrimidin-7-yl]-propyl}-amide [2];
Cyclobutanecarboxylic acid {3-[2-(4-morpholin-4-yl-phenylamino)-pyrrolo[2,3-d]pyrimidin-7-yl]-propyl}-amide [3];
Cyclobutanecarboxylic acid (3-{2-[4-(4-methyl-piperafin-1-yl)-phenylaminol-pyrrolo[2,3-d]pyrimidin-7-yl}-propyl)-amide [4];
Cyclobutanecarboxylic acid {3-[2-(4-dimethylaminomethyl-phenylamino)-pyrrolo[2,3-d]pyrimidin-7-yl]-propyl}-amide [5];
Cyclobutanecarboxylic acid {3-[2-(3-pyrrolidin-1-ylmethyl-phenylamino)-pyrrolo[2,3-d]pyrimidin-7-yl]-propyl}-amide [6];
Cyclobutanecarboxylic acid {3-[2-(3-oxazol-5-yl-phenytamino)-pyrrolo[2,3-d]pyrimidin-7-yl]-propyl}-amide [7];
Cyclobutanecarboxylic acid {3-[2-(1-methyl-1H-pyrazol-4-ylamino)-pyrrolo[2,3-d]pyrimidin-7-yl]-propyl}-amide [8];
Cyclobutanecarboxylic acid (3-{2-[1-(2-morpholin-4-yl-ethyl)-1H-pyrazol-4-ylamino]-pyrrolo[2,3-d]pyrimidin-7-yl)-propyl)-amide [9];
Thiophene-2-carboxylic acid {3-[2-(3-fluoro-phenylamino)-pyrrolo[2,3-d]pyrimidin-7-yl]-propyl}-amide [10];
Thiophene-2-carboxylic acid {3-[2-(3-cyano-phenylamino)-pyrrolo[2,3-d]pyrimidin-7-yl]-propyl}-amide [11];
Thiophene-2-carboxylic acid {3-[2-(pyridin-3-ylamino)-pyrrolo[2,3-d]pyrimidin-7-yl]-propyl}-amide [12];
Thiopherie-2-carboxylic acid {3-[2-(3-trifluoromethyl-phenylamino)-pyrrolo[2,3-d]pyrimidin-7-yl]-propyl}-amide [13];
Pyrrolidine-1-carboxylic acid [3-(7-{3-[(thiophene-2-carbonyl)-amino]-propyl)-7H-pyrrolo[2,3-d]pyrimidin-2-ylamino)-phenyl]-amide [14];
Cyclobutanecarboxylic acid (3-{2-[4-(morphotine-4-carbonyl)-phenylamino]-pyrrolo[2,3-d]pyrimidin-7-yl}-propyl)-amide [15];
Cyclopentanecarboxylic acid {3-[2-(6-morpholin-4-yl-pyridin-3-ylamino)-pyrrolo[2,3-d]pyrimidin-7-yl]-propyl)-amide [16];
Pyrazine-2-carboxylic acid {3-[2-(6-morpholin-4-yl-pyridin-3-ylamino)-pyrrolo[2,3-d]pyrimidin-7-yl]-propyl}-amide [17];
2-Cyclopropyl-N-{3-[2-(6-morpholin-4-yl-pyridin-3-ylamino)-pyrrolo[2,3-d]pyrimidin-7-yl]-propyl}-acetamide [18];
3-Methyl-N-{3-[2-(6-morpholin-4-yl-pyridin-3-ylamino)-pyrrolo[2,3-dlpyrimidin-7-yl]-propyl}-butyramide [19];
Tetrahydro-furan-3-carboxylic acid {3-[2-(6-morpholin-4-yl-pyridin-3-ylamino)-pyrrolo[2,3-d]pyrimidin-7-yl]-propyl}-amide [20];
Thiazole-5-carboxylic acid {3-[2-(6-morpholin-4-yl-pyridin-3-ylamino)-pyrrolo[2,3-d]pyrimidin-7-yl]-propyl}-amide [21];
Cyclopropanecarboxylic acid {3-[2-(6-morpholin-4-yl-pyridin-3-ylamino)-pyrrolo[2,3-d]pyrimidin-7-yl]-propyl}-amide [22];
N-{3-[2-(6-Morpholin-4-yl-pyridin-3-ylamino)-pyrrolo[2,3-d]pyrimidin-7-yl]-propyl)-isobutyramide [23];
Cyclohexanecarboxylic acid {3-[2-(6-morpholin-4-yl-pyridin-3-ylamino)-pyrrolo[2,3-d]pyrimidin-7-yl]-propyl}-amide [24];
2-Cyclopentyl-N-{3-[2-(6-morpholin-4-yl-pyridin-3-ylamino)-pyrrolo[2,3-d]pyrimidin-7-yl]-propyl}-acetamide [25];
N-{3-[2-(6-Morpholin-4-yl-pyridin-3-ylamino)-pyrrolo[2,3-d]pyrimidin-7-yl]-propyl)-butyramide [26];
Cyclobutanecarboxylic acid {3-[2-(3-fluoro-phenylamino)-pyrrolo[2,3-d]pyrimidin-7-yl]-propyl}-amide [27];
Cyclobutanecarboxylic acid {3-[2-(3-acetylamino-phenylamino)-pyrrolo[2,3-d]pyrimidin-7-yl]-propyl}-amide [28];
Cyclobutanecarboxylic acid (3-{2-[3-(2-oxo-pyrrolidin-1-yl)-phenylamino]-pyrrolo[2,3-d]pyrimidin-7-yl}-propyl)-amide [29];
Cyclobutanecarboxylic acid {3-[2-(6-morpholin-4-yl-pyridin-3-ylamino)-pyrrolo[2,3-d]pyrimidin-7-yl]-propyl}-amide [30];
Cyclobutanecarboxylic acid (3-{2-[6-(4-methyl-piperazin-1-yl)-pyridin-3-ylamino]-pyrrolo[2,3-d]pyrimidin-7-yl}-propyl)-amide [31];
Cyclopentanecarboxylic acid {3-[2-(3-fluoro-phenylamino)-pyrrolo[2,3-d]pyrimidin-7-yl]-propyl}-amide [32];
Cyclopentanecarboxylic acid {3-[2-(3-acetylamino-phenylamino)-pyrrolo[2,3-d] pyrimidin-7-yl]-propyl}-amide [33];
Cyclobutanecarboxylic acid {3-[5-chloro-2-(3-fluoro-phenylamino)-pyrrolo[2,3-, d]pyrimidin-7-yl]-propyl}-amide [34];
Cyclobutanecarboxylic acid {3-[5-chloro-2-(6-morpholin-4-yl-pyridin-3-ylamino)-pyrrolo[2,3-d]pyrimidin-7-yl]-propyl}-amide [35];
Cyclobutanecarboxylic acid {3-[5-bromo-2-(3-fluoro-phenylamino)-pyrrolo[2,3-d]pyrimidin-7-yl]-propyl}-amide [36];
Cyclobutanecarboxylic acid {3-[5-cyano-2-(3-fluoro-phenylamino)-pyrrolo[2,3-d]pyrimidin-7-yl]-propyl}-amide [37];
Thiophene-2-carboxylic acid {3-[5-chloro-2-(3-fluoro-phenylamino)-pyrrolo[2,3-d]pyrimidin-7-yl]-propyl}-amide [38];
Cyclobutanecarboxylic acid (3-{2-[4-(2-oxo-pyrrolidin-1-yl)-phenylamino]-pyrrolo[2,3-d]pyrimidin-7-yl}-propyl)-amide [39];
Cyclobutanecarboxylic acid {3-[2-(1H-indazo)-5-ylamino)-pyrrolo[2,3-d]pyrimidin-7-yl]-propyl}-amide[40];
Cyclobutanecarboxylic acid {3-[2-(3-1,2,4-triazol-1-yl-phenylamino)-pyrrolo[2,3-d]pyrimidin-7-yl]-propyl}-amide [41];
Cyclobutanecarboxylic acid {3-[2-(4-1,2,4-triazol-1-yl-phenylamino)-pyrrolo[2,3-d]pyrimidin-7-yl]-propyl}-amide [42];
Cyclobutanecarboxylic acid {3-[2-(pyridin-3-ylamino)-pyrrolo[2,3-d]pyrimidin-7-yl]-propyl}-amide [43];
Cyclobutanecarboxylic acid {3-[2-(1-methyl-1H-pyrazolo[3,4-b]pyridin-5-ylamino)-pyrrolo[2,3-d]pyrimidin-7-yl]-propyl}-amide [44];
Cyclobutanecarboxylic acid {3-[2-(imidazo[1,2-a]pyridin-6-ylamino)-pyrrolo[2,3-d]pyrimidin-7-yl]-propyl}-amide [45];
Cyclobutanecarboxylic acid {3-[2-(quinoxalin-6-ylamino)-pyrrolo[2,3-d]pyrimidin-7-yl]-propyl]-amide [46];
Cyclobutanecarboxylic acid {3-[2-(1-ethyl-1H-pyrazol-4-ylamino)-pyrrolo[2,3-d]pyrimidin-7-yl]-propyl}-amide [47];
Cyclobutanecarboxylic acid {3-[2-(3-morpholin-4-yl-phenylamino)-pyrrolo[2,3-d]pyrimidin-7-yl]-propyl}-amide [48];
Cyclobutanecarboxylic acid {3-[2-(3-piperidin-1-ylmethyl-phenylamino)-pyrrolo[2,3-d]pyrimidin-7-yl]-propyl}-amide [49];
Cyclobutanecarboxylic acid {3-[2-(quinolin-6-ylamino)-pyrrolo[2,3-d]pyrimidin-7-yl]-propyl}-amide [50];
Cyclobutanecarboxylic acid (3-{2-[1-(1-isopropyl-piperidin-4-yl)-1H-pyrazol-4-ylamino]-pyrrolo[2,3-d]pyrimidin-7-yl}-propyl)-amide [51];
Cyclobutanecarboxylic acid {3-[2-(3-morpholin-4-ylmethyl-phenylamino)-pyrrolo[2,3-d]pyrimidin-7-yl]-propyl}-amide [52];
Cyclobutanecarboxylic acid {3-[2-(2-methyl-1,2,3,4-tetrahydro-isoquinolin-7-ylamino)-pyrrolo[2,3-dlpyrimidin-7-yl]-propyl}-amide [53];
Cyclobutanecarboxylic acid {3-[2-(4-oxazol-5-yl-phenylamino)-pyrrolo[2,3-d]pyrimidin-7-yl]-propyl}-amide [54];
Cyclobutanecarboxylic acid (3-{2-[6-(4-methyl-perhydro-1,4-diazepin-1-yl)-pyridin-3-ylamino]-pyrrolo[2,3-d]pyrimidin-7-yl}-propyl)-amide [55];
Cyclobutanecarboxylic acid (3-{2-[4-(1-methyl-piperidin-4-yl)-phenylamino]-pyrrolo[2,3-d]pyrimidin-7-yl}-propyl)-amide [56];
Cyclobutanecarboxylic acid (3-{2-[4-(4-hydroxy-1-methyl-piperidin-4-yl)-phenylamino]-pyrrolo[2,3-d]pyrimidin-7-yl}-propyl)-amide [57];
Cyclobutanecarboxylic acid (3-{2-[4-(4-methoxy-1-methyl-piperidin-4-yl)-phenylamino]-pyrrolo[2,3-d]pyrimidin-7-yl}-propyl)-amide [58];
Cyclobutanecarboxylic acid (3-{2-[4-(4-cyano-1-methyl-piperidin4-yl)-phenylamino]-pyrrolo[2,3-d]pyrimidin-7-yl}-propyl)-amide [59] and;
Cyclobutanecarboxylic acid [3-(2-{4-[1-(2-fluoro-ethyl)-piperidin-4-yl]-phenylamino}-pyrrolo[2,3-d]pyrimidin-7-yl)-propyl]-amide [60].

In one highly preferred embodiment of the invention, the compound has an IC₅₀ value against TBK1 of 10 µM or less. More preferably, the IC₅₀ value is between 1 µM and 10 µM, even more preferably, between 100 nM and 1 µM, even more preferably still, <100 nM.

In one especially preferred embodiment, the compound of the invention is selected from compounds [1]-[8], [10], [15], [16], [20], [27], [28], [30], [33]-[36], [39]-[54] and [56]-[60] as set forth above.

A further aspect of the invention relates to a compound as described above for use in medicine.

### THERAPEUTIC APPLICATIONS

Another aspect of the invention relates to a compound as described above for use in treating a disorder selected from cancer, septic shock, Primary open Angle Glaucoma (POAG), hyperplasia, rheumatoid arthritis, psoriasis, artherosclerosis, retinopathy, osteoarthritis, endometriosis, chronic inflammation and neurodegenerative diseases including Alzheimer's disease.

Another aspect relates to the use of a compound as described above in the preparation of a medicament for treating or preventing a disorder selected from cancer, septic shock, Primary open Angle Glaucoma (POAG), hyperplasia, rheumatoid arthritis, psoriasis, artherosclerosis, retinopathy, osteoarthritis, endometriosis, chronic inflammation and neurodegenerative diseases including Alzheimer's disease.

Preferably, the compound is administered in an amount sufficient to inhibit TBK1.

Yet another aspect relates to the use of a compound of the invention in the preparation of a medicament for the prevention or treatment of a disorder caused by, associated with or accompanied by any abnormal kinase activity, wherein the kinase is TBK1.

Preferably, the disorder is selected from cancer, septic shock, diseases of the eye, including Primary open Angle Glaucoma (POAG), hyperplasia, rheumatoid arthritis, autoimmune diseases, artherosclerosis, retinopathy, osteoarthritis, fibrotic diseases, endometriosis, chronic inflammation and neurodegenerative diseases including Alzheimet's disease.

In the innate immune system TBK1 is activated in response to LPS engagement of Toll-like receptor 4 (TLR4). or double-stranded RNA (from double stranded RNA viruses) engagement of TLR3. It is also activated in response to the pro-inflammatory cytokines TNF and IL-1. Once activated TBK1 phosphorylates and activates interferon-regulatory factor 3 (IRF3), a transcription factor that triggers the production of interferon beta and chemokines, like interleukin-8 (IL-8) and RANTES. These substances play a key role in mediating host defence against infection by bacteria and viruses. Mice that do not express interferon beta or IRF3 are resistant to LPS-induced septic shock. These observations suggest that a drug that inhibits TBK1 may have efficacy for the treatment/prevention of septic shock and/or the treatment of inflammatory disease.

TBK1 is also activated in response to hypoxia and stimulates the production of pro-angiogenic factors, such as VEGF and IL-1. The expression of TBK1 rises 2.5-3-fold after 24h of hypoxia, similar to the increase in expression of VEGF. The hypoxia induced increase in VEGF expression can be abolished by siRNA "knockdown" of TBK1. The level of TBK1 mRNA and protein is elevated in malignant colon and breast cancer cells (see Korherr et al (2006) PNAS 103, 4240-4245 and references therein). TBK1 is also recruited and activated by the RalB/Sec5 effector complex; in cancer cells, constitutive engagement of this pathway via chronic RalB activation, restricts the initiation of apoptotic programmes (Chien et al (2006) Cell 127, 157-170 and references there-in). For these reasons the drugs that inhibit TBK1 may have efficacy for the treatment of cancers.

In one preferred embodiment, the compounds of the invention are useful in the treatment of Primary open Angle Glaucoma (POAG).

Primary Open Angle Glaucoma (POAG) is a leading cause of irreversible blindness affecting 35 million people worldwide. The disease is genetically heterogeneous and mutations in the protein optineurin (OPTN) are associated with a form of POAG associated with normal intraocular pressure, termed Normal Tension Glaucoma (NTG) or Low Tension Glaucoma (LTG).^{1,2} A study of 54 families with autosomal dominant adult onset glaucoma, 17% had one of four sequence mutations in OPTN, the most prevalent being the OPTN[E50K] mutant. How this mutation in OPTN might cause POAG is unknown. However, tumour necrosis factor α (TNFα) has been reported to increase the severity of damage in optic nerve heads of POAG and LTG subjects^{3,4}. Moreover, exposure to TNFα¹⁰ induces the *de novo* expression of optineurin. These observations suggest that some forms of POAG may be caused by an abnormal response to this cytokine.¹¹ The compounds described herein may therefore find use in treating POAG and/or diseases associated with optineurin activity.

Preferably, the mammal is a human.

The term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

The term "administering" as used herein refers to a method for bringing a compound of the present invention and a target kinase together in such a manner that the compound can affect the enzyme activity of the kinase either directly; i.e., by interacting with the kinase itself or indirectly; i.e., by interacting with another molecule on which the catalytic activity of the kinase is dependent. As used herein, administration can be accomplished either *in vitro,* i.e. in a test tube, or *in vivo,* i.e., in cells or tissues of a living organism.

Herein, the term 'treating" includes abrogating, substantially inhibiting, slowing or reversing the progression of a disease or disorder, substantially ameliorating clinical symptoms of a disease or disorder or substantially preventing the appearance of clinical symptoms of a disease or disorder.

Herein, the term "preventing" refers to a method for barring an organism from acquiring a disorder or disease in the first place.

The term "therapeutically effective amount" refers to that amount of the compound being administered which will relieve to some extent one or more of the symptoms of the disease or disorder being treated.

For any compound used in this invention, a therapeutically effective amount, also referred to herein as a therapeutically effective dose, can be estimated initially from cell culture assays. For example, a dose can be formulated in animal models to achieve a circulating concentration range that includes the IC₅₀ or the IC₁₀₀ as determined in cell culture. Such information can be used to more accurately determine useful doses in humans. Initial dosages can also be estimated from *in vivo* data. Using these initial guidelines one of ordinary skill in the art could determine an effective dosage in humans.

Moreover, toxicity and therapeutic efficacy of the compounds described herein can be determined by standard pharmaceutical procedures in cell cultures or experimental animals, e.g., by determining the LD₅₀ and the ED₅₀. The dose ratio between toxic and therapeutic effect is the therapeutic index and can be expressed as the ratio between LD₅₀ and ED₅₀. Compounds which exhibit high therapeutic indices are preferred. The data obtained from these cell cultures assays and animal studies can be used in formulating a dosage range that is not toxic for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (see, e.g., Fingl *et al,* 1975, In: The Pharmacological Basis of Therapeutics, chapter 1, page 1).

Dosage amount and interval may be adjusted individually to provide plasma levels of the active compound which are sufficient to maintain therapeutic effect. Usual patient dosages for oral administration range from about 50-2000 mg/kg/day, commonly from about 100-1000 mg/kg/day, preferably from about 150-700 mg/kg/day and most preferably from about 250-500 mg/kg/day. Preferably, therapeutically effective serum levels will be achieved by administering multiple doses each day. In cases of local administration or selective uptake, the effective local concentration of the drug may not be related to plasma concentration. One skilled in the art will be able to optimize therapeutically effective local dosages without undue experimentation.

As used herein, "kinase related disease or disorder" refers to a disease or disorder characterized by inappropriate kinase activity or over-activity of a kinase as defined herein, wherein the kinase is TBK1. Inappropriate activity refers to either; (i) kinase expression in cells which normally do not express said kinase; (ii) increased kinase expression leading to unwanted cell proliferation, differentiation and/or growth; or, (iii) decreased kinase expression leading to unwanted reductions in cell proliferation, differentiation and/or growth. Over-activity of kinase refers to either amplification of the gene encoding a particular kinase or production of a level of kinase activity, which can correlate with a cell proliferation, differentiation and/or growth disorder (that is, as the level of the kinase increases, the severity of one or more of the symptoms of the cellular disorder increases). Over activity can also be the result of ligand independent or constitutive activation as a result of mutations such as deletions of a fragment of a kinase responsible for ligand binding.

Preferred diseases or disorders that the compounds described herein may be useful in preventing, treating and/or studying are cell proliferative disorders, especially cancer such as, but not limited to, papilloma, blastoglioma, Kaposi's sarcoma, melanoma, lung cancer, ovarian cancer, prostate cancer, squamous cell carcinoma, astrocytoma, head cancer, neck cancer, skin cancer, liver cancer, bladder cancer, breast cancer, lung cancer, uterus cancer, prostate cancer, testis carcinoma, colorectal cancer, thyroid cancer, pancreatic cancer, gastric cancer, hepatocellular carcinoma, leukemia, lymphoma, Hodgkin's disease and Burkitt's disease.

Another condition to which the compounds described herein may be useful in preventing, treating and/or studying is septic shock.

Another condition to which the compounds described herein may be useful in preventing, treating and/or studying is inflammatory disease.

P. Cohen et al have observed that TBK1 binds in an enhanced manner to the mutant form of optineurin which causes a form of Primary Open Angle Glaucoma (POAG).¹¹ The compounds described herein may therefore find use in treating POAG and/or diseases associated with optineurin activity.

A further aspect relates to the use of a compound which is capable of inhibiting the binding of TBK1 to a mutant form of OPTN for the manufacture of a medicament for treating POAG and/or a disease where it would be desirable to inhibit or reduce TBK1 binding to mutant form of OPTN. One such mutant is the OPTN (E50K) mutant. Suitable compounds may include the compounds identified herein.

Thus, the present invention further provides use of compounds as defined herein for the manufacture of medicaments for the treatment of diseases where it is desirable to inhibit TBK1. Such diseases include colon and breast cancer, septic shock and/or POAG. A number of papers^{5,6,7} have described that TBK1 and IKKepsilon modulate expression of interferon and interferon inducible genes, without affecting induction of pro-inflammatory cytokines. This indicates that the compounds disclosed herein, may find applications in treating/preventing septic shock or viral infection. Mice that do not express interferon beta or IRF3 are resistant to lipopolysaccharide induced septic shock so that inhibitors of TBK1 should be expected to have a similar effect.

### PHARMACEUTICAL COMPOSTIONS

For use according to the present invention, the compounds or physiologically acceptable salt, ester or other physiologically functional derivative thereof, described herein, may be presented as a pharmaceutical formulation, comprising the compounds or physiologically acceptable salt, ester or other physiologically functional derivative thereof, together with one or more pharmaceutically acceptable carriers therefore and optionally other therapeutic and/or prophylactic ingredients. The carrier(s) must be acceptable in the sense of being compatible with the other ingredients of the formulation and not deleterious to the recipient thereof. The pharmaceutical compositions may be for human or animal usage in human and veterinary medicine.

Examples of such suitable excipients for the various different forms of pharmaceutical compositions described herein may be found in the "Handbook of Pharmaceutical Excipients, 2nd Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s), buffer(s), flavouring agent(s), surface active agent(s), thickener(s), preservative(s) (including anti-oxidants) and the like, and substances included for the purpose of rendering the formulation isotonic with the blood of the intended recipient.

Examples of suitable binders include starch, gelatin, natural sugars such as glucose, anhydrous lactose, free-flow lactose, beta-lactose, com sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

Pharmaceutical formulations include those suitable for oral, topical (including dermal, buccal and sublingual), rectal or parenteral (including subcutaneous, intradermal, intramuscular and intravenous), nasal and pulmonary administration e.g., by inhalation. The formulation may, where appropriate, be conveniently presented in discrete dosage units and may be prepared by any of the methods well known in the art of pharmacy. All methods include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of active compound. A tablet may be made by compression or moulding, optionally with one or more accessory ingredients. Compressed tablets may be prepared by compressing in a suitable machine an active compound in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersible granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms, e.g., tablets wherein an active compound is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active compound with the softened or melted carrier(s) followed by chilling and shaping in moulds.

Pharmaceutical formulations suitable for parenteral administration include sterile solutions or suspensions of an active compound in aqueous or oleaginous vehicles.

Injectable preparations may be adapted for bolus injection or continuous infusion. Such preparations are conveniently presented in unit dose or multi-dose containers which are sealed after introduction of the formulation until required for use. Alternatively, an active compound may be in powder form which is constituted with a suitable vehicle, such as sterile, pyrogen-free water, before use.

An active compound may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing an active compound and desirably having a diameter in the range of 0.5 to 7 microns are delivered in the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active compound, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active compound is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active compound may be in the form of a solution or suspension for use in an atomizer or nebuliser whereby an accelerated airstream or ultrasonic agitation is employed to produce a fine droplet mist for inhalation.

Formulations suitable for nasal administration include preparations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

Pharmaceutically acceptable carriers are well known to those skilled in the art and include, but are not limited to, 0.1 M and preferably 0.05 M phosphate buffer or 0.8%. saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Formulations suitable for topical formulation may be provided for example as gels, creams or ointments. Such preparations may be applied e.g. to a wound or ulcer either directly spread upon the surface of the wound or ulcer or carried on a suitable support such as a bandage, gauze, mesh or the like which may be applied to and over the area to be treated.

Liquid or powder formulations may also be provided which can be sprayed or sprinkled directly onto the site to be treated, e.g. a wound or ulcer. Alternatively, a carrier such as a bandage, gauze, mesh or the like can be sprayed or sprinkle with the formulation and then applied to the site to be treated.

According to a further aspect of the invention, there is provided a process for the preparation of a pharmaceutical or veterinary composition as described above, the process comprising bringing the active compound(s) into association with the carrier, for example by admixture.

In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of general formula (I) in conjunction or association with a pharmaceutically or veterinarily acceptable carrier or vehicle.

### SALTS/ESTERS

The compounds of the invention can be present as salts or esters, in particular pharmaceutically and veterinarily acceptable salts or esters.

Pharmaceutically acceptable salts of the compounds of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. hydrohalic acids such as hydrochloride, hydrobromide and hydroiodide, sulphuric acid, phosphoric acid sulphate, bisulphate, hemisulphate, thiocyanate, persulphate and sulphonic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Salts which are not pharmaceutically or veterinarily acceptable may still be valuable as intermediates.

Preferred salts include, for example, acetate, trifluoroacetate, lactate, gluconate, citrate, tartrate, maleate, malate, pantothenate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-naphthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

### ENANTIOMERSITAUTOMERS

In all aspects of the present invention previously discussed, the invention includes, where appropriate all enantiomers, diastereoisomers and tautomers of the compounds of the invention. The person skilled in the art will recognise compounds that possess optical properties (one or more chiral carbon atoms) or tautomeric characteristics. The corresponding enantiomers and/or tautomers may be isolated/prepared by methods known in the art.

Enantiomers are characterised by the absolute configuration of their chiral centres and described by the R- and S-sequencing rules of Cahn, Ingold and Prelog. Such conventions are well known in the art (e.g. see 'Advanced Organic Chemistry', 3rd edition, ed. March, J., John Wiley and Sons, New York, 1985).

Compounds of the invention containing a chiral centre may be used as a racemic mixture, an enantiomerically enriched mixture, or the racemic mixture may be separated using well-known techniques and an individual enantiomer may be used alone.

### STEREO AND GEOMETRIC ISOMERS

Some of the compounds of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present invention also includes all suitable isotopic variations of the agent or a pharmaceutically acceptable salt thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O ¹⁸O, ³¹P, ³²P ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. For example, the invention includes compounds of general formula (I) where any hydrogen atom has been replaced by a deuterium atom. Isotopic variations of the agent of the present invention and pharmaceutically acceptable salts thereof of this invention can generally be prepared by conventional procedures using appropriate isotopic variations of suitable reagents.

### PRODRUGS

The invention further includes the compounds of the present invention in prodrug form, i.e. covalently bonded compounds which release the active parent drug according to general formula (I) *in vivo.* Such prodrugs are generally compounds of the invention wherein one or more appropriate groups have been modified such that the modification may be reversed upon administration to a human or mammalian subject. Reversion is usually performed by an enzyme naturally present in such subject, though it is possible for a second agent to be administered together with such a prodrug in order to perform the reversion *in vivo.* Examples of such modifications include ester (for example, any of those described above), wherein the reversion may be carried out be an esterase etc. Other such systems will be well known to those skilled in the art.

### SOLVATES

The present invention also includes solvate forms of the compounds of the present invention. The terms used in the claims encompass these forms.

### POLYMORPHS

The invention further relates to the compounds of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

### ADMINISTRATION

The pharmaceutical compositions of the present invention may be adapted for rectal, nasal, intrabronchial, topical (including buccal and sublingual), vaginal or parenteral (including subcutaneous, intramuscular, intravenous, intraarterial and intradermal), intraperitoneal or intrathecal administration. Preferably the formulation is an orally administered formulation. The formulations may conveniently be presented in unit dosage form, i.e., in the form of discrete portions containing a unit dose, or a multiple or sub-unit of a unit dose. By way of example, the formulations may be in the form of tablets and sustained release capsules, and may be prepared by any method well known in the art of pharmacy.

Formulations for oral administration in the present invention may be presented as: discrete units such as capsules, gellules, drops, cachets, pills or tablets each containing a predetermined amount of the active agent; as a powder or granules; as a solution, emulsion or a suspension of the active agent in an aqueous liquid or a non-aqueous liquid; or as an oil-in-water liquid emulsion or a water-in-oil liquid emulsion; or as a bolus etc. Preferably, these compositions contain from 1 to 250 mg and more preferably from 10-100 mg, of active ingredient per dose.

For compositions for oral administration (e.g. tablets and capsules), the term acceptably carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Other forms of administration comprise solutions or emulsions which may be injected intravenously, intraarterially, intrathecally, subcutaneously, intradermally, intraperitoneally or intramuscularly, and which are prepared from sterile or sterilisable solutions. Injectable forms typically contain between 10 - 1000 mg, preferably between 10 - 250 mg, of active ingredient per dose.

The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

### DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

In accordance with this invention, an effective amount of a compound of general formula (I) may be administered to inhibit the kinase implicated with a particular condition or disease. Of course, this dosage amount will further be modified according to the type of administration of the compound. For example, to achieve an "effective amount" for acute therapy, parenteral administration of a compound of general formula (I) is preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg, in a manner to maintain the concentration of drug in the plasma at a concentration effective to inhibit a kinase. The compounds may be administered one to four times daily at a level to achieve a total daily dose of about 0.4 to about 400 mg/kg/day. The precise amount of an inventive - compound which is therapeutically effective, and the route by which such compound is best administered, is readily determined by one of ordinary skill in the art by comparing the blood level of the agent to the concentration required to have a therapeutic effect. The compounds of this invention may also be administered orally to the patient, in a manner such that the concentration of drug is sufficient to achieve one or more of the therapeutic indications disclosed herein. Typically, a pharmaceutical composition containing the compound is administered at an oral dose of between about 0.1 to about 50 mg/kg in a manner consistent with the condition of the patient. Preferably the oral dose would be about 0.5 to about 20 mg/kg.

No unacceptable toxicological effects are expected when compounds of the present invention are administered in accordance with the present invention. The compounds of this invention, which may have good bioavailability, may be tested in one of several biological assays to determine the concentration of a compound which is required to have a given pharmacological effect.

### COMBINATIONS

In a particularly preferred embodiment, the one or more compounds of the invention are administered in combination with one or more other active agents, for example, existing drugs available on the market. In such cases, the compounds of the invention may be administered consecutively, simultaneously or sequentially with the one or more other active agents.

Drugs in general are more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease the emergence of resistance.

Beneficial combinations may be suggested by studying the inhibitory activity of the test compounds with agents known or suspected of being valuable in the treatment of a particular disorder. This procedure can also be used to determine the order of administration of the agents, i.e. before, simultaneously, or after delivery. Such scheduling may be a feature of all the active agents identified herein.

### ASSAY

A further aspect of the invention relates to the use of a compound as described above in an assay for identifying further candidate compounds capable of inhibiting TBK1.

Preferably, the assay is a competitive binding assay.

More preferably, the competitive binding assay comprises contacting a compound of the invention with TBK1,
and a candidate compound and detecting any change in the interaction between the compound according to the invention and the kinase.

Preferably, the candidate compound is generated by conventional SAR modification of a compound of the invention.

As used herein, the term "conventional SAR modification" refers to standard methods known in the art for varying a given compound by way of chemical derivatisation.

Thus, in one aspect, the identified compound may act as a model (for example, a template) for the development of other compounds. The compounds employed in such a test may be free in solution, affixed to a solid support, borne on a cell surface, or located intracellularly. The abolition of activity or the formation of binding complexes between the compound and the agent being tested may be measured.

The assay of the present invention may be a screen, whereby a number of agents are tested. In one aspect, the assay method of the present invention is a high through-put screen.

This. invention also contemplates the use of competitive drug screening assays in which neutralising antibodies capable of binding a compound specifically compete with a test compound for binding to a compound.

Another technique for screening provides for high throughput screening (HTS) of agents having suitable binding affinity to the substances and is based upon the method described in detail in WO 84/03564.

It is expected that the assay methods of the present invention will be suitable for both small and large-scale screening of test compounds as well as in quantitative assays.

Preferably, the competitive binding assay comprises contacting a compound of the invention with a kinase in the presence of a known substrate of said kinase and detecting any change in the interaction between said kinase and said known substrate.

A further aspect of the invention provides a method of detecting the binding of a ligand to a kinase, said method comprising the steps of:
(i) contacting a ligand with a kinase in the presence of a known substrate of said kinase;
(ii) detecting any change in the interaction between said kinase and said known substrate;
and wherein said ligand is a compound of the invention.

One aspect of the invention relates to a process comprising the steps of:
(a) performing an assay method described hereinabove;
(b) identifying one or more ligands capable of binding to a ligand binding domain; and
(c) preparing a quantity of said one or more ligands.

Another aspect of the invention provides a process comprising the steps of:
(a) performing an assay method described hereinabove;
(b) identifying one or more ligands capable of binding to a ligand binding domain; and
(c) preparing a pharmaceutical composition comprising said one or more ligands.

Another aspect of the invention provides a process comprising the steps of:
(a) performing an assay method described hereinabove;
(b) identifying one or more ligands capable of binding to a ligand binding domain;
(c) modifying said one or more ligands capable of binding to a ligand binding domain;
(d) performing the assay method described hereinabove;
(e) optionally preparing a pharmaceutical composition comprising said one or more ligands.

The invention also relates to a ligand identified by the method described hereinabove.

Yet another aspect of the invention relates to a pharmaceutical composition comprising a ligand identified by the method described hereinabove.

Another aspect of the invention relates to the use of a ligand identified by the method described hereinabove in the preparation of a pharmaceutical composition for use in the treatment of one or more disorders selected from cancer, septic shock, diseases of the eye, including Primary open Angle Glaucoma (POAG), hyperplasia, rheumatoid arthritis, autoimmune diseases, artherosclerosis, retinopathy, osteoarthritis, fibrotic diseases, endometriosis and chronic inflammation.

The above methods may be used to screen for a ligand useful as an inhibitor of one or more kinases.

Compounds of general formula (I) are useful both as laboratory tools and as therapeutic agents. In the laboratory certain compounds of the invention are useful in establishing whether a known or newly discovered kinase contributes a critical or at least significant biochemical function during the establishment or progression of a disease state, a process commonly referred to as 'target validation'.

### SYNTHESIS

A further aspect of the invention relates to a process for preparing a compound of formula VII, wherein R¹¹ and R² are as defined above, said process comprising the steps of: 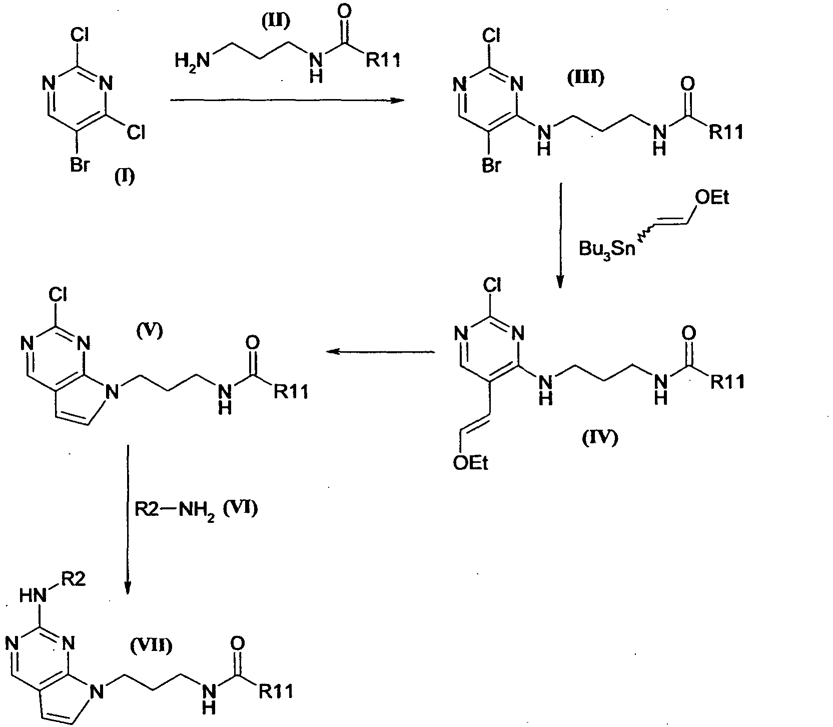
(i) reacting 5-bromo-2,4-diclooropyrimidine (I) with an amine of formula II to give a compound of formula III;
(ii) reacting said compound of formula III with ethoxyvinyltin to give a compound of formula IV;
(iii) cyclising said compound of formula IV to form a compound of formula V;
(iv) reacting said compound of formula V with an amine of formula VI to give a compound of formula VII.

Scheme 1 illustrates the conversion of compounds with formula I to compounds with formula VII wherein R¹¹ and R² are as defined above. Compounds of formula (II) and compounds of formula (VI) are commercially available, known in the literature or are readily obtainable by the skilled person by following standard chemical procedures.

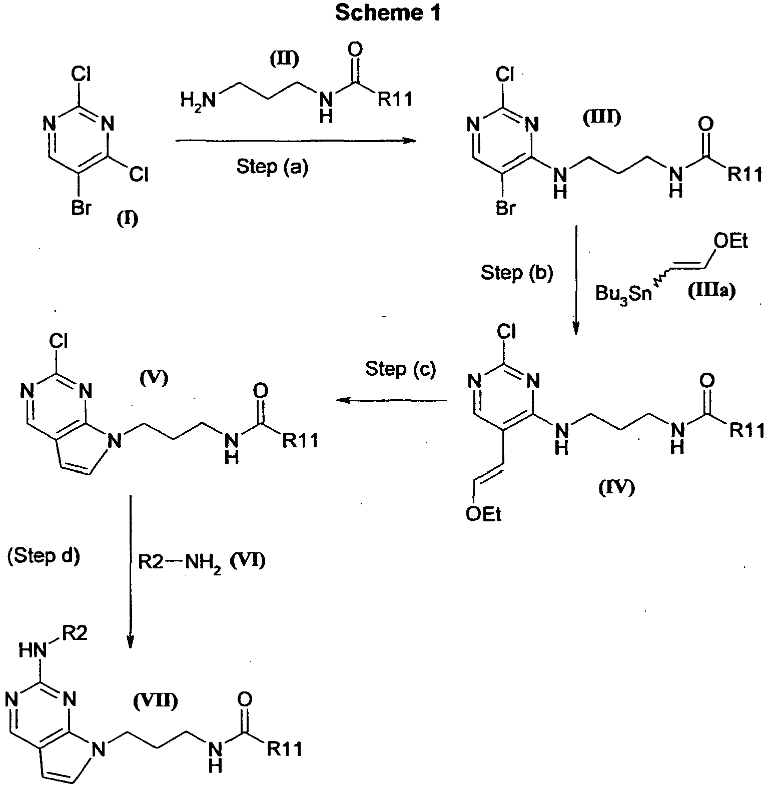

### Step (a)

This step involves the displacement of a chloride, in formula (I) with an amino group of formula (II) to give compounds with formula (III), preferably in a suitable solvent (such as isopropanol or dioxane), preferably in the presence of an organic base (such as triethylamine) preferably at temperatures in the range of 0-80 °C for reaction times of up to 24 h.

Preferred conditions: 1eq. of formula (I), 1.2eq. of formula (II), 5eq. of triethylamine in dioxane at room temperature for 6 h.

### (Step (b)

This step involves a cross-coupling coupling of an ethoxyvinylstannane with formula (IIIa) to a 5-bromopyrimidine with formula (III) to give a 5-(2-ethoxyvinyl)pyrimidine with formula (IV). The reaction is preferably carried in a suitable solvent (e.g. toluene) preferably in the presence of a suitable palladium catalyst (e.g. Pd(PPh₃)₄) preferably at temperatures up to the reflux temperature of the solvent under an inert atmosphere (e.g. nitrogen or argon).

Preferred conditions: 1eq. of formula (III), 1.2eq. of formula (IIIa), 0.05eq. of Pd(PPh₃)₄ in toluene at reflux for 18 h.

### Step (c)

This step involves the intramolecular cyclization of compounds with formula (IV) to give compounds with formula (V). The reaction preferably takes place in the presence of a Brønsted acid (such as glacial acetic acid) in a suitable solvent, preferably heating up to the reflux temperature of the solvent. For this reaction, glacial acetic acid can be used as the solvent.

Preferred conditions: Formula (IV) is heated in glacial acetic acid under reflux for 1 h.

### Step (d)

This step involves reaction of a 2-chloro-pyrrolopyrimidine derivative with formula (V) with an amine with formula (VI) to give compounds with formula (VII), preferably in the presence of a palladium source (e.g. Pd(OAc)₂ or Pd₂(dba)₃), a suitable ligand (e.g. bis(diphenylphosphino)-9,9-dimethylxanthene) and a suitable base (e.g. Cs₂CO₃ or sodium *tert*-butoxide) preferably in a suitable solvent (e.g. dioxane). The reaction is preferably carried out at around the reflux temperature of the solvent under an inert atmosphere.

Preferred method: 1 eq. of formula (V), 1.3eq. of amine (VI), 0.05eq. of Pd₂(dba)₃, 0.08eq. of bis(diphenylphosphino)-9,9-dimethylxanthene, 2.8eq. of sodium *tert*-butoxide in dioxane at 105 °C for 2 h.

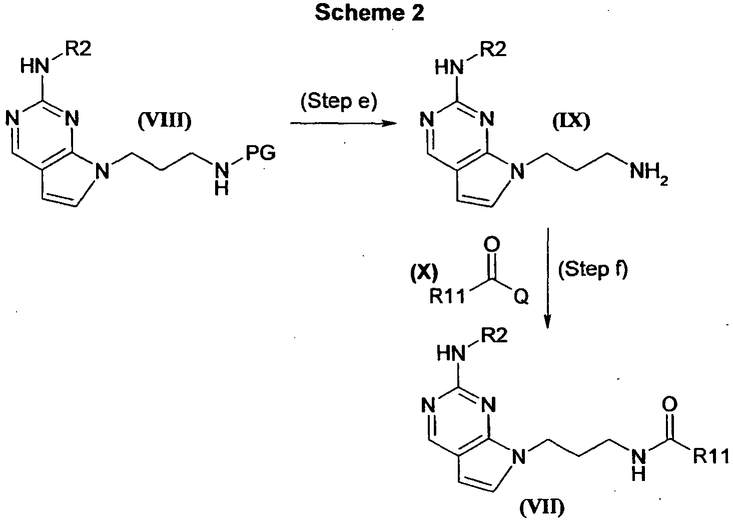

Alternatively, compounds of formula (VII) can be prepared according to Scheme 2, wherein PG represents a protecting group (such as but not limited to benzyloxycarbonyl), Q refers to a leaving group such as a halogen, or an -OH group R11 and R2 are as defined previously.

### Ste (e)

Compounds of formula (IX) can be prepared from compounds with formula (VIII) (wherein PG is benzyloxycarbonyl) under hydrogenation conditions preferably in the presence of a suitable catalyst (such as 10% palladium on carbon). The hydrogen source can be either hydrogen gas or can be generated *in situ* using a transfer hydrogenation reagent (such as ammonium formate).
Preferred conditions:
Formula (VIII) is stirred in ethyl acetate / ethanol in the presence of 10% Pd/C under an atmosphere of hydrogen at room temperature for 18h.

### Step (f)

Compounds with formula (VII) can be prepared by reacting formula (IX) with formula (X). It will be appreciated by a person shilled in the art that there are many methods of carrying out transformations of this type where formula (X) is a carboxylic acid. For example an OH group can be activated *in situ* in the form of a mixed anhydride, or using one of many coupling reagents, such as DCC or HATU.
Preferred conditions:
1.2 eq of a carboxylic acid of formula (X) was treated with 1.3 eq of isobutyl chloroformate in the presence of 1.3 eq of triethylamine in DMF at room temperature for 15 min. Then 1eq of formula (IX) was added and stirring continued for 3 h at room temperature.

The present invention is further described by way of the following non-limiting examples.

### EXAMPLES

### Materials and Methods

### Source and purification of kinases

All protein kinases were of human origin and encoded full length proteins, unless indicated otherwise. They were either expressed as glutathione S-transferase (GST) fusion proteins in *Escherichia Coli* or as hexahistidine (His6)-tagged proteins in insect Sf21 cells. GST fusion proteins were purified by affinity chromatography on glutathione-Sepharose, and His6-tagged proteins on nickel/nitriloacetate-agarose. The procedures for expressing some of the protein kinases used herein have been detailed previously.^{8,9} The following sections outline the DNA vectors synthesised and the procedures used to express and purifying protein kinases that have not been reported previously.

### Expression in E.coli

The following proteins were expressed in E.coli:- CHK2[5-543], cyclin-dependent protein kinase 2 (CDK2), MAP kinase-interacting kinase 2 (MNK2), extra-cellular signal-regulated kinase 1 (ERK1).

### Expression in Sf21 cells

The following kinases were expressed in Sf21 cells:Aurora B and Aurora C, extra-cellular signal-regulated kinase 8 (ERK8), microtubule affinity regulating kinase 3 (MARK3), protein kinase Bα[118-480][S473D], protein kinase Bβ (PKBβ[120-481][S474D], 3-phosphoinositide-dependent protein kinase-1 [52-556] (PDK1[52-556], IKKε, TBK1,

### Activation of protein kinases

In order to produce activated forms of Aurora B and Aurora C, insect Sf21 cells were incubated for 1 h with the protein phosphatase inhibitor okadaic acid (50 nM). JNK isoforms were activated with MKK4 and MKK7, MNK2 with p38α MAPkinase; PKBα, PKBβ, with PDK1, and ERK1 with MKK1. To activate CDK2, bacterial pellets expressing cyclin A2 and CDK2 were mixed together, lyse and purified on glutathione Sepharose. The GST-tags were removed by cleavage with PreScission protease and the CDK2-cyclin A2 complex purified by chromatography on SP-Sepharose. It was then activated with CAK1/CDK7 followed by chromatography on nickel-nitrilotriacetate agarose to remove CAK1/CDK7, which binds to this column by virtue of its C-terminal His6 tag. All the other protein kinases were active as expressed.

### Protein kinase assays

All assays (25.5µl) were carried out at room temperature (21 °C) and were linear with respect to time and enzyme concentration under the conditions used. Assays were performed for 30 min using Multidrop Micro reagent dispensers (Thermo Electron Corporation, Waltham, MA 02454, USA) in a 96- well format. The concentration of magnesium acetate in the assays was 10 mM and the [γ-33P] ATP (800 cpm / pmol) was used at 5, 20 or 50 µM as indicated, in order to be at or below the Km for ATP for each enzyme. Protein kinases assayed at 5 µM ATP were:- ERK1, ERK8, PKBα, MARK3, Aurora C. Protein kinases assayed at 20 µM ATP were:- JNK1, PDK1,CHK1, CHK2, CDK2 and Aurora B. Protein kinases assayed at 50 µM ATP were:-MNK2, IKKepsilon and TBK1.

The assays were initiated with MgATP, stopped by addition of 5 µl of 0.5 M orthophosphoric acid and spotted on to P81 filterplates using a unifilter harvester (PerkinElmer, Boston, MA 02118, USA). The IC50 values of inhibitors were determined after carrying out assays at 10 different concentrations of each compound.

ERK1 and ERK8 were both assayed against myelin basic protein (MBP, 0.33mg/ml). MARK3 was assayed against the peptide KKKVSRSGLYRSPSMPENLNRPR (300µM), MNK2 against the elF4E protein (0.5mg/ml). PKBβ was assayed against the peptide GRPRTSSFAEGKK (30µM). TBK1 were assayed against (AKPKGNKDYHLQTCCGSLAYRRR) (300 µM). The substrates used for other protein kinases were described previously.^{8,9}

Unless stated otherwise, enzymes were diluted in 50 mM Tris/HCl pH 7.5, 0.1 mM EGTA, 1 mg/ml BSA, 0.1% (v/v) 2-mercaptoethanol and assayed in 50 mM Tris/HCl pH 7.5, 0.1 mM EGTA, 0.1% (v/v) 2-mercaptoethanol.

### General procedures for synthesis of compounds

### Chromatography

Preparative high pressure liquid chromatography was carried out using apparatus made by Agilent. The apparatus is constructed such that the chromatography (column: either a 30 x 100 mm (10 µm) C-18 Phenomenex Gemini column at a flow rate of 50 ml/min, or a 21.2 x 100 mm (5 µm) C-18 Phenomenex Gemini column at a flow rate of 20 ml/min) is monitored by a multi-wavelength UV detector (G1365B manufactured by Agilent) and an MM-ES+APCI mass spectrometer (G-1956A, manufactured by Agilent) connected in series, and if the appropriate criteria are met the sample is collected by an automated fraction collector (G1364B manufactured by Agilent). Collection can be triggered by any combination of UV or mass spectrometry or can be based on time. Typical conditions for the separation process are as follows: The gradient is run over a 10 minute period (gradient at start: 10% methanol and 90% water, gradient at finish: 100% methanol and 0% water; as buffer: either 0.1 % trifluoroacetic acid is added to the water (low pH buffer), or ammonium bicarbonate (10 mmol/l) and 35% ammonium hydroxide (1.6 ml/l) is added to the water (high pH buffer). It will be appreciated by those skilled in the art that it may be necessary or desirable to modify the conditions for each specific compound, for example by changing the solvent composition at the start or at the end, modifying the solvents or buffers, changing the run time or changing the flow rate.

Flash chromatography refers to silica gel chromatography and carried out using an SP4 MPLC system (manufactured by Biotage); pre-packed silica gel cartridges (supplied by Biotage); or using conventional glass column chromatography.

### Analytical Methods

¹H Nuclear magnetic resonance (NMR) spectroscopy was carried out using an ECX400 spectrometer (manufactured by JEOL) in the stated solvent at around room temperature unless otherwise stated. In all cases, NMR data were consistent with the proposed structures. Characteristic chemical shifts (δ) are given in parts-per-million using conventional abbreviations for designation of major peaks: e.g. s, singlet; d, doublet; t, triplet; q, quartet; dd, doublet of doublets; br, broad. Mass spectra were recorded using a MM-ES+APCI mass spectrometer (G-1956A, manufactured by Agilent). Where thin layer chromatography (TLC) has been used it refers to silica gel TLC using silica gel MK6F 60A plates, R_{f} is the distance travelled by the compound divided by the distance travelled by the solvent on a TLC plate.

### Compound preparation

Where the preparation of starting materials is not described, these are commercially available, known in the literature, or readily obtainable by those skilled in the art using standard procedures. Where it is stated that compounds were prepared analogously to earlier examples or intermediates, it will be appreciated by the skilled person that the reaction time, number of equivalents of reagents and temperature can be modified for each specific reaction and that it may be necessary or desirable to employ different work-up or purification techniques. Where reactions are carried out using microwave irradiation, the microwave used is an Initiator 60 supplied by Biotage. The actual power supplied varies during the course of the reaction in order to maintain a constant temperature.

### Abbreviations

- DCM: = Dichloromethane
- DMF: = N,N-Dimethylformamide
- THF: = Tetrahydrofuran
- MeOH: = Methanol
- TFA: = Trifluoroacetic acid
- Xantphos: = 4,5-Bis(diphenylphosphino)-9,9-dimethylxanthene
- HATU: = N,N,N',N'-Tetramethyl-O-(7-azabenzotriazol-1-yl)uronium hexafluorophospate
- EDCI: = 1,3-Propanediamine-N3-(ethylcarbonimidoyl)-N1,N1-dimethyl hydrochloride
- DCC: = 1,3-Dicyclohexylcarbodiimide
- Pd₂(dba)₃: = Tris(dibenzylideneacetone)dipalladium(0)
- TEA: = Triethylamine
- rm: = Reaction mixture
- rt: = Room temperature
- AcOH: = Acetic acid
- IPA: = Isopropanol
- DIPEA: = N,N-diisopropylethylamine
- TBSMSCl: = *Tert*-butyldimethylsilyl chloride
- MeCN: = Acetonitrile
- NH3: = Ammonia
- EtOH: = Ethanol
- EtOAc: = Ethyl acetate
- NCS: = *N*-chlorosuccinimide
- LCMS: = Mass spectrometry directed high pressure liquid chromatography
- UV: = Ultraviolet
- SCX: = Strong cation exchange

### Intermediate 1

### Cyclobutanecarboxylic acid [3-(2-chloro-pryrrolo[2,3-d]pyrimidin-7-yl)-propyl]-amide

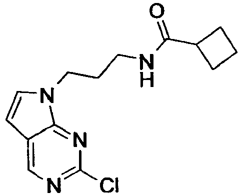

### Step 1

### Cyclobutanecarboxylic acid (3-amino-propyl)-amide hydrochloride

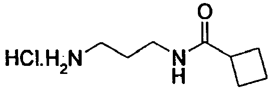

A solution of (3-amino-propyl)-carbamic acid *tert*-butyl ester (2.00 g, 11.5 mmol) in CH₂Cl₂ (50 mL) at 0 °C was treated with triethylamine (4.0 mL, 28.8 mmol) and then dropwise with cyclobutanecarbonyl chloride (1.57 mL, 13.77 mmol). After 1 h at 0 °C, the reaction was allowed to warm to room temperature and stirred for 6 h. It was then washed with water (100 mL) and brine (100 mL), dried (MgSO₄) and concentrated in *vacuo* to give the crude product, which was re-dissolved in 4M HCl in dioxane (30 mL) and stirred at room temperature for 1 h. Evaporation of the solvents *in* vacuo gave the product as a viscous oil (2.55 g). δ_{H} (400 MHz, d₆-DMSO) 8.03 (s, br, 2H), 7.93 (t, J = 5.5 Hz, 1 H), 3.08 (q, J = 6.4 Hz, 2H), 2.99 (quintet, J = 7.8 Hz, 1 H), 2.72 (q, J = 6.4 Hz, 2H), 2.12-1.63 (m, 8H).

### Step 2

### Cyclobutanecarboxylic acid [3-(5-bromo-2-chloro-pyrimidin-4-ylamino)-propyl]-amide

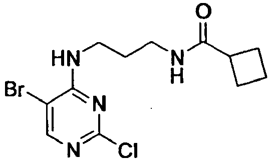

To a suspension of cyclobutanecarboxylic acid (3-amino-propyl)-amide hydrochloride (2.55 g, 13.23 mmol) in dioxane (50 mL) was added triethylamine (9.22 mL, 66.2 mmol) followed by 2,4-dichloro-5-bromopyrimidine (2.51 g, 11.03 mmol), and the reaction was stirred at room temperature for 6 h. The dioxane was removed *in vacuo* and the residue partitioned between water (40 mL) and EtOAc (40 mL). The aqueous layer was reextracted with EtOAc (2 x 30 mL) and the combined organic extracts washed with water (70 mL) and brine (70 mL), dried (MgSO₄) and concentrated *in vacuo.* Purification by flash chromatography using a Biotage SP4 (40-60% petroleum ether-EtOAc gradient) gave the product as a white solid (2.71 g, 71%). δ_{H} (400 MHz, d₆-DMSO) 8.23 (s, 1H), 7.75 (t, J = 5.5 Hz, 1 H), 7.69 (t, J = 5.5 Hz, 1H), 3.33 (q, J = 6.4 Hz, 2H), 3.05 (q, J = 6.4 Hz, 2H), 2.96 (quintet, J = 8.2 Hz, 1H), 2.16-1.59 (m, 8H); m/z (ES+APCl)⁺: 347 / 349 / 351 [M+H]⁺.

### Step 3

### Cyclobutanecarboxylic acid {3-[2-chloro-5-((E)-2-ethoxy-vinyl)-pyrimidin-4-ylamino]-propyl}-amide

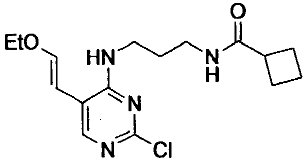

A solution of (Z)-1-ethoxy-2-(tributylstannyl)ethane (1.62 g, 4.49 mmol) and cyclobutanecarboxylic acid [3-(5-bromo-2-chloro-pyrimidin-4-ylamino)-propyl]-amide (1.30 g, 3.74 mmol) in toluene (20 mL) was degassed for 10 minutes and then Pd(Ph₃P)₄ (216 mg, 0.19 mmol) was added. The mixture was evacuated and backfilled with nitrogen (3 cycles) and then heated at reflux for 18 h. Concentration *in vacuo* directly onto silica and purification by flash chromatography using a Biotage SP4 (40-60% petroleum ether-EtOAc 1:1 gradient) gave the product as a pale yellow solid (582 mg, 46%). δ_{H} (400 MHz, CDCl₃) 7.81 (d, J = 0.9 Hz, 1H), 6.76 (d, J = 12.8 Hz, 1H), 6.14 (s, br, 1H), 6.01 (t, J = 6.0 Hz, 1H), 5.48 (d, J = 12.8 Hz, 1H), 3.98 (q, J = 6.9 Hz, 2H), 3.53 (q, J = 6.0 Hz, 2H), 3.30 (q, J = 6.4 Hz, 2H), 3.07 (quintet of doublets, J = 8.7, 0.9 Hz, 1 H), 2.34-1.83 (m, 8H), 1.36 (t, J = 6.5 Hz, 3H); m/z (ES+APCI)⁺: 339 / 341 [M+H]⁺.

### Step 4

### Cyclobutanecarboxylic acid [3-(2-chloro-pyrrolo[2,3-d]pyrimidin-7-yl)-propyl]-amide

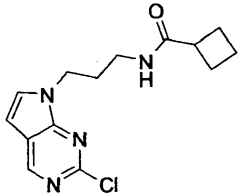

A solution of cyclobutanecarboxylic acid {3-[2-chloro-5-((E)-2-ethoxy-vinyl)-pyrimidin-4-ylamino]-propyl}-amide described in Step 3 (266 mg, 0.78 mmol) in glacial acetic acid (6 mL) was stirred at reflux for 1 h. The acetic acid was then removed *in vacuo* and the residue partitioned between saturated aqueous NaHCO₃ (10 mL) and CH₂Cl₂ (10 mL). The aqueous layer was extracted with CH₂Cl₂ (2 x 5 mL) and EtOAc (5 mL) and the combined organic extracts dried (MgSO₄), concentrated *in vacuo* and purified by flash chromatography using the Biotage SP4 (SP4 - 12 M - petroleum ether-EtOAc, 1:1 gradient) to give the product (131 mg, 57%) as a colourless solid; δ_{H} (400 MHz, CDCl₃) 8.83 (s, 1H), 7.29 (d, J = 3.7 Hz, 1H), 6.62 (d, J = 3.7 Hz, 1 H), 6.45 (s, br, 1 H), 4.30 (dd, J = 6.4, 6.0 Hz, 2H), 3.12 (q, J = 6.0 Hz, 2H), 3.10 (quintet, J = 8.7 Hz, 1H), 2.38-1.88 (m, 8H); m/z (ES+APCI)⁺: 293 / 295 [M+H]⁺.

### Intermediate 2

### Cyclopentanecarboxylic acid [3-(2-chloro-pyrrolo[2,3-d]pyrimidin-7-yl)-propyl]amide

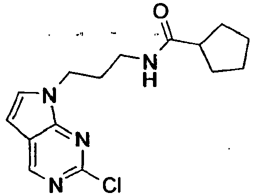

Prepared analogously to Intermediate 1, except cyclopentanecarbonyl chloride was used in Step 1. The product was isolated as a white solid. δ_{H} (400 MHz, CDCl₃) 8.83 (s, 1H), 7.28 (d, J = 3.2 Hz, 1 H), 6.62 (d, J = 3.7 Hz, 1 H), 6.54 (s, br, 1 H), 4.30 (app. t, J = 6.4 Hz, 2H), 3.12 (q, J = 6.4 Hz, 2H), 2.64 (quintet, J = 8.2 Hz, 1H), 2.04-1.60 (m, 10H); m/z (ES+APCI)⁺: 307 / 309 [M+H]⁺.

### Intermediate 3

### Thiophene-2-carboxylic acid [3-(2-chloro-pyrrolo[2,3-d]pyrimidin-7-yl)-propyl]-amide

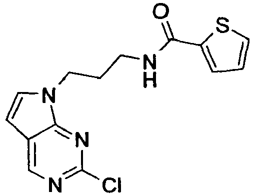

Prepared analogously to Intermediate 1, except thiophene-2-carbonyl chloride was used in Step 1. The product was isolated as a white solid. ¹H NMR (400 MHz, CDCl₃) δH ppm 1.98 - 2.15 (m, 2 H), 3.27 (m, 2 H), 4.31 - 4.40 (m, 2 H), 6.63 (d, *J*=3.66 Hz, 1 H), 7.13 (dd, J=5.04, 3.66 Hz, 1 H), 7.20 - 7.28 (m, 1 H), 7.40 (br. s, 1 H), 7.51 (dd, J=5.04, 0.92 Hz, 1 H), 7.73 - 7.81 (m, 1 H), 8.83 (s, 1 H); m/z (ES+APCI)⁺: 321 / 323 [M+H]⁺.

### Intermediate 4

### Cyclobutanecarboxylic acid [3-(2,5-dichloro-pyrrolo[2,3-d]pyrimidin-7-yl)-propyl]-amide

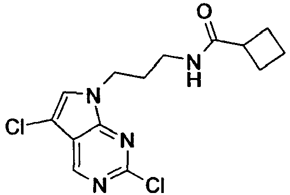

A solution of Intermediate 1 (56 mg, 0.19 mmol) in THF (1.5 mL) at room temperature was treated with NCS (28 mg, 0.21 mmol) and stirred at room temperature for 18 h. The mixture was concentrated *in vacuo* directly onto silica and purification by flash chromatography using the Biotage SP4 (petroleum ether b.p. 40-60°C / EtOAc gradient) gave the product as a white solid (60 mg, 96%); δ_{H} (400 MHz, CDCl₃) 8.80 (s, 1 H), 7.25 (s, 1H), 6.32 (s, br, 1 H), 4.23 (t, J = 6.4 Hz, 2H), 3.11 (q, J = 6.4 Hz, 2H), 3.06 (quintet of doublets, J = 8.7, 0.9 Hz, 1H), 2.32-1.83 (m, 8H); m/z (ES+APCI)`: 327 / 329 / 331 [M+H]⁺.

### Intermediate 5

### Thiophene-2-carboxylic acid [3-(2,5-dichloro-pyrrolo[2,3-d]pyrimidin-7-yl)-propyl]-amide

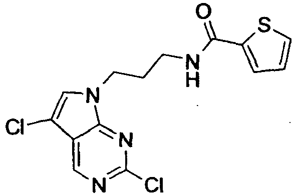

Prepared analogously to Intermediate 4 from Intermediate 3 (50 mg, 0.156 mmol) to give the product as a cream solid (45 mg, 81%). ¹H NMR (400 MHz, DMSO-d₆) δH ppm 2.01 (m, 2 H), 3.15 - 3.22 (m, 2 H), 4.22 (t, *J*=6.87 Hz, 2 H), 7.10 (dd, *J*=5.0, 3.66 Hz, 1 H), 7.66 (dd, *J*=3.7, 1.4 Hz, 1 H), 7.71 (dd, *J*=5.0, 0.9 Hz, 1 H), 7.97 (s, 1 H), 8.46 (t, *J*=5.5 Hz, 1 H), 8.91 (s, 1 H).

### Intermediate 6

### Cyclobutanecarboxylic acid [3-(5-bromo-2-chloro-pyrrolo[2,3-d]pyrimidin-7-yl)-propyl]-amide

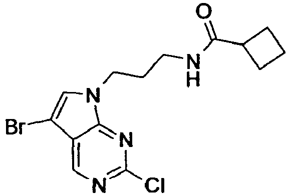

A solution of Intermediate 1 (50 mg, 0.17 mmol) in THF (1 mL) at room temperature was treated with NBS (1.1 eq, 0.19 mmol, 33 mg) and stirred for 1.5 h. The mixture was concentrated *in vacuo* directly onto silica and purification by flash chromatography using the Biotage SP4 (petroleum ether b.p. 40-60°C / EtOAc gradient) to give the product as a white solid (60 mg, 94%); δ_{H} (400 MHz, CDCl₃) 8.72 (s, 1H), 7.30 (s, 1H), 6.31 (s, br, 1H), 4.24 (dd, J = 6.4, 6.0 Hz, 2H), 3.12 (q, J = 6.4 Hz, 2H), 3.06 (quintet, J = 8.7 Hz, 1 H), 2.32-1.80 (m, 8H); m/z (ES+APCI): 371 / 373 / 375 [M+H]⁺.

### Intermediate 7

### [7-(3-Amino-propyl)-7H-pyrrolo[2,3-d]pyrimidin-2-yl]-(6-morpholin-4-yl-pyridin-3-yl)-amine

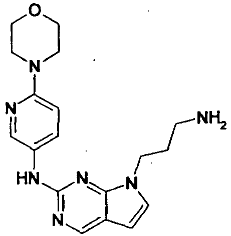

### Step 1

### [3-(5-Bromo-2-chloro-pyrimidin-4-ylamino)-propyl]-carbamic acid benzyl ester

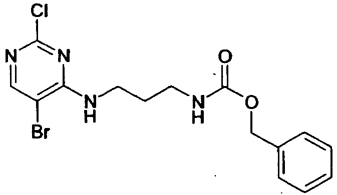

A solution of (3-amino-propyl)-carbamic acid benzyl ester hydrochloride (10.7g, 43.7 mmol) in isopropylalcohol (40 mL) was added to a stirred solution of DIPEA (30.5 mL, 0.18 mol) and 2,4-dichloro-5-bromopyrimidine (10 g, 43.9 mmol) in isopropylalcohol (160 mL) with ice cooling. The reaction was allowed to warm to room temperature then stirred at 60 °C for 18 h. The solvent was removed *in vacuo* and the residue partitioned between water (200 mL) and EtOAc (200 mL). The organic extract was dried (MgSO₄) and concentrated *in vacuo.* The crude material was purified by flash chromatography on the Biotage SP4 (gradient elution from 0 to 6% methanol in DCM) to give the desired product as a cream solid (17.3 g, 99%). ¹H NMR (400 MHz, DMSO-*d₆*) δH ppm 1.63 - 1.72 (m, 2 H), 2.99 - 3.07 (m, 2 H), 3.33 - 3.40 (m, 2 H), 5.01 (br. s, 2 H), 7.27 - 7.39 (m, 6 H), 7.70 (br. s, 1 H), 8.24 (s, 1 H); m/z (ES+APCI)⁺: 399.0 [M+H]⁺.

### Step 2

### {3-[2-Chloro-5-((Z)-2-ethoxy-vinyl)-pyrimidin-4-ylamino]-propyl}-carbamic acid benzyl ester

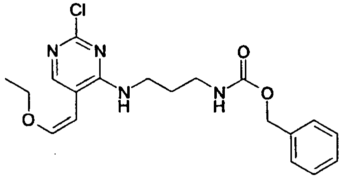

A solution of tributyl-((Z)-2-ethoxy-vinyl)-stannane (9.79 g, 27.1 mmol) and [3-(5-bromo-2-chloro-pyrimidin-4-ylamino)-propyl]-carbamic acid benzyl ester (Step 1), (9 g, 22.6 mmol) in toluene (120 mL) was degassed for 10 minutes and then Pd(PPh₃)₄ (1.3 g, 1.13 mmol) was added. The mixture was evacuated and backfilled with nitrogen (3 cycles) and then heated at reflux for 18 h. Concentration *in vacuo* directly onto silica and purification by flash chromatography on the Biotage SP4 (gradient elution from 15 to 80% ethyl acetate in petroleum ether) gave the desired product as a yellow oil (5.37 g, 61%). NMR shows approximately 10% impurity; used in the nest step without further purification. ¹H NMR (400 MHz, DMSO-*d₆*) δH ppm 1.24 (t, *J*=7.1 Hz, 3 H), 1.62 - 1.72 (m, 2 H), 3.00 - 3.07 (m, 2 H), 3.28 - 3.36 (m, 2 H), 4.00 (q, *J*=7.2 Hz, 2 H), 5.01 (br. m, 2 H), 5.13 (d, *J*=6.9 Hz, 1 H), 6.59 (d, *J*=7.3 Hz, 1 H), 7.26 - 7.41 (m, 7 H), 8.33 (s, 1 H). m/z (ES+APCl)⁺: 391 [M+H]⁺.

### Step 3

### [3-(2-Chloro-pyrrolo[2,3-d]pyrimidin-7-y/)-propy/]-carbamic acid benzyl ester

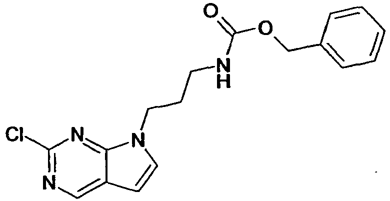

A solution of {3-[2-chloro-5-((Z)-2-ethoxy-vinyl)-pyrimidin-4-ylamino]-propyl}-carbamic acid benzyl ester (5.37g, 13.5 mmol) in glacial acetic acid (60 mL) was stirred at reflux for 1 h. The acetic acid was then removed *in vacuo* and the residue partitioned between saturated aqueous NaHCO₃ (40 mL) and DCM (80 mL). The aqueous layer was extracted with DCM (50 mL) and the combined organic extracts dried (MgSO₄), concentrated *in vacuo* and purified by flash chromatography on the Biotage SP4, eluting with 12 to 100% Ethyl acetate / Petroleum ether gradient). This gave the desired product as a yellow oil (2.95g, 62%). NMR shows approximately 8% of an impurity; used in the next step without further purification. ¹H NMR (400 MHz, CDCl₃) δH ppm 2.03 - 2.10 (m, 2 H), 3.13 - 3.21 (m, 2 H), 4.31 - 4.36 (m, 2 H), 5.13 (s, 2 H), 5.38 (br. s, 1 H), 6.63 (d, J=3.7 Hz, 1 H), 7.32 - 7.41 (m, 6 H), 8.82 - 8.86 (m, 1 H). m/z (ES+APCl)⁺: 345 [M+H]⁺.

### Step 4

### {3-[2-(6-Morpholin-4-yl-pyridin-3-ylamino)-pyrrolo[2,3-d]pyrimidin-7-yl]-propyl}-carbamic acid benzyl ester

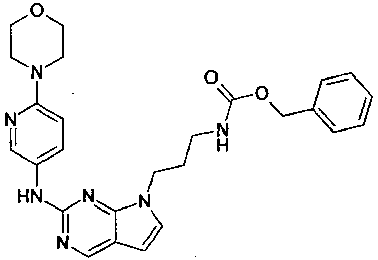

[3-(2-Chloro-pyrrolo[2,3-d]pyrimidin-7-yl)-propyl]-carbamic acid benzyl ester (1.13 g, 3.28 mmol), 6-morpholin-4-yl-pyridin-3-ylamine (704 mg, 3.93 mmol), palladium (II) acetate (44 mg, 0.012 mmol), 2,2'-bis(diphenylphosphino)-1,1'-binaphthyl (163 mg, 0.26 mmol) and cesium carbonate (3.19 g, 9.82 mmol) were combined with dioxane (25 mL), sealed and then purged with nitrogen gas. The reaction mixture was heated at 100 °C for 6 hours. The mixture was evaporated, then purified by preparative LCMS (high pH buffer) to give the desired product as a light brown solid (1.05 g, 66%). ¹H NMR (400 MHz, DMSO-*d₆*) δH ppm 1.88 - 1.97 (m, 2 H), 2.94 - 3.03 (m, 2 H), 3.30 - 3.34 (m, 4 H), 3.66 - 3.73 (m, 4 H), 4.08 - 4.15 (m, 2 H), 4.99 (s, 2 H), 6.40 (d, *J*=3.7 Hz, 1 H), 6.82 (d, *J*=9.2 Hz, 1 H), 7.25 (d, *J*=3.7 Hz, 1 H), 7.27 - 7.40 (m, 6 H), 8.07 (dd, *J*=8.7, 2.7 Hz, 1 H), 8.58 (d, *J*=2.7 Hz, 1 H), 8.63 (s, 1 H), 9.20 (br. s, 1 H). m/z (ES+APCI)⁺: 488 [M+H]⁺.

### Step 5

### [7-(3-Amino-propyl)-7H-pyrrolo[2,3-d]pyrimidin-2-yl]-(6-morpholin-4-yl-pyridin-3-yl)-amine

To a solution of {3-[2-(6-morpholin-4-yl-pyridin-3-ylamino)-pyrrolo[2,3-*d*]pyrimidin-7-yl]-propyl}-carbamic acid benzyl ester as described in step 4 (0.5 g, 1.03 mmol) in ethanol (25 mL) and ethyl acetate (10 mL) was added 10 % palladium on carbon (50 mg) and the reaction stirred under a hydrogen atmosphere for 18 h, which gave a partial reaction. The mixture was filtered through Celite, washing with further ethyl acetate (200 mL), the filtrate was evaporated and then re-dissolved in the ethanol / ethyl acetate (2.5:1), fresh 10 % palladium on carbon (50 mg) was added and the reaction stirred under a hydrogen atmosphere for a further 18 h. The mixture was again filtered through Celite, washing with ethyl acetate (200 mL) and the filtrate was evaporated to dryness. The crude product was purified by pre-absorbing onto silica, then eluting through a pre-packed silica cartridge (10 g) with 0 to 5% (0.1 % ammonia in methanol) in DCM gradient to give an off-white solid (230 mg, 63%). ¹H NMR (400 MHz, DMSO-d₆) δH ppm 1.79 - 1.87 (m, 2 H), 2.43 - 2.48 (m, 2 H), 3.27 - 3.53 (m, 6 H), 3.69 - 3.73 (m, 4 H), 4.14 - 4.19 (m, 2 H), 6.40 (d, *J*=3.7 Hz, 1 H), 6.83 (d, *J*=8.7 Hz, 1 H), 7.23 (d, *J*=3.7 Hz, 1 H), 8.05 (dd, *J*=9.2, 2.7 Hz, 1 H), 8.61 (d, *J*=2.7 Hz, 1 H), 8.63 (s, 1 H), 9.19 (br. s, 1 H). m/z (ES+APCl)⁺: 354 [M⁺H]⁺.

### Intermediate 8

### (4-Amino-phenyl)-morpholin-4-yl-methanone

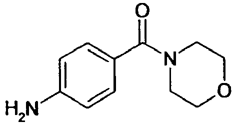

### Step 1

To a solution of morpholine (73 µl, 0.84 mmol) in DMF (5 mL) was added 4-*tert-*butoxycarbonylamino-benzoic acid (300 mg, 1.27 mmol), HATU (510 mg, 1.35 mmol) and DIPEA (0.88 mL, 5.06 mmol). The reaction mixture was evaporated then diluted with DCM (10 mL), partitioned with water (20 mL) and the layers separated. The aqueous layer was extracted with further DCM (2 x 20 mL). The combined organic phases were washed with dilute HCl, brine, dried (MgSO₄), and evaporated to give a yellow oil, which was used in Step 2 without further purification.

### Step 2

[4-(Morpholine-4-carbonyl)-phenyl]-carbamic acid *tert*-butyl ester as described in Step 1 (180 mg, 0.59 mmol) and 4M hydrogen chloride solution in dioxan (3 x mL) were combined and stirred at room temperature for 3 h. The volatiles were evaporated and water was added to the residue (20 mL), and pH adjusted to 8 with saturated NaHCO₃. The resulting mixture was then extracted with DCM (3 x 20 mL), the combined organic phases were washed with brine, dried (MgSO₄), and evaporated under reduced pressure to give the desired product as a yellow oil (159 mg, 91%). ¹H NMR (400 MHz, DMSO-*d₆*) δH ppm 3.46 - 3.49 (m, 4 H), 3.55 - 3.59 (m, 4 H), 5.54 (br. s, 2 H), 6.54 (d, *J*=8.2 Hz, 2 H), 7.12 (d, *J*=8.2 Hz, 2 H); m/z (ES+APCI)⁺: 207 [M+H]⁺.

### Intermediate 9

### 1-(2-Morpholin-4-yl-ethyl)-1H-pyrazol-4-ylamine

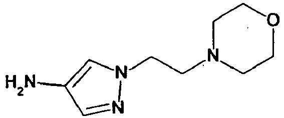

### Step 1

### 4-[2-(4-Nitro-pyrazol-1-yl)-ethyl]-morpholine

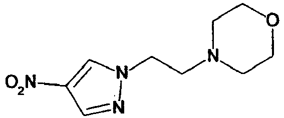

**N**-(2-chloroethyl)morpholine HCl salt (2.1 g, 11.06 mmol) was added portionwise to a stirred mixture of 4-nitro-1H-pyrazole (1.0 g, 8.85 mmol) and KOH (1.24 g, 22.12 mmol) in EtOH (20 mL). The mixture was heated under reflux for 2 h and allowed to cool to rt. After dilution with EtOAc and water the organic phase was washed with brine, dried and concentrated. The residue was purified by flash column chromatography on silica gel (100 g) eluting with 50:1 DCM-MeOH to provide an orange oil (987 mg, 49 %). ¹H NMR (400 MHz, DMSO-d₆) δH ppm 2.30 - 2.49 (m, 4 H), 2.73 (t, *J*=6.2 Hz, 2 H), 3.42 - 3.61 (m, 4 H), 4.30 (t, *J*=6.2 Hz, 2 H), 8.26 (s, 1 H), 8.88 (s, 1 H); m/z (ES+APCI)⁺ : 227 [M+H]⁺.

### Step 2

### 1-(2-Morpholin-4-yl-ethyl)-1H-pyrazol-4-ylamine

A mixture of 4-[2-(4-nitro-pyrazol-1-yl)-ethyl]-morpholine (964 mg, 4.35 mmol) and 10% Pd/C (117 mg) in EtOH (30 mL) was stirred at rt overnight under a balloon of hydrogen. The mixture was filtered through Celite and the filtrate concentrated to give a red oil (775 mg, 91 %). ¹**H NMR** (400 MHz, DMSO-*d₆*) δH ppm 2.30 - 2.44 (m, 4 H), 2.61 (t, *J*=6.6 Hz, 2 H), 3.47 - 3.59 (m, 4 H), 3.84 (br. s, 2 H), 4.02 (t, *J*=6.6 Hz, 2 H), 6.88 (s, 1 H), 7.05 (s, 1 H); m/z (ES+APCI)⁺ : 197 [M+H]⁺.

### Intermediate 10

### 1-methyl-4-(5-nitropyridin-2-yl)-1,4-diazepane

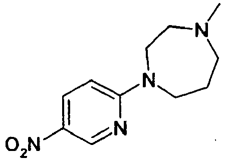

To a stirred solution of 2-chloro-5-nitropyridine (2 g, 12.6 mmol) in acetonitrile (40 mL) was added 1-methyl-1,4-diazepane (1.57 mL, 12.6 mmol) followed by the addition of DIPEA (2.20 mL, 12.6 mmol). The reaction mixture was stirred at rt for 18 hours. The mixture was then concentrated to dryness. The residue was diluted with EtOAc and washed with saturated sodium carbonate (aq). The organic layers were combined, dried and concentrated to afford the product as a yellow/orange solid (2.82 g, 95%). ¹H NMR (400 MHz, CDCl₃) δH ppm 2.08 - 2.30 (m, 2 H), 2.44 (s, 3 H), 2.65 (br. s, 2 H), 2.78 (br. s, 2 H), 3.65 - 3.85 (m, 2 H), 3.97 (br. s, 2 H), 6.48 (d, *J*=9.6 Hz, 1 H), 8.21 (dd, *J*=9.62, 2.8 Hz, 1 H), 9.05 (d, *J*=2.8 Hz, 1 H); m/z (ES+APCI)⁺: 237 [M+H]⁺.

### Intermediate 11

### 6-(4-methyl-1,4-diazepan-1-yl)pyridin-3-amine

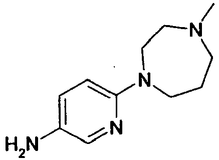

To a RB flask was added 1-methyl-4-(5-nitropyridin-2-yl)-1,4-diazepane (2.82 g) and 10% palladium on charcoal (282 mg) in ethanol (50 mL) and the mixture was stirred at rt under a hydrogen atmosphere for 18 hours. The reaction mixture was filtered through celite. The filtrate was concentrated to afford the product as a dark purple oil (2.4 g, 98% yield). ¹H NMR (400 MHz, DMSO-d₆) δH ppm 1.79 (dt, *J*=11.7, 6.1 Hz, 2 H), 2.19 (s, 3 H), 2:30 - 2.42 (m, 2 H), 2.42 - 2.53 (m, 2 H), 3.39 (t, *J*=6.2 Hz, 2 H), 3.47 - 3.59 (m, 2 H), 4.31 (br. s, 2 H), 6.33 (d, *J*=8.7 Hz, 1 H), 6.83 (dd, J=8.9, 3.0 Hz, 1 H), 7.48 (d, *J*=2.8 Hz, 1 H); m/z (ES+APCl)⁺: 207 [M+H]⁺.

### Intermediate 12

### 4-(4-bromophenyl)-1-methylpiperidin-4-ol

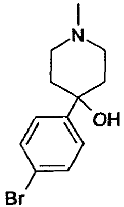

A 3-necked round bottom flask equipped with a magnetic stirrer, thermometer and addition funnel was charged with magnesium turnings (2.10 g, 0.087 mol) and diethyl ether (20 mL). 1,4-dibromobenzene (20 g, 0.085 mol) was dissolved in anhydrous diethyl ether (180 mL) and placed in the addition funnel. A few mLs of this solution was added to the reaction mixture followed by the addition of iodoethane (65 µl, 0.00081 mol) and a few granules of iodine in order to initiate the reaction. Localized heating was supplied by a hot air gun and once the reaction was able to maintain reflux, the remainder of the 1,4-dibromobenzene solution was added dropwise. After complete addition, the mixture was then heated under reflux for 30 min. The mixture was allowed to cool to rt and prior to dropwise addition of a solution of 1-methylpiperidin-4-one (10.4 mL, 0.0848 mol) in THF (200 mL). The reaction mixture was allowed to stir at rt overnight. The mixture was then poured into a solution of saturated ammonium chloride (aq). The mixture was concentrated. The residue was then basified using saturated sodium bicarbonate (aq). The mixture was then extracted using DCM. The organic layers were combined, dried and concentrated to afford a crude yellow solid. Purification by column chromatography using a Biotage SP4 (DCM/0.2M NH₃ in MeOH gradient) gave the product as a pale yellow solid (5.26 g, 23%). ¹H NMR (400 MHz, DMSO-d₆) δH ppm 1.50 (d, *J*=11.0 Hz, 2 H), 1.85 (m, 2 H), 2.15 (s, 3 H), 2.21 - 2.38 (m, 2 H), 2.42 - 2.58 (m, 2 H), 4.86 (s, 1 H), 7.39 (m, 2 H), 7.45 (m, 2 H); m/z (ES+APCI)⁺: 271/273 [M+H]⁺.

### Intermediate 13

### 4-[4-(Benzhydrylidene-amino)-phenyl]-1-methyl-piperidin-4-ol

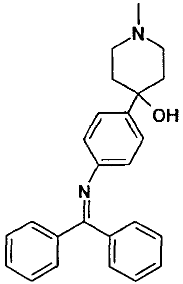

A mixture of 4-(4-bromophenyl)-1-methylpiperidin-4-ol (2 g, 7.4 mmol), benzophenone imine (1.49 mL, 8.88 mmol), caesium carbonate (7.24 g, 22.2 mmol), xantphos (343 mg, 0.592 mmol) and Pd₂(dba)₃ (407 mg, 0**.444** mmol) in dioxane (60 mL) was placed in a round bottomed flask. The mixture was degassed, placed under an atmosphere of nitrogen and stirred and heated at 100°C overnight. The reaction mixture was allowed to cool to rt. The mixture was filtered through a plug of SiO₂, washed with MeOH and DCM and the filtrate was concentrated to afford a crude red solid. DCM was added to the residue and the insolubles were removed by filtration. The filtrate was evaporated to give a red/orange solid. Purification by column chromatography using a Biotage SP4 (DCM/0.2M **N**H₃ in MeOH gradient) gave the product as an orange solid (2.18 g, 80%). ¹H NMR (400 MHz, CDCl₃) δH ppm 1.76 (d, *J*=12.4 Hz, 2 H), 2.26 (br. s, 2 H), 2.45 (br. s, 3 H), 2.65 (d, *J*=12.4 Hz, 2 H), 2.87 (br. s, 2 H), 6.67 - 6.72 (m, 2 H), 7.07 - 7.13 (m, 2 H), 7.22 - 7.32 (m, 8 H), 7.72 (d, *J*=6.87 Hz, 2 H); m/z (ES+APCI)⁺: 371 [M+H]⁺.

### Intermediate 14

### 4-(4-aminophenyl)-1-methylpiperidin-4-ol

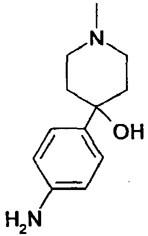

A mixture of 4-[4-(benzhydrylidene-amino)-phenyl]-1-methyl-piperidin-4-ol (2.18 g, 5.89 mmol), sodium acetate (1.16 g, 14.1 mmol) and hydroxylamine hydrochloride (736 mg, 10.6 mmol) in methanol (80 mL) was stirred at rt for 45 min. The mixture was concentrated and diluted with DCM and 0.1M NaOH (aq). The organic layers were combined, dried and concentrated to afford a crude orange solid. Purification by column chromatography using a Biotage SP4 (DCM/0.2M NH₃ in MeOH gradient) gave the product as a pale yellow solid (558 mg, 46%). ¹H NMR (400 MHz, DMSO-d₆) δH ppm 1.48 (d, *J*=11.5 Hz, 2 H), 1.78 (m, 2 H), 2.14 (s, 3 H), 2.20 - 2.34 (m, 2 H), 2.39 - 2.54 (m, 2 H), 4.35 (s, 1 H), 4.81 (s, 2 H), 6.45 (d, *J*=8.7 Hz, 2 H), 7.06 (d, *J*=8.7 Hz, 2 H); m/z (ES+APCI)⁺: 207 [M+H]⁺.

### Intermediate 15

### 4-(4-bromophenyl)-4-fluoro-1-methylpiperidine

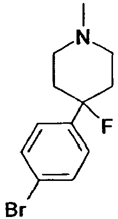

### Step 1

4-(4-Bromophenyl)-1-methylpiperidin-4-ol (4.08 g, 15.1 mmol) was dissolved in anhydrous DCM (125 mL) and cooled to -78 °C. DAST (7.98 mL, 60.4 mmol) was added to the reaction mixture and this was stirred at -78 °C for 6 h. The mixture was allowed to warm to rt and then stirred at rt overnight. The mixture was diluted with saturated sodium bicarbonate (aq) and DCM. The organic layers were combined, dried and concentrated. The crude product was purified by column chromatography using a Biotage SP4 (DCM/0.2M NH₃ in MeOH gradient) to give the crude product (3.79 g), which was used in the next step without purification.

### Step 2

To a mixture of tert-butanol (50 mL) and water (50 mL) was added AD-mix α (11.5 g) at rt. The mixture was cooled to 0°C and the product of Step 1 (3.79 g) was added to the reaction mixture, which was stirred at 0 °C for 24 hours. Solid sodium sulfite (11.5 g) was added to the reaction mixture, which was then stirred at rt for 30 min. The mixture was diluted with DCM (450 mL) and water (400 mL). The organic layer was dried and concentrated. The residue was diluted with DCM and the insolubles were removed by filtration. The filtrate was then evaporated. Purification by column chromatography using a Biotage SP4 (DCM/0.2M NH₃ in MeOH gradient) gave the title compound as a pale yellow solid (2.27 g, 60%). ¹H NMR (400 MHz, DMSO-d₆) δH ppm 1.72 - 1.89 (m, 2 H), 1.93 - 2.12 (m, 2 H), 2.14 - 2.24 (m, 5 H), 2.66 (d, J=10.5 Hz, 2 H), 7.25 - 7.45 (m, 2 H), 7.54 (d, *J*=7.8 Hz, 2 H); m/z (ES+APCI)⁺: 273/275 [M+H]⁺.

### Intermediate 16

### Benzhydrylidene-[4-(4-fluoro-1-methyl-piperidin-4-yl)-phenyl]-amine

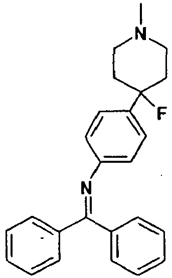

A mixture of 4-(4-bromophenyl)-4-fluoro-1-methylpiperidine (2.27 g, 8.34 mmol), benzophenone imine (1.68 mL, 10 mmol), caesium carbonate (8.16 g, 25 mmol), xantphos (386 mg, 0.667 mmol) and Pd₂(dba)₃ (459 mg, 0.50 mmol) in dioxane (70 mL) was placed in a round bottomed flask. The mixture was degassed, placed under an atmosphere of nitrogen and stirred and heated at 100°C overnight. The reaction mixture was allowed to cool to rt. The mixture was filtered through a plug of SiO₂, washed with MeOH and DCM and the filtrate was concentrated. DCM was added to the residue and the insolubles were removed by filtration. The filtrate was evaporated to give a crude red solid. Purification by column chromatography using a Biotage SP4 (DCM/0.2M NH₃ in MeOH gradient) gave the desired product (2.83 g, 91%). ¹H NMR (400 MHz, DMSO-*d₆*) δH ppm 1.68 - 1.84 (m, 2 H), 1.87 (d, *J*=8.7 Hz, 1 H), 1.99 (br. s, 1 H), 2.06 - 2.33 (m, 5 H), 2.62 (d, *J*=10.5 Hz, 2 H), 6.66 (d, *J*=6.9 Hz, 2 H), 6.95 - 7.23 (m, 4 H), 7.29 (br. s, 3 H), 7.32 - 7.57 (m, 3 H), 7.60 (d, *J*=7.33 Hz, 2 H); m/z (ES+APCl)⁺: 373 [M+H]⁺.

### Intermediate 17

### 4-(4-methoxy-1-methylpiperidin-4-yl)aniline

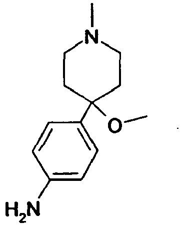

A mixture of benzhydrylidene-[4-(4-fluoro-1-methyl-piperidin-4-yl)-phenyl]-amine (2.83 g, 7.61 mmol), sodium acetate (1.5 g, 18.3 mmol) and hydroxylamine hydrochloride (952 mg, 13.7 mmol) in methanol (125 mL) was stirred at rt overnight, and concentrated under reduced pressure. Purification by column chromatography using a Biotage SP4 (DCM/0.2M NH₃ in MeOH gradient) gave a yellow solid (600 mg, 38%). ¹H NMR (400 MHz, CDCl₃) δH ppm 1.87 - 2.17 (m, 4 H), 2.42 (s, 3 H), 2.58 (t, *J*=11.5 Hz, 2 H), 2.79 - 3.05 (m, 5 H), 4.00 (br. s, 2 H), 6.7 (dd, *J*=8.5, 2.1 Hz, 2 H), 7.14 (dd, *J*=8.47, 2.06 Hz, 2 H); m/z (ES+APCI)⁺: 221 [M+H]⁺.

### Intermediate 18

### 4-(4-bromophenyl)-1-methylpiperidine-4-carbonitrile

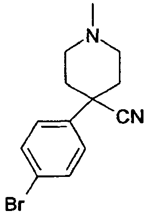

Sodium hydride, 60 % dispersion in oil (2.87 g, 71.8 mmol) was added portionwise to a solution of mechlorethamine hydrochloride salt (3.88 g, 20.2 mmol) and 4-bromophenylacetonitrile (3.52 g, 18 mmol) in anhydrous dimethylformamide (100 mL). The mixture was heated at 60 °C for 1 hour and then stirred at rt for 18 hours. The reaction mixture was poured into ice/water and extracted with EtOAc (x3). The organic layers were combined, dried and concentrated to afford a crude red solid. Purification by column chromatography using a Biotage SP4 (DCM/0.2M NH₃ in MeOH gradient) gave the desired product (4.53 g, 90%). ¹H NMR (400 MHz, DMSO-*d₆*) δH ppm 1.90 - 2.12 (m, 4 H), 2.12 - 2.39 (m, 5 H), 2.86 (d, *J*=12.4 Hz, 2 H), 7.47 (d, *J*=8.7 Hz, 2 H), 7.60 (d, *J*=8.7 Hz, 2 H).

### Intermediate 19

### 4-[4-(Benzhydrylidene-amino)-phenyl]-1-methyl-piperidine-4-carbonitrile

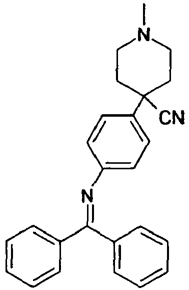

A mixture of 4-(4-bromophenyl)-1-methylpiperidine-4-carbonitrile (4.53 g, 16.2 mmol), benzophenone imine (3.26 mL, 19.4 mmol), caesium carbonate (15.8 g, 48.5 mmol), xantphos (749 mg, 1.29 mmol) and Pd₂(dba)₃ (888 mg, 0.970 mmol) in dioxane (100 mL) was placed in a round bottomed flask. The mixture was degassed, placed under an atmosphere of nitrogen and stirred and heated at 100 °C overnight. The reaction mixture was allowed to cool to rt. The mixture was filtered through a plug of SiO₂, washed with MeOH and DCM and the filtrate was concentrated. Purification by column chromatography using a Biotage SP4 (DCM/0.2M NH₃ in MeOH gradient) gave the desired product (1.53 g, 25%). ¹H NMR (400 MHz, DMSO-d₆) δH ppm 1.66 - 1.92 (m, 2 H), 1.92 - 2.06 (m, 2 H), 2.06 - 2.34 (m, 5 H), 2.81 (d, *J*=11.9 Hz, 2 H), 6.72 (d, *J*=7.3 Hz, 2 H), 6.92 - 7.19 (m, 2 H), 7.19 - 7.36 (m, 5 H), 7.36 - 7.66 (m, 5 H); m/z (ES+APCl)⁺: 380 [M+H]⁺.

### Intermediate 20

### 4-(4-aminophenyl)-1-methylpiperidine-4-carbonitrile

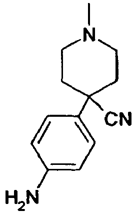

A mixture of 4-[4-(benzhydrylidene-amino)-phenyl]-1-methyl-piperidine-4-carbonitrile (1.53 g, 4.03 mmol), sodium acetate (794 mg, 9.68 mmol) and hydroxylamine hydrochloride (504 mg, 7.26 mmol) in methanol (75 mL) was stirred at rt overnight. Solvent removal followed by purification by column chromatography using a Biotage SP4 (DCM/0.2M NH₃ in MeOH gradient) gave the desired product as an orange/brown oil (771 mg, 89%). ¹H NMR (400 MHz, DMSO-d₆) δH ppm 1.76 - 1.93 (m, 2 H), 1.93 - 2.10 (m, 2 H), 2.10 - 2.34 (m, 5 H), 2.83 (d, *J*=11.9 Hz, 2 H), 5.12 (br. s, 2 H), 6.54 (d, *J*=6.9 Hz, 2 H), 7.08 (d, *J*=8.7 Hz, 2 H); m/z (ES+APCl)⁺: 216 [M+H]⁺.

### Intermediate 21

### 2-[4-(4-Nitro-phenyl)-piperidin-1-yl]-ethanol

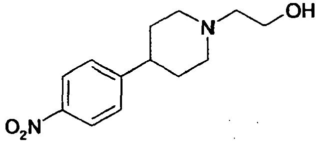

To a stirred solution of 4-(4-nitrophenyl)piperidine (2 g, 9.70 mmol) and anhydrous potassium carbonate (2.01 g, 14.5 mmol) in dry acetonitrile (25 mL) was added 2-bromoethanol (687 µl, 9.70 mmol). The reaction mixture was then heated under reflux for 4 hours. The reaction mixture was allowed to cool to rt. The mixture was filtered and the filtrate was concentrated. The mixture was diluted with EtOAc and water. The organic layer was dried and concentrated. Purification by column chromatography using a Biotage SP4 (DCM/0.2M NH₃ in MeOH gradient) gave the product as a pale yellow/orange oil (1.29 g, 53% yield). ¹H NMR (400 MHz, DMSO-d₆) δH ppm 1.58 - 1.81 (m, 4 H), 2.05 (m, 2 H), 2.40 (t, *J*=6.41 Hz, 2 H), 2.52 - 2.70 (m, 1 H), 2.97 (d, *J*=11.5 Hz, 2 H), 3.41 - 3.57 (m, 2 H), 4.40 (t, *J*=5.3 Hz, 1 H), 7.54 (m, 2 H), 8.15 (m, 2 H); m/z (ES+APCl)⁺: 251 [M+H]⁺.

### Intermediate 22

### 1-(2-Fluoro-ethyl)-4-(4-nitro-phenyl)-piperidine

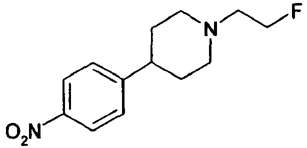

To a stirred solution of 2-[4-(4-nitro-phenyl)-piperidin-1-yl]-ethanol (640 mg, 2.56 mmol) in anhydrous DCM (20 mL) was added DAST (811 µl, 6.14 mmol) in dropwise portions. The reaction was allowed to stir at rt overnight. The mixture was diluted with DCM and 2N NaOH (aq). The organic layers were combined, dried and concentrated. Purification by column chromatography using a Biotage SP4 (DCM/0.2M NH₃ in MeOH gradient) gave the product as a brown/orange oil (200 mg, 31% yield). ¹H NMR (400 MHz, DMSO-d₆) δH ppm 1.60 -1.87 (m, 4 H), 2.11 (m, 2 H), 2.55 - 2.72 (m, 3 H), 3.00 (d, *J*=11.5 Hz, 2 H), 4.48 (t, *J*=4.8 Hz, 1 H), 4.60 (t, J=5.0 Hz, 1 H), 7.55 (m, J=8.7 Hz, 2 H), 8.15 (m, 2 H); m/z (ES+APCI)⁺: 253 [M+H]⁺.

### Intermediate 23

### 4-[1-(2-Fluoro-ethyl)-piperidin-4-yl]-phenylamine

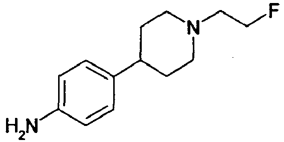

To a RB flask was added 1-(2-fluoro-ethyl)-4-(4-nitro-phenyl)-piperidine (200 mg) and 10% palladium on charcoal (20 mg) in ethanol (7 mL) and the mixture was stirred at rt under a hydrogen atmosphere for 18 hours. The reaction mixture was filtered through celite and washed with EtOH. The filtrate was concentrated to afford the crude product as an orange oil. Purification by column chromatography using a Biotage SP4 (DCM/0.2M NH₃ in MeOH gradient) gave the desired product as an orange solid (106 mg, 60% yield). ¹H NMR (400 MHz, DMSO-d₆) δH ppm 1.47 - 1.73 (m, 4 H), 1.99 - 2.16 (m, 2 H), 2.21 - 2.30 (m, 1 H), 2.51 - 2.69 (m, 2 H), 2.90 - 3.03 (m, 2 H), 4.44 - 4.50 (m, 1 H), 4.56 - 4.62 (m, 1 H), 4.79 - 4.86 (m, 2 H), 6.5 (m, 2 H), 6.86 (m, *J*=8.7 Hz, 2 H); m/z (ES+APCl)⁺: 223 [M+H]⁺.

### Example 1

### Cyclobutanecarboxylic acid {3-[2-(1-methyl-1H-indazol-5-ylamino)-pyrrolo[2,3-d]pyrimidin-7-yl]-propyl}-amide

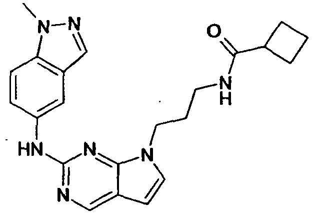

Intermediate 1 (50 mg, 0.17 mmol), 1-methyl-1*H*-indazol-5-ylamine (30 mg, 0.21 mmol), Pd₂(dba)₃ (9.4 mg, 0.01 mmol), xantphos (8 mg, 0.014 mmol) and sodium *tert*-butoxide (66 mg, 0.51 mmol) were combined with dioxane (1.5ml), sealed and then purged with nitrogen gas. The reaction mixture was heated at 90 °C for 18 hours, evaporated and purified through a silica plug, eluting with 0 to 10% methanol in DCM. The crude product was further purified by preparative LCMS (low pH buffer). The resulting TFA salt was then eluted through a 1g Isolute-NH₂ cartridge with 9:1 DCM: methanol to liberate the free base as a white solid (29 mg, 43%). ¹H **NMR** (400 MHz, DMSO-*d₆*) δH ppm 1.63 - 1.84 (m, 2 H), 1.86 - 1.98 (m, 4 H), 2.00 - 2.13 (m, 2 H), 2.83 - 2.95 (m, 1 H), 3.01 - 3.10 (m, 2 H), 4.01 (s, 3 H), 4.11 - 4.20 (m, 2 H), 6.43 (d, *J*=3.2 Hz, 1 H), 7.28 (d, *J*=3.7 Hz, 1 H), 7.54 (d, *J*=8.7 Hz, 1 H), 7.64 - 7.72 (m, 2 H), 7.97 (s, 1 H), 8.43 (d, *J*=1.4 Hz, 1 H), 8.68 (s, 1 H), 9.41 (br. s, 1 H). m/z (ES+APCl)⁺: 404 [M+H]⁺.

### Examples 2-9

Examples 2-9 were prepared analogously to Example 1 (the general structure is shown below followed by the tabulated examples).

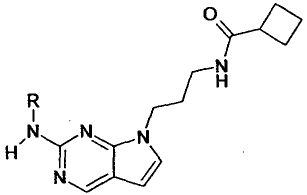

| **Example** | **R group** | **Name** | **m/z (ES+APCl)⁺** | **HPLC retention time (min)*** |
|---|---|---|---|---|
| **2** | 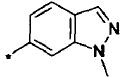 | Cyclobutanecarboxylic acid {3-[2-(1-methyl-1*H*-indazol-6-ylamino)-pyrrolo[2,3-*d*]pyrimidin-7-yl]-propyl}amide | 404 | 4.47 |
| **3** | 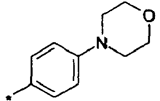 | Cyclobutanecarboxylic acid {3-[2-(4-morpholin-4-yl-phenylamino)-pyrrolo[2,3-*d*]pyrimidin-7-yl]-propyl}-amide | 435 | 3.88 |
| **4** | 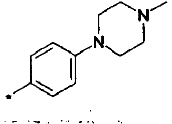 | Cyclobutanecarboxylic acid (3-{2-[4-(4-methyl-piperazin-1-yl)-phenylamino]-pyrrolo[2,3-*d*]pyrimidin-7-yl}-propyl)-amide | 448 | 3.07 |
| **5** | 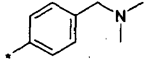 | Cyclobutanecarboxylic acid {3-[2-(4-dimethylaminomethyl-phenylamino)-pyrrolo[2,3-*d*]pyrimidin-7-yl]-propyl}-amide | 362 [M-NMe₂]⁺ | 2.90 |
| **6** | 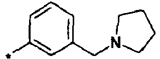 | Cyclobutanecarboxylic acid {3-[2-(3-pyrrolidin-1-ylmethyl-phenylamino)-pyrrolo[2,3-*d*]pyrimidin-7-yl]-propyl}-amide | 433 | 3.18 |
| **7** | 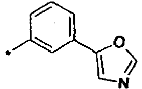 | Cyclobutanecarboxylic acid {3-[2-(3-oxazol-5-yl-phenylamino)-pyrrolo[2,3-*d*]pyrimidin-7-yl)-propyl}-amide | 417 | 4.62 |
| **8** | 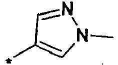 | Cyclobutanecarboxylic acid {3-[2-(1-methyl-1*H*-pyrazol-4-ylamino)-pyrrolo[2,3-*d*]pyrimidin-7-yl]-propyl}-amide | 354 | 3.37 |
| **9** | 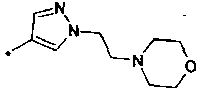 | Cyclobutanecarboxylic acid (3-{2-[1-(2-morpholin-4-yl-ethyl)-1*H*-pyrazol-4-ylamino]-pyrrolo[2,3-*d*]pyrimidin-7-yl}-propyl)-amide | 453 | 2.75 |

| | | | | |
|---|---|---|---|---|
| *HPLC column: 21.2 x 100mm (5 µm) C-18 Phenomenex Gemini; flow rate: 20ml/min; run time: 9 min; gradient at start: 10% methanol and 90% water, gradient at finish: 100% methanol and 0% water; as buffer: 0.1 % trifluoroacetic acid is added to the water. | | | | |

### Examples 10-14

Examples 10-14 were prepared analogously to Example 1, from Intermediate 3 and the appropriate amine (the general structure is shown below followed by the tabulated examples).

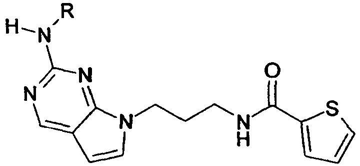

| **Example** | **R group** | **Name** | **m/z (ES+APCl)*** |
|---|---|---|---|
| **10** | 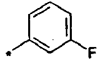 | Thiophene-2-carboxylic acid {3-[2-(3-fluoro-phenylamino)-pyrrolo[2,3-d]pyrimidin-7-yl]-propyl}-amide | 396 |
| **11** | 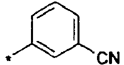 | Thiophene-2-carboxylic acid {3-[2-(3-cyano-phenylamino)-pyrrolo[2,3-d]pyrimidin-7-yl]-propyl}amide | 403 |
| **12** | 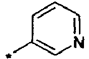 | Thiophene-2-carboxylic acid {3-[2-(pyridin-3-ylamino)-pyrrolo[2,3-d]pyrimidin-7-yl]-propyl}-amide | 379 |
| **13** | 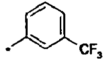 | Thiophene-2-carboxylic acid {3-[2-(3-trifluoromethyl-phenylamino)-pyrrolo[2,3-d]pyrimidin-7-yl]-propyl}-amide | 446 |
| **14** | 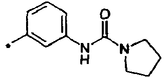 | Pyrrolidine-1-carboxylic acid [3-(7-{3-[(thiophene-2-carbonyl)-amino]-propyl}-7H-pyrrolo[2,3-d]pyrimidin-2-ylamino)-phenyl]-amid | 490 |

### Example 15

### Cyclobutanecarboxylic acid (3-{2-[4-(morpholine-4-carbonyl)-phenylamino]-pyrrolo[2,3-d]pyrimidin-7-yl}-propyl)-amide

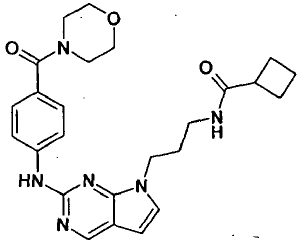

Intermediate 1 (236 mg, 0.81 mmol), Intermediate 8 (200 mg, 0.97 mmol), Pd₂(dba)₃ (26 mg, 0.028 mmol), xantphos (22 mg, 0.038 mmol) and sodium *tert*-butoxide (135 mg, 2.42 mmol) were combined with dioxane (6 mL), sealed and then purged with nitrogen gas, and the reaction mixture was heated at 90°C for 18 hours. The mixture was evaporated and purified through a silica plug, eluting with 0 to 10% methanol in DCM. Further purification by preparative LCMS (high pH buffer) gave the desired product as a white solid (113 mg, 36%). ¹H NMR (400 MHz, DMSO-*d₆*) δH ppm 1.66 -1.75 (m, 1 H), 1.77 - 2.00 (m, 5 H), 2.02 - 2.13 (m, 2 H), 2.90 - 2.99 (m, 1 H), 3.01 - 3.08 (m, 2 H), 3.48 - 3.56 (m, 4 H), 3.57 - 3.66 (m, 4 H), 4.12 - 4.20 (m, 2 H), 6.46 (d, *J*=3.7 Hz, 1 H), 7.31 - 7.41 (m, 3 H), 7.72 (br. s, 1 H), 7.94 (d, *J*=8.7 Hz, 2 H), 8.72 (s, 1 H), 9.71 (br. s, 1 H). m/z (ES+APCI)⁺: 463.3 [M+H]⁺.

### Example 16

### Cyclopentanecarboxylic acid {3-[2-(6-morpholin-4-yl-pyridin-3-ylamino)-pyrrolo[2,3-d]pyrimidin-7-yl]-propyl}-amide

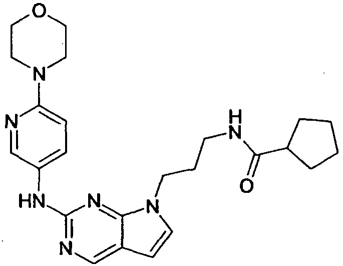

A solution of cyclopentanecarboxylic acid (18 µl, 0.17 mmol) in DMF (0.5 mL) at room temperature was treated with triethylamine (26 µl, 0.18 mmol) and isobutyl chloroformate (24 µL, 0.18 mmol) and stirred for 15 minutes. A solution of intermediate 7 (50 mg, 0.14 mmol) in DMF (1 mL) was added and the mixture stirred at room temperature for 3 h. The mixture was diluted with DCM (10 mL), partitioned with water (15 mL) and separated. The aqueous layer was extracted with DCM (2x 10 mL), the organic layers were combined, washed with brine, dried (MgSO₄) and evaporated. The crude product was purified by preparative LCMS (high pH buffer) to give the desired product as a cream solid (35 mg, 55%). ¹H NMR (400 MHz, DMSO-d₆) δH ppm 1.40 - 1.49 (m, 2 H), 1.51 - 1.63 (m, 4 H), 1.63 - 1.72 (m, 2 H), 1.86 - 1.94 (m, 2 H), 2.42 - 2.51 (m, 1 H), 2.97 - 3.04 (m, 2 H), 3.31 - 3.35 (m, 4 H), 3.69 - 3.73 (m, 4 H), 4.08 - 4.13 (m, 2 H), 6.41 (d, *J*=3.7 Hz, 1 H), 6.84 (d, *J*=9.2 Hz, 1 H), 7.25 (d, *J*=3.7 Hz, 1 H), 7.77 - 7.82 (m, 1 H), 8.05 (dd, *J*=9.2, 2.7 Hz, 1 H), 8.61 (d, *J*=2.7 Hz, 1 H), 8.63 (s, 1 H), 9.21 (br. s, 1 H). m/z (ES+APCI)⁺: 450 [M+H]⁺

### Example 17

### Pyrazine-2-carboxylic acid {3-[2-(6-morpholin-4-yl-pyridin-3-ylamino)-pyrrolo[2,3-d]pyrimidin-7-yl]-propyl}-amide

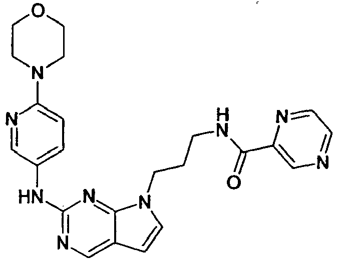

A solution of pyrazine-2-carboxylic acid (21 mg, 0.17 mmol) in DMF (0.5 mL) at room temperature was treated with triethylamine (26 µl, 0.18 mmol) and isobutylchloroformate (24 µL, 0.18 mmol) and stirred for 15 min. A solution of intermediate 7 (50 mg, 0.14 mmol) in DMF (1 mL) was added and the mixture stirred at room temperature for 3 h. The mixture was evaporated to dryness and purified by preparative LCMS (high pH buffer) to give the desired product as a yellow solid (41 mg, 63%). ¹H NMR (400 MHz, DMSO-*d₆*) δH ppm 2.03 - 2.15 (m, 2 H), 3.26 - 3.34 (m, 6H), 3.67 - 3.74 (m, 4 H), 4.11 - 4.22 (m, 2 H), 6.41 (d, *J*=3.7 Hz, 1 H), 6.74 (d, *J*=9.2 Hz, 1 H), 7.31 (d, *J*=3.7 Hz, 1 H), 8.03 (dd, *J*=9.2, 2.7 Hz, 1 H), 8.56 (d, *J*=2.3 Hz, 1 H), 8.62 (s, 1H), 8.67 (dd, *J*=2.5, 1.6 Hz, 1 H), 8.84 (d, *J*=2.7 Hz, 1 H), 9.02 - 9.07 (m, 1 H), 9.14 (d, *J*=1.4 Hz, 1 H), 9.18 (br. s, 1 H). m/z (ES+APCI)⁺: 460.2 [M+H]⁺

### Example 18

### 2-Cyclopropyl-N-{3-[2-(6-morpholin-4-yl-pyridin-3-ylamino)-pyrrolo[2,3-d]pyrimidin-7yl]-propyl}-acetamide

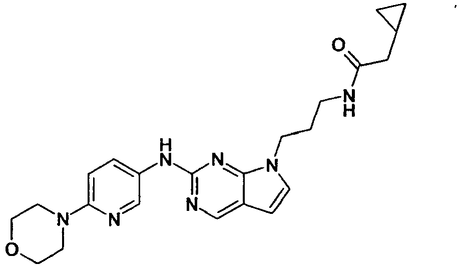

A solution of cyclopropylacetic acid (17 mg, 0.17 mmol) in DMF (0.5 mL) at room temperature was treated with triethylamine (26 µl, 0.18 mmol) and isobutylchloroformate (24 µl, 0.18 mmol) and stirred for a further 15 minutes. A solution of intermediate 7 (50 mg, 0.14 mmol) in DMF (1 mL) was added and the mixture stirred at room temperature for 3 h. The mixture was evaporated to dryness then purified by preparative LCMS (low pH buffer). The resulting formic acid salt was eluted through a 0.5 g isolute-NH₂ cartridge with 9:1 DCM: methanol to liberate the free base as a white solid (17 mg, 27%). ¹H NMR (400 MHz, DMSO-d₆) δH ppm 0.04 - 0.09 (m, 2 H), 0.36 - 0.42 (m, 2 H), 0.86 - 0.97 (m, 1 H), 1.87 - 1.97 (m, 4 H), 2.99 - 3.06 (m, 2 H), 3.32 - 3.35 (m, 4 H), 3.69 - 3.73 (m, 4 H), 4.09 - 4.14 (m, 2 H), 6.41 (d, *J*=3.2 Hz, 1 H), 6.84 (d, *J*=9.2 Hz, 1 H), 7.26 (d, *J*=3.7 Hz, 1 H), 7.75 - 7.80 (m, 1 H), 8.03 (dd, *J*=9.2, 2.7 Hz, 1 H), 8.62 (d, *J*=2.3 Hz, 1 H), 8.63 (s, 1 H), 9.21 (br. s, 1 H). m/z (ES+APCI)⁺: 436 [M+H]⁺.

### Examples 19-22

Examples 19-22 were prepared analogously to Example 18 (the general structure is shown below followed by the tabulated examples).

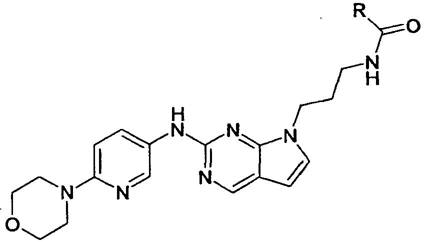

| **Example** | **R group** | **Name** | **m/z (ES+APCl)⁺** | **HPLC retention time (min)** |
|---|---|---|---|---|
| **19** | 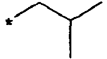 | 3-Methyl-*N*-{3-[2-(6-morpholin-4-yl-pyridin-3-ylamino)-pyrrolo[2,3-*d*]pyrimidin-7-yl]-propyl}-butyramide | 438 | 2.23 *^{a}* |
| **20** | 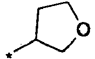 | Tetrahydro-furan-3-carboxylic acid {3-[2-(6-morpholin-4-yl-pyridin-3-ylamino)-pyrrolo[2,3-*d*]pyrimidin-7-yl]-propyl}-amide | 452 | 1.88 *^{a}* |
| **21** | 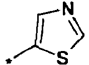 | Thiazole-5-carboxylic acid {3-[2-(6-morpholin-4-yl-pyridin-3-ylamino)-pyrrolo[2,3-*d*]pyrimidin-7-yl]-propyl}-amide | 465 | 1.34 *^{b}* |
| **22** | 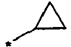 | Cyclopropanecarboxyli c acid {3-[2-(6-morpholin-4-yl-pyridin-3-ylamino)-pyrrolo[2,3-*d*]pyrimidin-7-yl]-propyl}-amide | 422 | 1.40 *^{b}* |

| | | | | |
|---|---|---|---|---|
| ^{a} HPLC column: 4.6x50mm (5µm) C-18 Phenomenex Gemini-NX; flow rate: 2ml/min; Run time: 4.6 min: Solvent A: 0.1% Formic acid in water, Solvent B: Methanol; Gradient - 10-100%B; Gradient time: 3.5min. ^{b} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min: | | | | |

### Examples 23 - 26

Examples 23 - 26 were prepared analogously to Example 18 (the general structure is shown below followed by the tabulated examples).

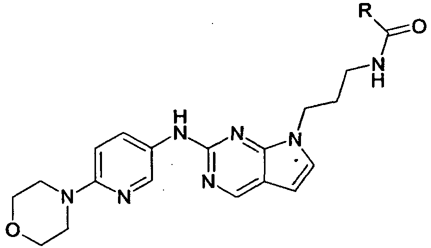

| **Example** | **R group** | **Name** | **m/z (ES+APCI)⁺** | **HPLC retention time (min)** |
|---|---|---|---|---|
| **23** | 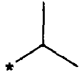 | *N*-{3-[2-(6-Morpholin-4-yl-pyridin-3-ylamino)-pyrrolo[2,3-*d*]pyrimidin-7-yl]-propyl}-isobutyramide | 424 | 2.03 *^{a}* |
| **24** | 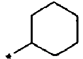 | Cyclohexanecarboxylic acid {3-[2-(6-morpholin-4-yl-pyridin-3-ylamino)-pyrrolo[2,3-*d*]pyrimidin-7-yl]-propyl]-amide | 464 | 2.51 *^{a}* |
| **25** | 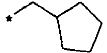 | 2-Cyclopentyl-*N*-{3-[2-(6-morpholin-4-yl-pyridin-3-ylamino)-pyrrolo[2,3-*d*]pyrimidin-7-yl]-propyl}-acetamide | 464 | 2.55 *^{a}* |
| **26** | 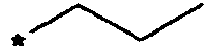 | *N*-{3-[2-(6-Morpholin-4-yl-pyridin-3-ylamino)-pyrrolo[2,3-*d*]pyrimidin-7-yl]-propyl}-butyramide | 424 | 3.17 *^{b}* |

| | | | | |
|---|---|---|---|---|
| *^{a}* HPLC column: 4.6x50mm (5µm) C-18 Phenomenex Gemini-NX; flow rate: 2ml/min; Run time: 4.6 min: Solvent A: 0.1% Formic acid in water, Solvent B: Methanol; Gradient - 10-100%B; Gradient time: 3.5min. *^{b}* HPLC column: 4.6x50mm (5µm) C-18 Phenomenex Gemini-NX; flow rate: 2ml/min; Run time: 4.6 min: Solvent A: 0.1% Ammonium Hydroxide in water, Solvent B: Methanol; Gradient - 10-100%B; Gradient time: 3.5min: | | | | |

### Example 27

### Cyclobutanecarboxylic acid {3-[2-(3-fluoro-phenylamino)-pyrrolo[2,3-d]pyrimidin-7-y]-propyl}-amide

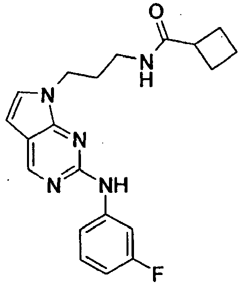

A 4 mL Chromacol tube was charged with Intermediate **1** (33 mg, 0.11 mmol), Pd₂(dba)₃ (5 mg, 0.0056 mmol), xantphos (5 mg, 0.009 mmol) and sodium tert-butoxide (30 mg, 0.31 mmol). 3-fluoroaniline (16 mg, 0.15 mmol) and dioxane (2 mL) were added, the solution purged with nitrogen for 2 minutes and then heated at 105 °C for 2 h. The mixture was cooled and filtered through a short pad of silica (eluting with CH₂Cl₂-MeOH, 9:1) and the filtrate concentrated *in vacuo.* Purification by preparative LCMS gave the product as a pale yellow solid; δ_{H} (400 MHz, d₆-DMSO) 9.72 (s, 1 H), 8.74 (s, 1H), 7.90 (dt, J = 12.8, 2.3 Hz, 1H), 7.68 (t, J = 5.5 Hz, 1H), 7.60 (dd, J = 8.2, 1.8 Hz, 1 H), 7.36 (d, J = 3.2 Hz, 1 H), 7.29 (q, J = 8.2 Hz, 1H), 6.70 (td, J = 8.2, 1.8 Hz, 1H), 6.48 (d, J = 3.7 Hz, 1H), 4.15 (t, J = 6.9 Hz, 2H), 3.03 (q, J = 6.4 Hz, 2H), 2.93 (quintet, J = 7.8 Hz, 1H), 2.12-1.69 (m, 8H); m/z (ES+APCI)⁺: 368 ([M+H]⁺.

### Examples 28-31

Examples 28-31 were prepared analogously to Example 27 from Intermediate 1 and the appropriate amine.

### Example 28

### Cyclobutanecarboxylic acid {3-[2-(3-acetylamino-phenylamino)-pyrrolo[2,3-d]pyrimidin-7-yl]-propyl}-amide

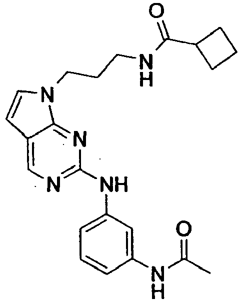

Pale yellow solid; δ_{H} (400 MHz, d₆-DMSO) 9.89 (s, 1 H), 9.58 (s, 1H), 8.72 (s, 1 H), 8.40 (s, 1 H), 7.65 (t, J = 5.5 Hz, 1 H), 7.38-7.36 (m, 2H), 7.19 (t, J = 8.2 Hz, 1 H), 7.05 (d, br, J = 8.7 Hz, 1H), 6.49 (d, J = 3.7 Hz, 1H), 4.22 (t, J = 6.9 Hz, 2H), 3.03 (q, J = 6.9 Hz, 2H), 2.90 (quintet, J = 8.7 Hz, 1H), 2.05 (s, 3H), 2.04-1.68 (m, 8H); m/z (ES+APCl)⁺: 407 [M+H]⁺.

### Example 29

### Cyclobutanecarboxylic acid (3-{2-[3-(2-oxo-pyrrolidin-1-yl)-phenylamino]-pyrrolo[2,3-d]pyrimidin-7-yl}-propyl)-amide

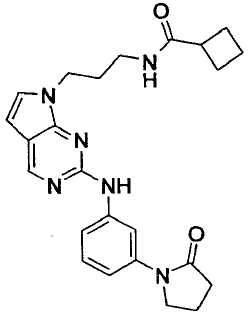

Off-white solid; δ_{H} (400 MHz, MeOD) 8.64 (s, 1 H), 8.56 (s, br, 1 H), 7.43 (d, br, J = 6.9 Hz, 1 H), 7.36 (t, br, J = 7.8 Hz, 1 H), 7.18 (d, br, J = 7.8 Hz, 1 H), 7.21 (d, J = 3.7 Hz, 1H), 6.51 (d, J = 3.7 Hz, 1H), 4.36 (t, J = 6.9 Hz, 2H), 4.03 (t, J = 6.9 Hz, 2H), 3.16 (t, J = 6.9 Hz, 2H), 2.91 (quintet, J = 8.2 Hz, 1 H), 2.68 (t, J = 7.8 Hz, 2H), 2.30-1.78 (m, 12H); m/z (ES+APCI)⁺: 433 [M+H]⁺.

### Example 30

### Cyclobutanecarboxylic acid {3-[2-(6-morpholin-4-yl-pyridin-3-ylamino)-pyrrolo[2,3-d]pyrimidin-7-yl]-propyl}-amide

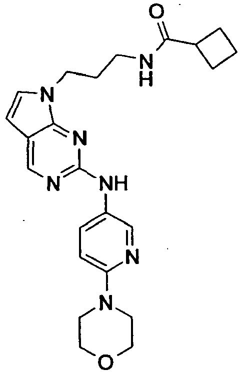

Pale yellow solid; δ_{H} (400 MHz, MeOD) 8.61 (d, J = 2.8 Hz, 1 H), 8.58 (s, 1 H), 8.04 (dd, J = 9.2, 2.8 Hz, 1H), 7.16 (d, J = 3.7 Hz, 1H), 6.92 (d, J = 9.2 Hz, 1H), 6.47 (d, J = 3.7 Hz, 1 H), 4.23 (t, J = 6.9 Hz, 2H), 3.86 (dd, J = 5.0, 4.6 Hz, 4H), 3.45 (t, J = 5.0 Hz, 4H), 3.16 (t, J = 6.9 Hz, 2H), 2.95 (quintet, J = 8.2 Hz, 1H), 2.29-1.77 (m, 8H); m/z (ES+APCI)⁺: 436 [M+H]⁺.

### Example 31

### Cyclobutanecarboxylic acid (3-{2-[6-(4-methyl-piperazin-1-yl)-pyridin-3-ylamino]-pyrrolo[2,3-d]pyrimidin-7-yl}-propyl)-amide

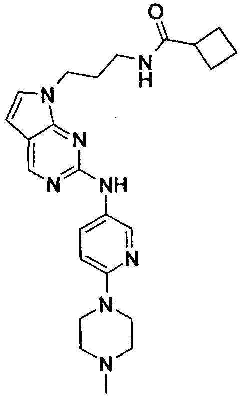

Pale yellow solid; δ_{H} (400 MHz, MeOD) 8.63 (d, J = 2.8 Hz, 1 H), 8.57 (s, 1 H), 8.01 (dd, J = 8.7, 2.8 Hz, 1 H), 7.15 (d, J = 3.7 Hz, 1 H), 6.92 (d, J = 9.2 Hz, 1 H), 6.47 (d, J = 3.7 Hz, 1 H), 4.23 (t, J = 6.9 Hz, 2H), 3.54-3.51 (m, 4H), 3.16 (t, J = 6.9 Hz, 2H), 2.94 (quintet, J = 8.2 Hz, 1H), 2.66-2.62 (m, 4H), 2.39 (s, 3H), 2.33-1.77 (m, 8H); m/z (ES+APCI)⁺: 449 ([M+H]⁺.

### Example 32, Example 33

Example 32, Example 33 were prepared analogously to Example 27 from Intermediate 2 and the appropriate amine.

### Example 32

### Cyclopentanecarboxylic acid {3-[2-(3-fluoro-phenylamino)-pyrrolo[2,3-d]pyrimidin-7-yl]-propyl}-amide

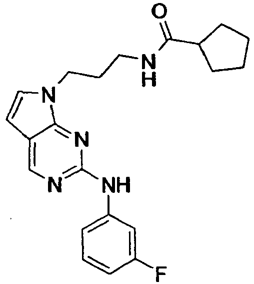

Pale yellow solid; δ_{H} (400 MHz, d₆-DMSO) 9.74 (s, 1 H), 8.74 (s, 1 H), 7.90 (dt, J = 12.8, 2.3 Hz, 1H), 7.80 (t, J = 5.0 Hz, 1H), 7.56 (dd, J = 8.2, 1.4 Hz, 1 H), 7.37 (d, J = 3.7 Hz, 1H), 7.29 (q, J = 7.3 Hz, 1 H), 6.70 (td, J = 8.2, 2.8 Hz, 1H), 6.49 (d, J = 3.7 Hz, 1H), 4.16 (t, J = 6.9 Hz, 2H), 3.03 (q, J = 6.4 Hz, 2H), 2.49 (quintet, J = 7.3 Hz, 1H), 1.94 (quintet, J = 6.9 Hz, 2H), 1.70-1.44 (m, 8H); m/z (ES+APCI)⁺: 382 [M+H]⁺.

### Example 33

### Cyclopentanecarboxylic acid {3-[2-(3-acetylamino-phenylamino)-pyrrolo[2,3-d] pyrimidin-7-yl]-propyl}-amide

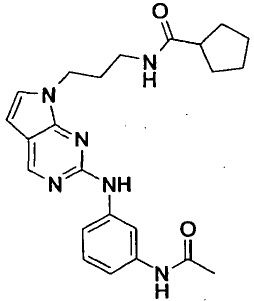

Pale yellow solid; δ_{H} (400 MHz, d₆-DMSO) 9.87 (s, 1 H), 9.51 (s, 1 H), 8.70 (s, 1 H), 8.42 (t, J = 2.2 Hz, 1H), 7.75 (t, J = 5.5 Hz, 1 H), 7.38 (dd, J = 7.3, 1.4 Hz, 1 H), 7.34 (d, J = 3.7 Hz, 1H), 7.17 (t, J = 7.8 Hz, 1H), 7.04 (d, br, J = 9.2 Hz, 1 H), 6.74 (d, J = 3.7 Hz, 1 H), 4.23 (t, J = 7.3 Hz, 2H), 3.04 (q, J = 6.4 Hz, 2H), 2.46 (quintet, J = 7.8 Hz, 1H), 2.05 (s, 3H), 1.90 (quintet, J = 6.9 Hz, 2H), 1.67-1.42 (m, 8H); m/z (ES+APCI)⁺: 421 [M+H]⁺.

### Example 34

### Cyclobutanecarboxylic acid {3-[5-chloro-2-(3-fluoro-phenylamino)-pyrrolo[2,3-d)pyrimidin-7-yl]-propyl}-amide

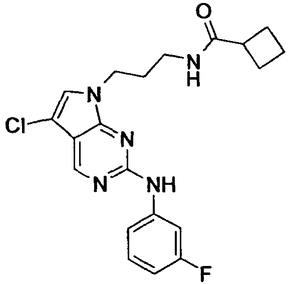

Prepared analogously to Example 27 from Intermediate 4 and 3-fluoroaniline to give a pale yellow solid; δ_{H} (400 MHz, MeOD) 8.64 (s, 1H), 7.86 (dt, J = 12.4, 1.8 Hz, 1 H), 7.43 (ddd, J = 8.2, 1.8, 0.9 Hz, 1H), 7.30 (qd, J = 8.2, 1.4 Hz, 1H), 7.27 (s, 1H), 6.72 (tdd, J = 8.2, 2.3, 0.9 Hz, 1H), 4.25 (t, J = 6.9 Hz, 2H), 3.19 (t, J = 6.9 Hz, 2H), 3.00 (quintet, J = 8.7 Hz, 1H), 2.23-1.77 (m, 10H); m/z (ES+APCI)⁺: 402 / 404 [M+H]⁺.

### Example 35

### Cyclobutanecarboxylic acid {3-[5-chloro-2-(6-morpholin-4-yl-pyridin-3-ylamino)-pyrrolo[2,3-d]pyrimidin-7-yl]-propyl}-amide

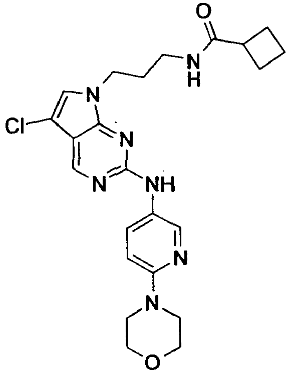

Prepared analogously to Example 27 from Intermediate 4 and 6-morpholin-4-yl-pyridin-3-ylamine to give an off-white solid; δ_{H} (400 MHz, MeOD) 8.62 (d, J = 2.8 Hz, 1 H), 8.56 (s, 1H), 8.01 (dd, J = 9.2, 2.8 Hz, 1H), 7.20 (s, 1H), 6.90 (d, J = 9.2 Hz, 1H), 4.19 (t, J = 6.9 Hz, 2H), 3.87-3.84 (m, 4H), 3.47-3.43 (m, 4H), 3.17 (t, J = 6.9 Hz, 2H), 2.96 (quintet, J = 8.7 Hz, 1H), 2.23-1.77 (m, 10H); m/z (ES+APCI)⁺: 470 / 472 [M+H]⁺.

### Example 36

### Cyclobutanecarboxylic acid {3-[5-bromo-2-(3-fluoro-phenylamino)-pyrrolo[2,3-d]pyrimidin-7-yl]-propyl}-amide

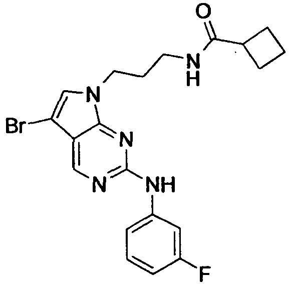

Prepared analogously to Example 27 from Intermediate 6 and 3-fluoroaniline to give the product as a pale yellow solid; δ_{H} (400 MHz, d₆-DMSO) 9.89 (s, 1 H), 8.64 (s, 1H), 7.88

(dt, J = 12.4, 2.3 Hz, 1H), 7.68 (t, J = 5.5 Hz, 1H), 7.59 (s, 1H), 7.57 (ddd, J = 7.3, 1.8, 0.9 Hz, 1 H), 7.30 (q, J = 8.2 Hz, 1 H), 6.73 (tdd, J = 9.2, 2.8, 0.9 Hz, 1H), 4.14 (t, J = 6.6 Hz, 2H), 3.03 (q, J = 6.4 Hz, 2H), 2.92 (quintet, J = 8.7 Hz, 1H), 2.11-1.65 (m, 8H); m/z (ES+APCI)⁺: 446 / 448 [M+H]⁺.

### Example 37

### Cyclobutanecarboxylic acid {3-[5-cyano-2-(3-fluoro-phenylamino)-pyrrolo[2,3-d]pyrimidin-7-yl]-propyl}-amide

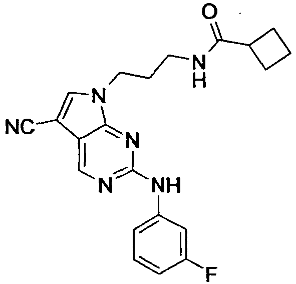

A solution of the bromide Example 36 (15 mg, 0.034 mmol) in DMF (0.5 mL) was treated with Pd₂dba₃ (1.5 mg, 0.0017 mmol), dppf (2 mg, 0.0034 mmol) and Zn(CN)₂ (39 mg, 0.34 mmol), degassed for 1 minute and then stirred with microwave heating at 180 °C for 30 minutes. Filtration through a short pad of Celite, concentration *in vacuo* and purification by preparative LCMS gave the TFA salt after evaporation *in vacuo.* The residue was dissolved in CH₂Cl₂-MeOH, 9:1 and passed through a 500 mg aminopropyl cartridge eluting with CH₂Cl₂-MeOH, 9:1 to liberate the free base (7 mg, 53%) as a colourless solid; δ_{H} (400 MHz, d₆-DMSO) 10.03 (s, 1H), 8.94 (s, 1H), 8.33 (s, 1H), 7.85 (dt, J = 12.8, 2.3 Hz, 1H), 7.68 (t, J = 6.0 Hz, 1H), 7.58 (ddd, J = 8.2, 1.8, 0.9 Hz, 1H), 7.32, (q, J = 8.2 Hz, 1 H), 6.76 (tdd, J = 8.9, 2.8, 0.9 Hz, 1H), 4.20 (t, J = 6.7 Hz, 2H), 3.04 (q, J = 6.4 Hz, 2H), 2.92 (quintet, J = 8.7 Hz, 1H), 2.10-1.68 (m, 8H); m/z (ES+APCI)⁺: 393 [M+H]⁺.

### Example 38

### Thiophene-2-carboxylic acid {3-[5-chloro-2-(3-fluoro-phenylamino)-pyrrolo[2,3-d]pyrimidin-7-yl]-propyl}-amide

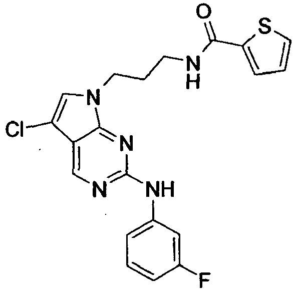

Prepared analogously to Example 27 from Intermediate 5 and 3-fluoroaniline to give a white solid. ¹H NMR (400 MHz, DMSO-*d*₆) δ_{H} ppm 2.03 (2 H, m), 3.21 (2 H, q, *J*=6.41 Hz), 4.17 (2 H, t, *J*=6.87 Hz), 6.62 - 6.69 (1 H, m), 7.08 (1 H, dd, *J*=5.04, 3.66 Hz), 7.16 - 7.24 (1 H, m), 7.53 - 7.55 (1 H, m), 7.56 (1 H, s), 7.66 (1 H, dd, *J*=3.89, 1.14 Hz), 7.69 (1 H, dd, *J*=5.04, 1.37 Hz), 7.81 (1 H, dt, *J*=12.48, 2.23 Hz), 8.50 (1 H, t, *J*=5.72 Hz), 8.70 (1 H, s), 9.84 (1 H, s); m/z (ES+APCI)⁺: 430 / 432 [M+H]⁺.

### Example 39

### Cyclobutanecarboxylic acid (3-{2-[4-(2-oxo-pyrrolidin-1-yl)-phenylamino]-pyrrolo[2,3-d]pyrimidin-7-yl}-propyl)-amide

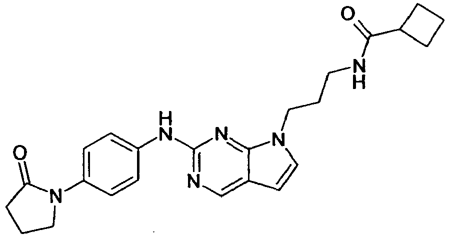

Intermediate 1 (40 mg, 0.137 mmol), 1-(4-amino-phenyl)-pyrrolidin-2-one (29 mg, 0.164 mmol), xantphos (6.3 mg, 0.011 mmol), Pd₂(dba)₃ (7.5 mg, 0.008 mmol) and sodium tert-butoxide (39 mg, 0.410 mmol) in dioxane (1.5 mL) were charged into a sealed Chromacol tube. The contents were degassed and placed under an atmosphere of nitrogen. The mixture was stirred and heated at 100 °C overnight. The mixture was cooled to rt and concentrated to dryness. The residue was dissolved in 5:1 DCM:MeOH, passed through a plug of silica gel and concentrated to dryness. The residue was dissolved in DMSO (1 mL) and purified by preparative HPLC (low pH buffer) to provide the product (21 mg, 35 %). ¹H NMR (400 MHz, DMSO-*d*₆) δH ppm 1.66 - 1.75 (m, 1 H) 1.79 - 1.99 (m, 5 H) 2.01 - 2.12 (m, 4 H) 2.44 - 2.48 (m, 2 H) 2.90 - 2.95 (m, 1 H) 2.99 - 3.06 (m, 2 H) 3.79 - 3.84 (m, 2 H) 4.14 (t, *J*=6.87 Hz, 2 H) 6.43 (d, *J*=3.21 Hz, 1 H) 7.28 (d, *J*=3.66 Hz, 1 H) 7.53 - 7.57 (m, 2 H) 7.70 (t, *J*=5.72 Hz, 1 H) 7.83 - 7.87 (m, 2 H) 8.67 (s, 1 H) 9.42 (s, 1 H); m/z (ES+APCI)⁺: 433 [M+H]⁺.

### Example 40

### Cyclobutanecarboxylic acid {3-[2-(1H-indazol-5-ylamino)-pyrrolo[2,3-d]pyrimidin-7-yl]-propyl}-amide

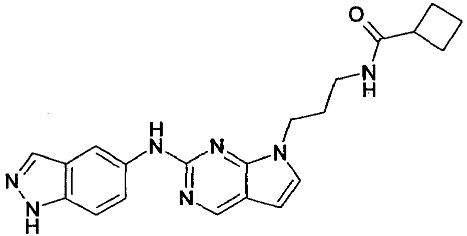

A mixture of Intermediate 1 (40 mg, 0.137 mmol), 1*H*-indazol-5-ylamine (44 mg, 0.331 mmol) and glacial acetic acid (78 µL, 1.37 mmol) in n-butanol (1 mL) was charged into a sealed microwave reactor vial. The reaction mixture was irradiated in the Biotage I-60 microwave reactor for 40 minutes at 150 °C. The mixture was concentrated, the residue was dissolved in DMSO (1 mL) and purified by preparative HPLC (high pH buffer) to provide the product (8.5 mg, 16 %). ¹H NMR (400 MHz, DMSO-*d*₆) δH ppm 1.64 - 1.73 (m, 1 H) 1.75 - 1.83 (m, 1 H) 1.87 - 1.99 (m, 4 H) 2.00 - 2.12 (m, 2 H) 2.85 - 2.92 (m, 1 H) 3.06 (q, *J*=6.56 Hz, 2 H) 4.16 (t, *J*=6.87 Hz, 2 H) 6.43 (d, *J*=3.66 Hz, 1 H) 7.28 (d, *J*=3.66 Hz, 1 H) 7.45 (d, *J*=8.70 Hz, 1 H) 7.63 (dd, *J*=9.16, 1.83 Hz, 1 H) 7.67 - 7.73 (m, 1 H) 8.00 (s, 1 H) 8.43 (d, *J*=1.37 Hz, 1 H) 8.68 (s, 1 H) 9.39 (s, 1 H); m/z (ES+APCI)⁺: 390 [M+H]⁺.

### Examples 41-53

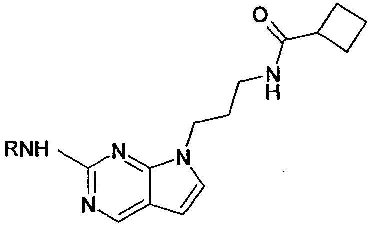

The following tabulated examples were synthesized analogously to Example 40 from Intermediate 1 and the appropriate amine:

| **Example** | **R** | **Name** | **m/z (ES+APCl)⁺** | **HPLC retention time (min)*** |
|---|---|---|---|---|
| **41** | 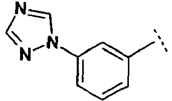 | Cyclobutanecarboxylic acid {3-[2-(3-1,2,4-triazol-1-yl-phenylamino)-pyrrolo[2,3-d]pyrimidin-7-yl]-propyl}-amide | 417 | 1.56 |
| **42** | 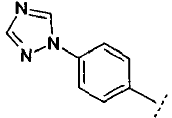 | Cyclobutanecarboxylic acid {3-[2-(4-1,2,4-triazol-1-yl-phenylamino)-pyrrolo[2,3-d]pyrimidin-7-yl]-propyl}-amide^{<} | 417 | 1.51 |
| **43** | 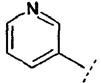 | Cyclobutanecarboxylic acid {3-[2-(pyridin-3-ylamino)-pyrrolo[2,3-d]pyrimidin-7-yl]-propyl}-amide | 351 | 1.42 |
| **44** | 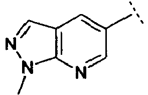 . | Cyclobutanecarboxylic acid {3-[2-(1-methyl-1H-pyrazolo[3,4-b]pyridin-5-ylamino)-pyrrolo[2,3-d]pyrimidin-7-yl]-propyl}-amide** | 405 | 1.46 |
| **45** | 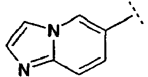 | Cyclobutanecarboxylic acid {3-[2-(imidazo[1,2-a]pyridin-6-ylamino)-pyrrolo[2,3-d]pyrimidin-7-yl]-propyl}-amide | 390 | 1.42 |
| **46** | 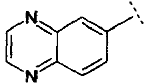 | Cyclobutanecarboxylic acid {3-[2-(quinoxalin-6-ylamino)-pyrrolo[2,3-d]pyrimidin-7-yl]-propyl}-amide | 402 | 1.51 |
| **47** | 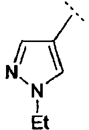 | Cyclobutanecarboxylic acid {3-[2-(1-ethyl-1H-pyrazol-4-ylamino)-pyrrolo[2,3-d]pyrimidin-7-yl]-propyl}-amide | 368 | 1.43 |
| **48** | 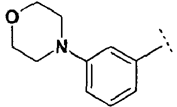 | Cyclobutanecarboxylic acid {3-[2-(3-morpholin-4-yl-phenylamino)-pyrrolo[2,3-d]pyrimidin-7-yl]-propyl}-amide | 435 | 1.70 |
| **49** | 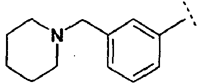 | Cyclobutanecarboxylic acid {3-[2-(3-piperidin-1-ylmethyl-phenylamino)-pyrrolo[2,3-d]pyrimidin-7-yl]-propyl}-amide | 447 | 1.99 |
| **50** | 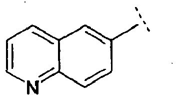 | Cyclobutanecarboxylic acid {3-[2-(quinolin-6-ylamino)-pyrrolo[2,3-d]pyrimidin-7-yl]-propyl}-amide | 401 | 1.58 |
| **51** | 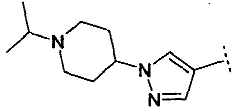 | Cyclobutanecarboxylic acid (3-{2-[1-(1-isopropyl-piperidin-4-yl)-1H-pyrazol-4-ylamino]-pyrrolo[2,3-d]pyrimidin-7-yl}-propyl}-amide | 465 | 1.59 |
| **52** | 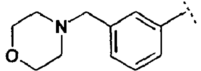 | Cyclobutanecarboxylic acid {3-[2-(3-morpholin-4-ylmethyl-phenylamino)-pyrrolo[2,3-d]pyrimidin-7-yl]-propyl}-amide | 449 | 1.62 |
| **53** | 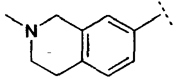 | Cyclobutanecarboxylic acid {3-[2-(2-methyl-1,2,3,4-tetrahydro-isoquinolin-7-ylamino)-pyrrolo[2,3-d]pyrimidin-7-yl]-propyl}-amide | 419 | 1.66 |

| | | | | |
|---|---|---|---|---|
| *HPLC column: 4.6x50mm (5µm) C-18 Xbridge; flow rate: 3ml/min; Run time: 3.2 min: Solvent A: 0.1% Ammonium Hydroxide in water Solvent B: Acetonitrile; Gradient - 10-100%B; Gradient time: 2.35min | | | | |

### Example 54

### Cyclobutanecarboxylic acid {3-[2-(4-oxazol-5-yl-phenylamino)-pyrrolo[2,3-d]pyrimidin-7-yl]-propyl}-amide

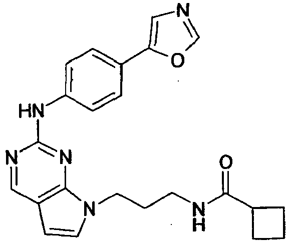

Cyclobutanecarboxylic acid [3-(2-chloro-pyrrolo[2,3-*d*]pyrimidin-7-yl)-propyl]-amide (40 mg, 0.137 mmol), 4-oxazol-5-yl-phenylamine (26.3 mg, 0.164 mmol), Pd₂(dba)₃ (7.5 mg, 0.008 mmol), xantphos (6.3 mg, 0.011 mmol) and sodium *tert*-butoxide (39.4 mg, 0.410 mmol) were combined with dioxane (1.5 mL) in a sealed chromacol tube, degassed and then purged with nitrogen gas. The reaction mixture was heated at 100°C overnight. The mixture was allowed to cool to rt, concentrated and purified through a silica plug, eluting with DCM/ methanol. The residue was purified by mass triggered preparative HPLC (low pH buffer). The purified material was passed through an aminopropyl cartridge to afford the product as a white solid (19 mg, 34%). ¹H NMR (400 MHz, DMSO-*d*₆) δH ppm 1.63 - 1.88 (m, 2 H) 1.93 (t, *J*=6.6 Hz, 4 H) 1.99 - 2.15 (m, 2 H) 2.93 (t, *J*=8.5 Hz, 1 H) 3.05 (q, *J*=6.0 Hz, 2 H) 4.17 (t, *J*=6.18 Hz, 2 H) 6.42 - 6.47 (m, 1 H) 7.33 (dd, *J*=3.6, 1.8 Hz, 1 H) 7.51 (s, 1 H) 7.56 - 7.75 (m, 3 H) 7.99 (d, *J*=7.3 Hz, 2 H) 8.37 (d, *J*=1.8 Hz, 1 H) 8.72 (d, *J*=1.8 Hz, 1 H) 9.67 (s, 1 H); m/z (ES+APCI)⁺: 417 [M+H]⁺.

### Examples 55-60

Examples 55-60 were prepared analogously to Example 54 from Intermediate 1 and the appropriate amine (the general structure is shown below followed by the tabulated examples).

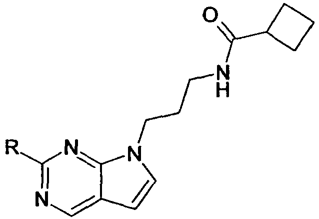

| **Example** | **R² group** | **Name** | **m/z (ES+APCI)⁺** | **HPLC retention time (min)*** |
|---|---|---|---|---|
| **55** | 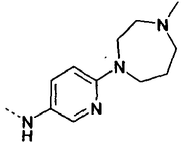 | Cyclobutanecarboxylic acid (3-{2-[6-(4-methyl-perhydro-1,4-diazepin-1-yl)-pyridin-3-ylamino]-pyrrolo[2,3-*d*]pyrimidin-7-yl}-propyl)-amide | 463 | 1.46 |
| **56** | 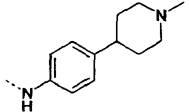 | Cyclobutanecarboxylic acid (3-{2-[4-(1-methyl-piperidin-4-yl)-phenylamino]-pyrrolo[2,3-*d*]pyrimidin-7-yl}-propyl)-amide | 447 | 1.76 |
| **57** | 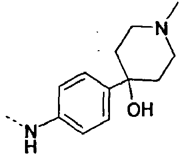 | Cyclobutanecarboxylic acid (3-{2-[4-(4-hydroxy-1-methyl-piperidin-4-yl)-phenylamino]-pyrrolo[2,3-*d*]pyrimidin-7-yl}-propyl)-amide | 463 | 1.39 |
| **58** | 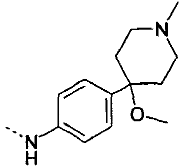 | Cyclobutanecarboxylic acid (3-{2-[4-(4-methoxy-1-methyl-piperidin-4-yl)-phenylamino]-pyrrolo[2,3-*d*]pyrimidin-7-yl}-propyl)-amide | 477 | 1.64 |
| **59** | 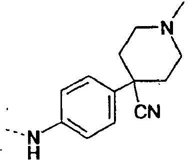 | Cyclobutanecarboxylic acid (3-{2-[4-(4-cyano-1-methyl-piperidin-4-yl)-phenylamino]-pyrrolo[2,3-*d*]pyrimidin-7-yl}-propyl)-amide | 472 | 1.63 |
| **60** | 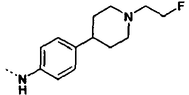 | Cyclobutanecarboxylic acid [3-(2-[4-[1-(2-fluoro-ethyl)-piperidin-4-yl]-phenylamino}-pyrrolo[2,3-*d*]pyrimidin-7-yl)-propyl]-amide | 479 | 1.80 |

| | | | | |
|---|---|---|---|---|
| *HPLC column: 4.6x50mm (5µm) C-18 Xbridge; flow rate: 3ml/min; Run time: 3.2 min: Solvent A: 0.1 % Ammonium Hydroxide in water Solvent B: Acetonitrile; Gradient - 10-100%B; Gradient time: 2.35min. | | | | |

### Results

All compounds exemplified below have IC₅₀ values against TBK1 of 10 µM or better. Table 2 shows a potency score for each compound (*** = TBK1 IC₅₀ <100 nM; ** = TBK1 IC₅₀ between 100 nM and 1 µM; * = TBK1 IC₅₀ between 1 µM and 10 µM).

### REFERENCES

1. Rezaie, T., Child, A., Hitchings, R., Brice, G., Miller, L., Coca-Prados, M., Heon, E., Krupin, T., Ritch, R., Kreutzer, D., Crick, R.P. and Sarfarazi, M. (2002) Adult-onset primary open-angle glaucoma caused by mutations in optineurin. Science 295, 1077-1079.
2. Sarfarazi, M. and Rezaie, T. (2003) Optineurin in primary open angle glaucoma. Ophthalmol Clin North Am 16, 529-541.
3. Tezel, G. and Wax, M.B. (2000) Increased production of tumour necrosis factor-alpha by glial cells exposed to stimulated ischemia or elevated hydrostatic pressure induces apoptosis in cocultured retinal ganglion cells. J Neurosci 20, 8693-8700.
4. Yuan, L. and Neufeld, A.H. (2000) Tumor necrosis factor-alpha: a potentially neurodestructive cytokine produced by glia in the human glaucomatous optic nerve head. Glia 32, 42-50.
5. Perry et al (J Exp Med 199, 1651-1658, 2004) compared the role of TBK1 in interferon responses induced by a number of stimuli. TBK-/- mice were deficient in their ability to up regulate IFN beta production.
6. McWhirter et al (PNAS 101, 233 238, 2004) Demonstrate that induction of type I interferon and related genes depends on TBK1. They also show that IKKepsilon and TBK1 directly phosphorylate serine residues that are critical for IRF3 activation.
7. Hemmi et al (J Exp Med 199, 1641-1650, 2004) indicate that TBK1 and IKK are essential for the activation of IFN beta and IFN inducible genes.
8. Davies, S. P., Reddy, H., Caivano, M. and Cohen, P. (2000) Specificity and mechanism of action of some commonly used protein kinase inhibitors. Biochem J 351, 95-105.
9. Bain, J., McLauchlan, H., Elliott, M. and Cohen, P. (2003) The specificities of protein kinase inhibitors: an update. Biochem J 371, 199-204.
10. Schwambom, K., Weil, R., Courtois, G., Whiteside, S.T. and Israel, A. (2000) Phorbol esters and cytokines regulate the expression of the NEMO-related protein, a molecule involved in a NF-kappa B-independent pathway. J Biol Chem 275, 22780-22789.
11. 11. Morton, S., Hesson, L., Peggie, M. and Cohen, P. (2008) Enhanced binding of TBK1 by an optineurin mutant that causes a familial form of primary open angle glaucoma. FEBS Letters 582, 997-1002.

**Table 1: Selected compounds according to the invention**

| | | | |
|---|---|---|---|
| 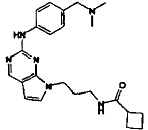 | Example 5 | 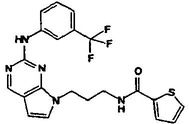 | Example 13 |
| 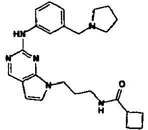 | Example 6 | 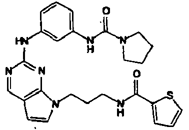 | Example 14 |
| 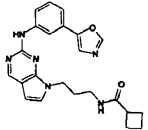 | Example 7 | 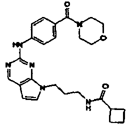 | Example 15 |
| 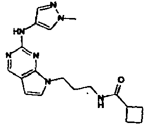 | Example 8 | 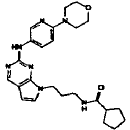 | Example 16 |
| 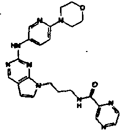 | Example 17 | 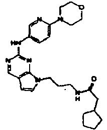 | Example 25 |
| 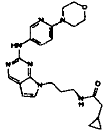 | Example 18 | 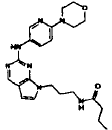 | Example 26 |
| 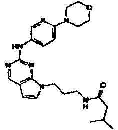 | Example 19 | 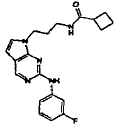 | Example 27 |
| 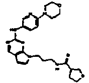 | Example 20 | 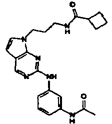 | Example 28 |
| 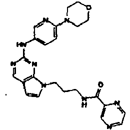 | Example 17 | 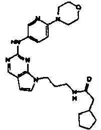 | Example 25 |
| 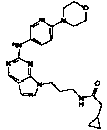 | Example 18 | 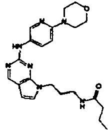 | Example 26 |
| 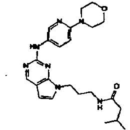 | Example 19 | 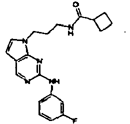 | Example 27 |
| 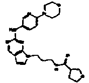 | Example 20 | 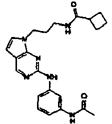 | Example 28 |
| 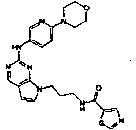 | Example 21 | 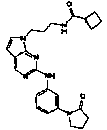 | Example 29 |
| 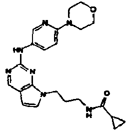 | Example 22 | 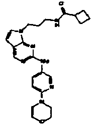 | Example 30 |
| 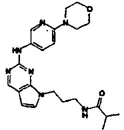 | Example 23 | 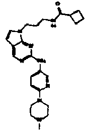 | Example 31 |
| 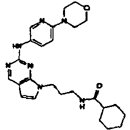 | Example 24 | 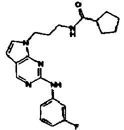 | Example 32 |
| 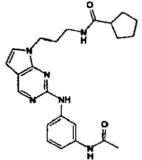 | Example 33 | | |
| 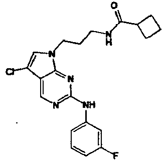 | Example 34 | | |
| 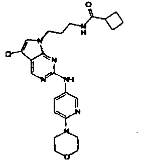 | Example 35 | | |
| 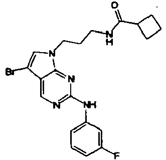 | Example 36 | | |
| 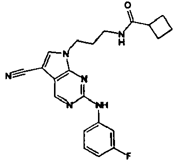 | Example 37 | | |
| 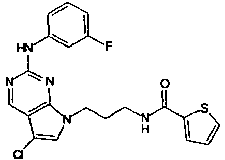 | Example 38 | | |

| Structure | Example | Strucrture | Example |
|---|---|---|---|
| 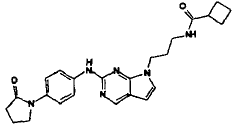 | Example 39 | 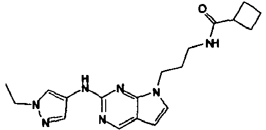 | Example 47 |
| 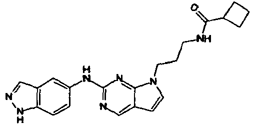 | Example 40 | 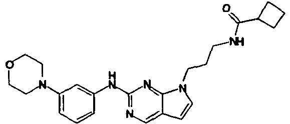 | Example 48 |
| 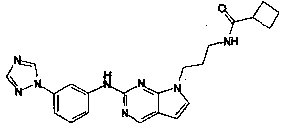 | Example 41 | 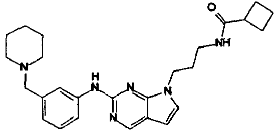 | Example 49 |
| 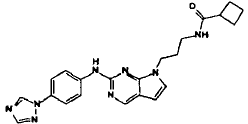 | Example 42 | 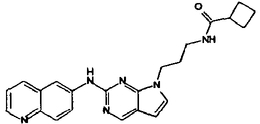 | Example 50 |
| 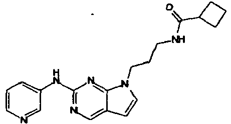 | Example 43 | 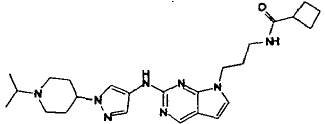 | Example 51 |
| 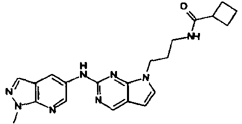 | Example 44 | 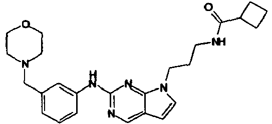 | Example 52 |
| 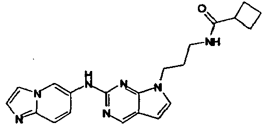 | Example 45 | 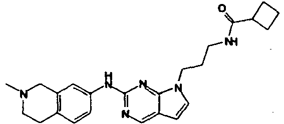 | Example 53 |
| 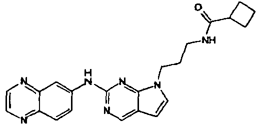 | Example 46 | 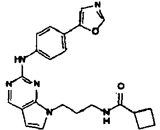 | Example 54 |

| Structure | Example | | |
|---|---|---|---|
| 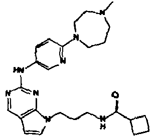 | Example 55 | | |
| 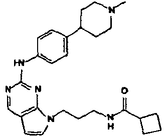 | Example 56 | | |
| 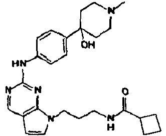 | Example 57 | | |
| 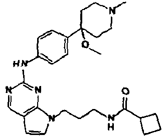 | Example 58 | | |
| 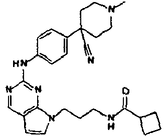 | Example 59 | | |
| 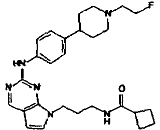 | Example 60 | | |

**Table 2: Potency scores for selected compounds of the invention**

| | |
|---|---|
| *** = TBK1 IC₅₀ <100 nM | |
| ** = TBK1 IC₅₀ between 100 nM and 1 µM | |
| * = TBK1 IC₅₀ between 1 µM and 10 µM) | |
| | |
| Example 1 | *** |
| Example 2 | *** |
| Example 3 | *** |
| Example 4 | *** |
| Example 5 | *** |
| Example 6 | *** |
| Example 7 | *** |
| Example 8 | *** |
| Example 9 | ** |
| Example 10 | *** |
| Example 11 | ** |
| Example 12 | ** |
| Example 13 | * |
| Example 14 | ** |
| Example 15 | *** |
| Example 16 | *** |
| Example 17 | ** |
| Example 18 | ** |
| Example 19 | ** |
| Example 20 | *** |
| Example 21 | ** |
| Example 22 | ** |
| Example 23 | * |
| Example 24 | ** |
| Example 25 | ** |
| Example 26 | ** |
| Example 27 | *** |
| Example 28 | *** |
| Example 29 | ** |
| Example 30 | *** |
| Example 31 | ** |
| Example 32 | ** |
| Example 33 | *** |
| Example 34 | *** |
| Example 35 | *** |
| Example 36 | *** |
| Example 37 | ** |
| Example 38 | ** |
| | |
| | |
| Example 39 | *** |
| Example 40 | *** |
| Example 41 | *** |
| Example 42 | *** |
| Example 43 | *** |
| Example 44 | *** |
| Example 45 | *** |
| Example 46 | *** |
| Example 47 | *** |
| Example 48 | *** |
| Example 49 | *** |
| Example 50 | *** |
| Example 51 | *** |
| Example 52 | *** |
| Example 53 | *** |
| Example 54 | *** |
| Example 55 | ** |
| Example 56 | *** |
| Example 57 | *** |
| Example 58 | *** |
| Example 59 | *** |
| Example 60 | *** |

## Claims

1. A compound of formula (I), or a pharmaceutically acceptable salt or ester thereof, 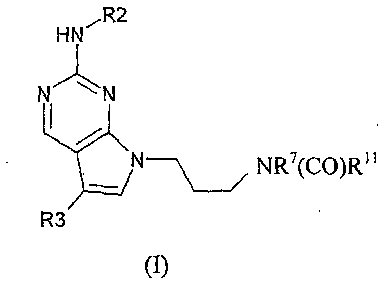 wherein:
R² is aryl, heteroaryl, fused aryl -C₃₋₆-heterocycloalkyl or fused heteroaryl-C₃₋₆-heterocycloalkyl, each of which is optionally substituted by one or more substituents selected from aryl, heteroaryl, C₁₋₆-alkyl, C₃₋₆-heterocycloalkyl and a group A, wherein said C₁₋₆-alkyl group is in turn optionally substituted by one or more substituents selected from aryl, heteroaryl, C₃₋₆-heterocycloalkyl and a group A, said heteroaryl group is optionally substituted by one or more R¹⁰ groups; and wherein each C₃₋₆-heterocycloalkyl group is optionally substituted by one or more groups selected from C₁₋₆-alkyl, C₁₋₆-haloalkyl, and A, and which optionally contains one or more groups selected from oxygen, sulphur, nitrogen and CO;
R³ is H, halogen, cyano or C₁₋₆-alkyl;
A is selected from halogen, hydroxyl, cyano, trifluoromethyl, -NO₂, -NH₂, -NR⁴R⁵, -OR⁶, -NR⁷(CO)R⁶, -NR⁷(CO)NR⁴R⁵, -NR⁷COOR⁷, -NR⁷(SO₂)R⁶, -CO₂H, -NR⁷(SO₂)NR⁴R⁵, -COOR⁷, -CONR⁴R⁵, COR⁶ and -SO₂CH₃;
each R⁴ and R⁵ is independently selected from hydrogen, C₃₋₇-cycloalkyl, aryl, heteroaryl, C₁₋₆-alkyl and a C₃₋₆-heterocycloalkyl ring optionally further containing one or more groups selected from oxygen, sulfur, nitrogen and CO, and optionally substituted by one or more R¹⁰ groups, wherein said C₁₋₆-alkyl is optionally substituted by one or more substituents selected from halogen, cyano, hydroxyl, aryl, heteroaryl, -NR⁸R⁹, -NR⁷(CO)R⁶, -NR⁷COOR⁶, -NR⁷(SO₂)R⁶, -COOR⁶, -CONR⁸R⁹, OR¹⁰, -SO₂R⁶ and a C₃₋₆-heterocycloalkyl ring optionally further containing one or more groups selected from oxygen, sulfur, nitrogen and CO and optionally substituted by one or more or R¹⁰ groups; or
R⁴ and R⁵ together with the N to which they are attached form a C₃₋₆-heterocycloalkyl ring optionally further containing one or more groups selected from oxygen, sulfur, nitrogen and CO, wherein said C₃₋₆-heterocycloalkyl ring may be saturated or unsaturated and is optionally substituted with one or more groups selected from NR⁸R⁹ and R¹⁰;
each R⁶ is independently selected from C₁₋₆-alkyl, C₃₋₇ cycloalkyl, C₄₋₇-heterocycloalkyl, aryl and heteroaryl, each of which may be optionally substituted by one or more substituents selected from halogen, R¹⁰ and -NR⁸R⁹;
each R⁷ is selected from hydrogen, C₁₋₆-alkyl and C₃₋₇-cycloalkyl, wherein said C₁₋₆-alkyl is optionally substituted by one or more halogens;
each of R⁸ and R⁹ is independently selected from hydrogen and C₁₋₆-alkyl, wherein said C₁₋₆-alkyl group is optionally substituted by one or more halogens; or
R⁸ and R⁹ together with the N to which they are attached form a C₄₋₆-heterocycloalkyl ring optionally further containing one or more heteroatoms selected from oxygen and sulfur, wherein said C₄₋₆-heterocycloalkyl ring is optionally substituted by one or more R¹⁰ groups; and
each R¹⁰ is selected from C₃₋₇-cycloalkyl and C₁₋₆-alkyl optionally substituted by one or more halogens, wherein where R¹⁰ is C₁₋₆-alkyl and two or more R¹⁰ groups are attached to the same carbon atom, the R¹⁰ groups may be linked to form a spiroalkyl group; and
each R¹¹ is independently selected from C₁₋₆-alkyl, C₃₋₇-cycloalkyl, C₁₋₆-alkyl-C₃₋₇-cycloalkyl, C₄₋₇-heterocycloalkyl, aryl and heteroaryl, each of which may be optionally substituted by one or more substituents selected from A.

2. A compound according to claim 1 wherein NR⁷(CO)R¹¹ is selected from NHCO-C₁₋₆-alkyl, NHCO-C₃₋₇-cycloalkyl, NHCO-C₁₋₆-alkyl-C₃₋₇-cycloalkyl, NHCO-heteroaryl, NHCO-C₄₋₇-heterocycloalkyl.

3. A compound according to claim 1 or claim 2 wherein NR⁷(CO)R¹¹ is NHCO-C₃₋₇-cycloalkyl.

4. A compound according to claim 1 or claim 2 wherein NR⁷(CO)R¹¹ is selected from NHCO-cyclobutyl, NHCO-thienyl, NHCO-cyclopentyl, NHCO-pyrazinyl, NHCOCH₂-cyclopropyl, NHCO-sec-butyl, NHCO-tetrahydrofuranyl, NHCO-thiazolyl, NHCO-cyclopropyl, NHCO-isopropyl, NHCO-cyclohexyl, NHCOCH₂-cyclopentyl and NHCO-n-propyl.

5. A compound according to claim 4 wherein NR⁷(CO)R¹¹ is selected from NHCO-cyclobutyl and NHCO-cyclopentyl, NHCO-cyclohexyl and NHCO-thien-2-yl.

6. A compound according to any preceding claim wherein R² is aryl or heteroaryl, each of which is optionally substituted by one or more substituents selected from aryl, heteroaryl, C₁₋₆-alkyl, C₃₋₆-heterocycloalkyl and a group A, wherein said C₁₋₆-alkyl group is in turn optionally substituted by one or more substituents selected from aryl, heteroaryl, C₃₋₆-heterocycloalkyl group and a group A, said heteroaryl group is optionally substituted by one or more R¹⁰ groups; and wherein said C₃₋₆-heterocycloalkyl group optionally contains one or more groups selected from oxygen, sulphur, nitrogen and CO, and is optionally substituted by one or more alkyl or A groups.

7. A compound according to any preceding claim wherein R² is selected from aryl or heteroaryl, each of which is optionally substituted by one or more substituents selected from halo, optionally substituted C₃₋₇-heterocycloalkyl, optionally substituted C₁₋₆-alkyl, heteroaryl, C₁₋₆-alkyl-C₃₋₇-heterocycloalkyl, CN, NHCO-C₃₋₇-heterocycloalkyl, CO-C₃₋₇-heterocycloalkyl and NHCO-C₁₋₆-alkyl, wherein said C₃₋₇-heterocycloalkyl is optionally substituted by one or more C₁₋₆-alkyl or A groups, and said C₁₋₆-alkyl is optionally substituted by one or more halo or NR⁴R⁵ groups.

8. A compound according to any one of claims 1 to 5 wherein R² is selected from phenyl, pyridin-3-yl, pyrazol-4-yl, indazol-5-yl, indazol-6-yl, quinolinyl, quinoxalinyl, pyrazolopyridinyl, imidazopyridinyl and tetrahydroisoquinolinyl, each of which may be optionally substituted.

9. A compound according to any one of claims 1 to 5 wherein R² is selected from:
(i) phenyl optionally substituted by:
halo, optionally substituted C₃₋₇-heterocycloalkyl, optionally substituted C₁₋₆-alkyl, heteroaryl, C₁₋₆-alkyl-C₃₋₇-heterocycloalkyl, CN, NHCO-C₃₋₇-heterocycloalkyl, CO-C₃₋₇-heterocycloalkyl or NHCO-C₁₋₆-alkyl, wherein said C₃₋₇-heterocycloalkyl is optionally substituted by one or more C₁₋₆-alkyl, CN, OH, alkoxy, haloalkyl, COR⁶ groups, and said C₁₋₆-alkyl is optionally substituted by one or more halo or NR⁴R⁵ groups;
(ii) pyridinyl optionally substituted by C₃₋₇-heterocycloalkyl, wherein said C₃₋₇-heterocycloalkyl is optionally further substituted by one or more C₁₋₆-alkyl groups;
(iii) pyrazolyl substituted by C₁₋₆-alkyl, C₁₋₆-alkyl-C₃₋₇-heterocycloalkyl or C₃₋₇-heterocycloalkyl, wherein said C₃₋₇-heterocycloalkyl is optionally further substituted by one or more C₁₋₆-alkyl groups;
(iv) indazolyl optionally substituted by C₁₋₆-alkyl;
(v) quinolinyl;
(vi) quinoxalinyl;
(vii) pyrazolopyridinyl;
(viii) imidazopyridinyl; and
(ix) tetrahydroisoquinolinyl.

10. A compound according to any preceding claim wherein R² is selected from:
(i) phenyl optionally substituted by F, N-morpholinyl, N-methylpiperazinyl, CH₂-NMe₂, CH₂-pyrrolidinyl, oxazolyl, CN, CF₃, NHCO-pyrrolidinyl, CO-moipholinyl, NHCOMe, 2-oxopyrrolidin-1-yl, 1,2,4-triazol-1-yl, 4-hydroxy-1-methylpiperidin-4-yl, 1-methyl-piperidin-4-yl, 4-methoxy-1-methylpiperidin-4-yl, morpholin-4-yl-methyl, 4-cyano-1-methylpiperidin-4-yl, piperidin-1-yl-methyl or 1-(2-fluoroethyl)-piperidin-4-yl,
(ii) pyridinyl optionally substituted by morpholinyl or 4-methyl-perhydro-1,4-diazepin-1-yl;
(iii) pyrazolyl optionally substituted by Me, Et, CH₂CH₂-morpholinyl, or 1-isopropyl-piperidin-4-yl;
(iv) indazolyl optionally substituted by Me.

11. A compound according to any one of claims 1 to 9 wherein R² is selected from:
pyridin-3-yl, 6-(morpholin-4-yl)-pyridin-3-yl, 6-(4-methylpiperazin-1-yl)-pyridin-3-yl, 1-Me-1*H*-pyrazol-4-yl, 1-(2-morpholin-4-yl-ethyl)-1*H*-pyrazol-4-yl, 1-Me-2*H*-indazol-5-yl, 1-Me-1*H*-indazol-6-yl, 3-fluorophenyl, 3-trifluoromethylphenyl, 3-cyanophenyl, 3-oxazol-5-yl-phenyl, 3-acetylaminophenyl, 4-dimethylaminomethylphenyl, 4-(4-methyl-piperazin-1-yl)-phenyl, 3-pymolidin-1-yl-methylphenyl, 4-(morpholin-4-yl)-phenyl, 4-(morpholine-4-carbonyl)-phenyl, 3-(2-oxo-pyrrolidin-1-yl)-phenyl, 3-(pyrrolidin-1-yl-carboxyamino)-phenyl, 4-(2-oxo-pyrrolidin-1-yl)-phenyl, 1*H*-indazol-5-yl, 3-(1,2,4-triazol-1-yl-phenyl), 4-(1,2,4-triazol-1-yl-phenyl), quinoxalin-6-yl, quinolin-6-yl, imidazo[1,2-a]pyridine-6-yl, 1-methyl-1*H*-pyrazolo[3,4b]pyridine-5-yl, 1-ethyl-1*H*-pyrazol-4-yl, piperidin-1-yl-methylphenyl, (1-isopropyl-piperidin-4-yl)-1*H*-pyrazol-4-yl, 6-(4-methyl-perhydro-1,4-diazepin-1-yl)-pyridin-3-yl, morpholin-4-yl-methyl-phenyl, 2-methyl-1,2,3,4-tetrahydroisoquinolin-7-yl, 4-oxazol-5-yl-phenyl, 4-(1-methylpiperidin-4-yl)-phenyl, 4-(4-hydroxy-1-methyl-piperidin-4-yl)-phenyl, 4-(4-methoxy-1-methyl-piperidin-4-yl)-phenyl, 4-(4-cyano-1-methyl-piperidin-4-yl)-phenyl and 4-(1-(2-fluoroethyl)-piperidin-4-yl)-phenyl.

12. A compound according to any preceding claim wherein R³ is selected from H, halo and CN

13. A compound according to any preceding claim wherein R⁷ is selected from H and C₁₋₆-alkyl, more preferably H.

14. A compound according to any preceding claim which is selected from the following:
Cyclobutanecarboxylic acid {3-[2-(1-methyl-1H-indazol-5-ylamino)-pyrrolo[2,3-d]pyrimidin-7-yl]-propyl}-amide [1];
Cyclobutanecarboxylic acid {3-[2-(1-methyl-1H-indazol-6-ylamino)-pyrriolo[2,3-d]pyrimidin-7-yl]-propyl}-amide [2].;
Cyclobutanecarboxylic acid {3-[2-(4-morpholin-4-yl-phenylamino)-pyrrolo[2,3-d]pyrimidin-7-yl]-propyl}-amide [3];
Cyclobutanecarboxylic acid (3-{2-[4-(4-methyl-piperazin-1-yl)-phenylamino]-pyrrolo[2,3-d]pyrimidin-7-yl}-propyl)-amide [4];
Cyclobutanecarboxylic acid {3-[2-(4-dimethylaminomethyl-phenylamino)-pyrrolo[2,3-d]pyrimidin-7-yl]-propyl}-amide [5];
Cyclobutanecarboxylic acid {3-[2-(3-pyrrolidin-1-ylmethyl-phenylamino)-pyrrolo[2,3-d]pyrimidin-7-yl]-propyl}-amide [6];
Cyclobutanecarboxylic acid {3-[2-(3-oxazol-5-yl-phenylamino)-pyrrolo[2,3-d]pyrimidin-7-yl]-propyl}-amide [7];
Cyclobutanecarboxylic acid {3-[2-{1-methyl-1H-pyrazol-4-ylamino)-pyrrolo[2,3-d]pyrimidin-7-yl]-propyl}-amide [8];
Cyclobutanecarboxylic acid (3-{2-[1-(2-morpholin-4-yl-ethyl)-1H-pyrazol-4-ylamino]-pyrrolo[2,3-d]pyrimidin-7-yl}-propyl)-amide [9];
Thiophene-2-carboxylic acid {3-[2-(3-fluoro-phenylamino)-pyrrolo[2,3-d]pyrimidin-7-yl]-propyl}-amide [10];
Thiophene-2-carboxylic acid {3-[2-(3-cyano-phenylamino)-pyrrolo[2,3-d]pyrimidin-7-yl]-propyl}-amide [11];
Thiophene-2-carboxylic acid {3-[2-(pyridin-3-ylamino)-pyrrolo[2,3-d]pyrimidin-7-yl]-propyl}-amide [12];
Thiophene-2-carboxylic acid {3-[2-(3-trifluoromethyl-phenylamino)-pyrrolo[2,3-d]pyrimidin-7-yl]-propyl}-amide [13];
Pyrrolidine-1-carboxylic acid [3-(7-{3-[(thiophene-2-carbonyl)-amino]-propyl}-7H-pyrrolo[2,3-d]pyrimidin-2-ylamino)-phenyl]-amide [14];
Cyclobutanecarboxylic acid (3-{2-[4-(morpholine-4-carbonyl)-phenylamino]-pyrrolo[2,3-d]pyrimidin-7-yl}-propyl)-amide [15];
Cyclopentanecarboxylic acid {3-[2-(6-morpholin-4-yl-pyridin-3-ylamino)-pyrrolo[2,3-d]pyrimidin-7-yl]-propyl}-amide [16];
Pyrazine-2-carboxylic acid {3-[2-(6-morpholin-4-yl-pyridin-3-ylamino)-pyrrolo[2,3-d]pyrimidin-7-yl]-propyl}-amide [17];
2-Cyclopropyl-N-{3-[2-(6-morpholin-4-yl-pyridin-3-ylamino)-pyrrolo[2,3-d]pyrimidin-7-yl]-propyl}-acetamide [18];
3-Methyl-N-{3-[2-(6-morpholin-4-yl-pyridin-3-ylamino)-pyrrolo[2,3-d]pyrimidin-7-yl]-propyl}-butyramide [19];
Tetrahydro-furan-3-carboxylic acid {3-[2-(6-morpholin-4-yl-pyridin-3-ylamino)-pyrrolo[2,3-d]pyrimidin-7-yl]-propyl}-amide [20];
Thiazole-5-carboxylic acid {3-[2-(6-morpholin-4-yl-pyridin-3-ylamino)-pyrrolo[2,3-d]pyrimidin-7-yl]-propyl}-amide [21];
Cyclopropanecarboxylic acid {3-[2-(6-morpholin-4-yl-pyridin-3-ylamino)-pyrrolo[2,3-d]pyrimidin-7-yl]-propyl}-amide [22];
N-{3-[2-(6-Morpholin-4-yl-pyridin-3-ylamino)-pyrrolo[2,3-d]pyrimidin-7-yl]-propyl}-isobutyramide [23];
Cyclohexanecarboxylic acid {3-[2-(6-morpholin-4-yl-pyridin-3-ylamino)-pyrrolo[2,3-d]pyrimidin-7-yl]-propyl}-amide [24];
2-Cyclopentyl-N-{3-[2-(6-morpholin-4-yl-pyridin-3-ylamino)-pyrrolo[2,3-d]pyrimidin-7-yl]-propyl}-acetamide [25];
N-{3-[2-(6-Morpholin-4-yl-pyridin-3-ylamino)-pynolo[2,3-d]pyrimidin-7-yl]-propyl}-butyramide [26];
Cyclobutanecarboxylic acid {3-[2-(3-fluoro-phenylamino)-pyrrolo[2,3-d]pyrimidin-7-yl]-propyl}-amide [27];
Cyclobutanecarboxylic acid {3-[2-(3-acetylamino-phenylamino)-pyrrolo[2,3-d]pyrimidin-7-yl]-propyl}-amide [28];
Cyclobutanecarboxylic acid (3-{2-[3-(2-oxo-pyrrolidin-1-yl)-phenylamino]-pyrrolo[2,3-d]pyrimidin-7-yl}-propyl)-amide [29];
Cyclobutanecarboxylic acid {3-[2-(6-morpholin-4-yl-pyridin-3-ylamino)-pyrrolo[2,3-d]pyrimidin-7-yl]-propyl}-amide [30];
Cyclobutanecarboxylic acid (3-{2-[6-(4-methyl-piperazin-1-yl)-pyridin-3-ylamino]-pyrrolo[2,3-d]pyrimidin-7-yl}-propyl)-amide [31];
Cyclopentanecarboxylic acid {3-[2-(3-fluoro-phenylamino)-pyrrolo[2,3-d]pyrimidin-7-yl]-propyl}-amide [32];
Cyclopentanecarboxylic acid {3-[2-(3-acetylamino-phenylamino)-pyrrolo[2,3-d] pyrimidin-7-yl]-propyl}-amide [33];
Cyclobutanecarboxylic acid {3-[5-chloro-2-(3-fluoro-phenylamino)-pyrrolo[2,3-d]pyrimidin-7-yl]-propyl}-amide [34];
Cyclobutanecarboxylic acid {3-[5-chloro-2-(6-morpholin-4-yl-pyridin-3-ylamino)-pyrrolo[2,3-d]pyrimidin-7-yl]-propyl}-amide [35];
Cyclobutanecarboxylic acid {3-[5-bromo-2-(3-fluoro-phenylamino)-pyrrolo[2,3-d]pyrimidin-7-yl]-propyl}-amide [36];
Cyclobutanecarboxylic acid {3-[5-cyano-2-(3-fluoro-phenylamino)-pyrrolo[2,3-d]pyrimidin-7-yl]-propyl}-amide [37];
Thiophene-2-carboxylic acid {3-[5-chloro-2-(3-fluoro-phenylamino)-pyrrolo[2,3-d]pyrimidin-7-yl]-propyl}-amide [38];
Cyclobutanecarboxylic acid (3-{2-[4-(2-oxo-pyrrolidin-1-yl)-phenylamino]-pyrrolo[2,3-d]pyrimidin-7-yl}-propyl)-amide [39];
Cyclobutanecarboxylic acid {3-[2-(1H-indazol-5-ylamino)-pyrrolo[2,3-d]pyrimidin-7-yl]-propyl}-amide[40];
Cyclobutanecarboxylic acid {3-[2-(3-1,2,4-triazol-1-yl-phenylamino)-pyrrolo[2,3-d]pyrimidin-7-yl]-propyl}-amide [41];
Cyclobutanecarboxylic acid {3-[2-(4-1,2,4-triazol-1-yl-phenylamino)-pyrrolo[2,3-d]pyrimidin-7-yl]-propyl}-amide [42];
Cyclobutanecarboxylic acid {3-[2-(pyridin-3-ylamino)-pyrrolo[2,3-d]pyrimidin-7-yl]-propyl}-amide [43];
Cyclobutanecarboxylic acid {3-[2-(1-methyl-1H-pyrazolo[3,4-b]pyridin-5-ylamino)-pyrrolo[2,3-d]pyrimidin-7-yl]-propyl}-amide [44];
Cyclobutanecarboxylic acid {3-[2-(imidazo[1,2-a]pyridin-6-ylamino)-pyrrolo[2,3-d]pyrimidin-7-yl]-propyl}-amide [45];
Cyclobutanecarboxylic acid {3-[2-(quinoxalin-6-ylamino)-pyrrolo[2,3-d]pyrimidin-7-yl]-propyl}-amide [46];
Cyclobutanecarboxylic acid {3-[2-(1-ethyl-1H-pyrazol-4-ylamino)-pyrrolo[2,3-d]pyrimidin-7-yl]-propyl}-amide [47];
Cyclobutanecarboxylic acid {3-[2-(3-morpholin-4-yl-phenylamino)-pyrrolo[2,3-d]pyrimidin-7-yl]-propyl}-amide [48];
Cyclobutanecarboxylic acid {3-[2-(3-piperidin-1-ylmethyl-phenylamino)-pyrrolo[2,3-d]pyrimidin-7-yl]-propyl}-amide [49];
Cyclobutanecarboxylic acid {3-[2-(quinolin-6-ylamino)-pyrrolo[2,3-d]pyrimidin-7-yl]-propyl}-amide [50];
Cyclobutanecarboxylic acid (3-{2-[1-(1-isopropyl-piperidin-4-yl)-1H-pyrazol-4-ylamino]-pyrrolo[2,3-d]pyrimidin-7-yl}-propyl)-amide [51];
Cyclobutanecarboxylic acid {3-[2-(3-morpbolin-4-ylmethyl-phenylamino)-pyrrolo[2,3-d]pyrimidin-7-yl]-propyl}-amide [52];
Cyclobutanecarboxylic acid {3-[2-(2-methyl-1,2,3,4-tetrahydro-isoquinolin-7-ylamino)-pyrrolo[2,3-d]pyrimidin-7-yl]-propyl}-amide [53];
Cyclobutanecarboxylic acid {3-[2-(4-oxazol-5-yl-phenylamino)-pyrrolo[2,3-d]pyrimidin-7-yl]-propyl}-amide [54];
Cyclobutanecarboxylic acid (3-{2-[6-(4-methyl-perhydro-1,4-diazepin-1-yl)-pyridin-3-ylamino]-pyrrolo[2,3-d]pyrimidin-7-yl}-propyl)-amide [55];
Cyclobutanecarboxylic acid (3-{2-[4-(1-methyl-piperidin-4-yl)-phenylamino]-pyrrolo[2,3-d]pyrimidin-7-yl}-propyl)-amide [56];
Cyclobutanecarboxylic acid (3-{2-[4-(4-hydroxy-1methyl-piperidin-4-yl)-phenylamino]-pyrrolo[2,3-d]pyrimidin-7-yl}-propyl)-amide [57];
Cyclobutanecarboxylic acid (3-{2-[4-(4-methoxy-1-methyl-piperidin-4-yl)-phenylamino]-pyrrolo[2,3-d]pyrimidin-7-yl}--propyl)-amide [58];
Cyclobutanecarboxylic acid (3-{2-[4-(4-cyano-1-methyl-piperidin-4-yl)-phenylamino]-pyrrolo[2,3-d]pyrimidin-7-yl}-propyl)-amide [59] and;
Cyclobutanecarboxylic acid [3-(2-{4-[1-(2-fluoro-ethyl)-piperidin-4-yl]-phenylamino}-pyrrolo[2,3-d]pyrimidin-7-yl)-propyl]-amide [60].

15. A pharmaceutical composition comprising a compound according to any one of claims 1 to 14 and a pharmaceutically acceptable carrier, diluent or excipient.

16. A compound according to any one of claims 1 to 14 for use in medicine.

17. A compound according to any one of claims 1 to 14 for use in treating or preventing a disorder selected from cancer, septic shock, Primary open Angle Glaucoma (POAG), hyperplasia, rheumatoid arthritis, psoriasis, artherosclerosis, retinopathy, osteoarthritis, endometriosis, chronic inflammation and Alzheimer's disease.

18. Use of a compound according to any one of claims 1 to 14 in the preparation of a medicament for treating or preventing a disorder selected from cancer, septic shock, Primary open Angle Glaucoma (POAG), hyperplasia, rheumatoid arthritis, psoriasis, artherosclerosis, retinopathy, osteoarthritis, endometriosis, chronic inflammation and Alzheimer's disease.

19. Use of a compound according to any one of claims 1 to 14 in the preparation of a medicament for the prevention or treatment of a disorder caused by, associated with or accompanied by any abnormal kinase activity, wherein the kinase is TBK1.

20. Use of a compound according to any one of claims 1 to 14 in an assay for identifying further candidate compounds capable of inhibiting TBK 1.

21. A process for preparing a compound of formula VII, wherein R¹¹ and R² are as defined in claim 1, said process comprising the steps of: 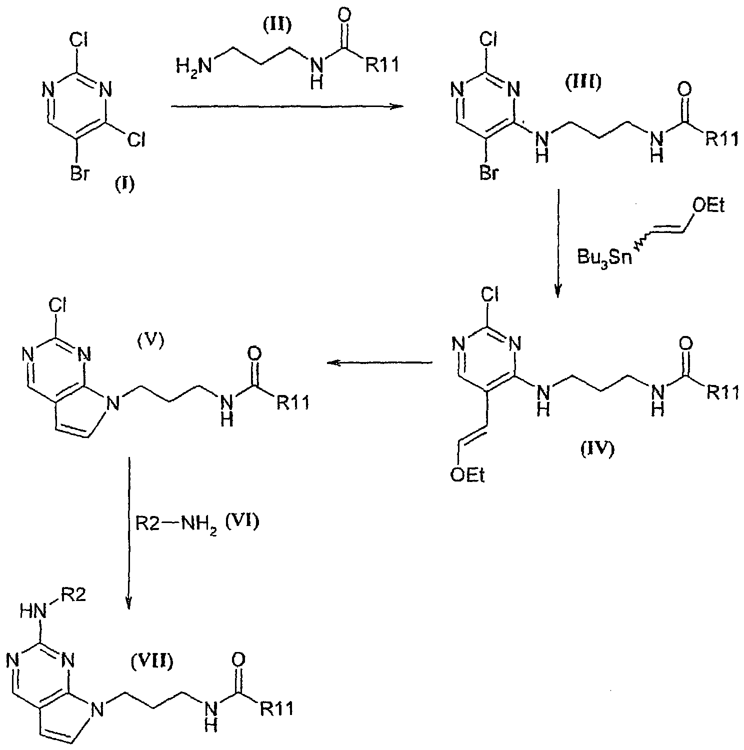
(i) reacting 5-bromo-2,4-dichloropyrimidine (I) with an amine of formula II to give a compound of formula III;
(ii) reacting said compound of formula III with ethoxyvinyltin to give a compound of formula IV;
(iii) cyclising said compound of formula IV to form a compound of formula V;
(iv) reacting said compound of formula V with an amine of formula VI to give a compound of formula VII.

22. A combination comprising a compound according to any one of claims 1 to 14 and a further therapeutic agent.

23. A pharmaceutical composition according to claim 15 which further comprises a second therapeutic agent.

## Patentansprüche

1. Verbindung der Formel (I) oder ein pharmazeutisch unbedenkliches Salz oder ein pharmazeutisch unbedenklicher Ester davon, 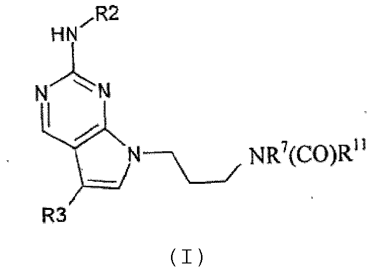 worin:
R² für Alkyl, Heteroaryl, anelliertes Aryl-C₃₋₆-heterocycloalkyl oder anelliertes Heteroaryl-C₃₋₆-heterocycloalkyl steht, wobei jede dieser Gruppen gegebenenfalls durch einen oder mehrere Substituenten, die aus Aryl, Heteroaryl, C₁₋₆-Alkyl, C₃₋₆-Heterocycloalkyl und einer Gruppe A ausgewählt sind, substituiert ist, wobei die C₁₋₆-Alkylgruppe wiederum gegebenenfalls durch einen oder mehrere Substituenten, die aus Aryl, Heteroaryl, C₃₋₆-Heterocycloalkyl und einer Gruppe A ausgewählt sind, substituiert ist, die Heteroarylgruppe gegebenenfalls durch eine oder mehrere R¹⁰-Gruppen substituiert ist und jede C₃₋₆-Hetero-cycloalkylgruppe gegebenenfalls durch einen oder mehrere Gruppen, die aus C₁₋₆-Alkyl, C₁₋₆-Halogenalkyl und A ausgewählt sind, substituiert ist und gegebenenfalls eine oder mehrere Gruppen, die aus Sauerstoff, Schwefel, Stickstoff und CO ausgewählt sind, enthält;
R³ für H, Halogen, Cyano oder C₁₋₆-Alkyl steht;
A aus Halogen, Hydroxyl, Cyano, Trifluormethyl, -NO₂, -NH₂, -NR⁴R⁵, -OR⁶, -NR⁷(CO)R⁶, -NR⁷(CO)NR⁴R⁵, -NR⁷COOR⁷, -NR⁷(SO₂)R⁶, -CO₂H, -NR⁷(SO₂)NR⁴R⁵, -COOR⁷, -CONR⁴R⁵, COR⁶ und -SO₂CH₃ ausgewählt ist;
R⁴ und R⁵ jeweils unabhängig voneinander aus Wasserstoff, C₃₋₇-Cycloalkyl, Aryl, Heteroaryl, C₁₋₆-Alkyl und einem C₃₋₆-Heterocycloalkylring, der gegebenenfalls ferner eine oder mehrere Gruppen, die aus Sauerstoff, Schwefel, Stickstoff und CO ausgewählt sind, enthält und gegebenenfalls durch eine oder mehrere R¹⁰-Gruppen substituiert ist, ausgewählt sind, wobei das C₁₋₆-Alkyl gegebenenfalls durch einen oder mehrere Substituenten, die aus Halogen, Cyano, Hydroxyl, Aryl, Heteroaryl, -NR⁸R⁹, -NR⁷(CO)R⁶, -NR⁷COOR⁷, -NR⁷(SO₂)R⁶, -COOR⁶, -CONR⁸R⁹, OR¹⁰, -SO₂R⁶ und einem C₃₋₆-Heterocyclo-alkylring, der gegebenenfalls ferner eine oder mehrere Gruppen, die aus Sauerstoff, Schwefel, Stickstoff und CO ausgewählt sind, enthält und gegebenenfalls durch eine oder mehrere R¹⁰-Gruppen substituiert ist, ausgewählt sind, substituiert ist; oder
R⁴ und R⁵ zusammen mit dem N, an das sie gebunden sind, einen C₃₋₆-Heterocycloalkylring bilden, der gegebenenfalls ferner eine oder mehrere Gruppen, die aus Sauerstoff, Schwefel, Stickstoff und CO ausgewählt sind, enthält, wobei der C₃₋₆-Hetero-cycloalkylring gesättigt oder ungesättigt ist und gegebenenfalls durch eine oder mehrere Gruppen, die aus NR⁸R⁹ und R¹⁰ ausgewählt sind, substituiert ist;
R⁶ jeweils unabhängig voneinander aus C₁₋₆-Alkyl, C₃₋₇-Cycloalkyl, C₄₋₇-Heterocycloalkyl, Aryl und Heteroaryl ausgewählt ist, wobei jede dieser Gruppen gegebenenfalls durch einen oder mehrere Substituenten, die aus Halogen, R¹⁰ und -NR⁸R⁹ ausgewählt sind, substituiert ist;
R⁷ jeweils aus Wasserstoff, C₁₋₆-Alkyl und C₃₋₇-Cycloalkyl ausgewählt ist, wobei das C₁₋₆-Alkyl gegebenenfalls durch ein oder mehrere Halogene substituiert ist;
R⁸ und R⁹ jeweils unabhängig voneinander aus Wasserstoff und C₁₋₆-Alkyl ausgewählt sind, wobei das C₁₋₆-Alkyl gegebenenfalls durch ein oder mehrere Halogene substituiert ist; oder
R⁸ und R⁹ zusammen mit dem N, an das sie gebunden sind, einen C₄₋₆-Heterocycloalkylring bilden, der gegebenenfalls ferner ein oder mehrere Heteroatome, die aus Sauerstoff und Schwefel ausgewählt sind, enthält, wobei der C₄₋₆-Heterocycloalkylring gegebenenfalls durch eine oder mehrere R¹⁰-Gruppen substituiert ist; und
R¹⁰ jeweils aus C₃₋₇-Cycloalkyl und C₁₋₆-Alkyl, das gegebenenfalls durch ein oder mehrere Halogene substituiert ist, ausgewählt ist, wobei dann, wenn R¹⁰ für C₁₋₆-Alkyl steht und zwei oder mehr Gruppen R¹⁰ an dasselbe Kohlenstoffatom gebunden sind, die R¹⁰-Gruppen zu einer Spiroalkylgruppe verknüpft sein können; und
R¹¹ jeweils aus C₁₋₆-Alkyl, C₃₋₇-Cycloalkyl, C₁₋₆-Alkyl-C₃₋₇-cycloalkyl, C₄₋₇-Heterocycloalkyl, Aryl und Heteroaryl ausgewählt ist, wobei jede dieser Gruppen gegebenenfalls durch einen oder mehrere Substituenten, die aus A ausgewählt sind, substituiert sein kann.

2. Verbindung nach Anspruch 1, wobei NR⁷(CO)R¹¹ aus NHCO-C₁₋₆-Alkyl, NHCO-C₃₋₇-Cycloalkyl, NHCO-C₁₋₆-Alkyl-C₃₋₇cycloalkyl, NHCO-Heteroaryl und NHCO-C₄₋₇-Heterocycloalkyl ausgewählt ist.

3. Verbindung nach Anspruch 1 oder Anspruch 2, wobei NR⁷(CO)R¹¹ für NHCO-C₃₋₇-Cycloalkyl steht.

4. Verbindung nach Anspruch 1 oder Anspruch 2, wobei NR⁷(CO)R¹¹ aus NHCO-Cyclobutyl, NHCO-Thienyl, NHCO-Cyclopentyl, NHCO-Pyrazinyl, NHCOCH2-Cyclopropyl, NHCO-sec.-Butyl, NHCO-Tetrahydrofuranyl, NHCO-Thiazolyl, NHCO-Cyclopropyl, NHCO-Isopropyl, NHCO-Cyclohexyl, NHCOCH₂-Cyclopentyl and NHCO-n-Propyl ausgewählt ist.

5. Verbindung nach Anspruch 4, wobei NR⁷(CO)R^{H} aus NHCO-Cyclobutyl und NHCO-Cyclopentyl, NHCO-Cyclohexyl und NHCO-Thien-2-yl ausgewählt ist.

6. Verbindung nach einem der vorhergehenden Ansprüche, wobei R² für Aryl oder Heteroaryl steht, wobei jede dieser Gruppen gegebenenfalls durch einen oder mehrere Substituenten, die aus Aryl, Heteroaryl, C₁₋₆-Alkyl, C₃₋₆-Heterocycloalkyl und einer Gruppe A ausgewählt sind, substituiert ist, wobei die C₁₋₆-Alkylgruppe wiederum gegebenenfalls durch einen oder mehrere Substituenten, die aus Aryl, Heteroaryl, C₃₋₆-Heterocycloalkyl und einer Gruppe A ausgewählt sind, substituiert ist, die Heteroarylgruppe gegebenenfalls durch eine oder mehrere R¹⁰-Gruppen substituiert ist und die C₃₋₆-Heterocycloalkylgruppe gegebenenfalls eine oder mehrere Gruppen, die aus Sauerstoff, Schwefel, Stickstoff und CO ausgewählt sind, enthält und gegebenenfalls durch eine oder mehrere Alkyl- oder A-Gruppen substituiert ist.

7. Verbindung nach einem der vorhergehenden Ansprüche, wobei R² aus Aryl oder Heteroaryl ausgewählt ist, wobei jede dieser Gruppen gegebenenfalls durch einen oder mehrere Substituenten, die aus Halogen, gegebenenfalls substituiertem C₃₋₇-Heterocycloalkyl, gegebenenfalls substituiertem C₁₋₆-Alkyl, Heteroaryl, C₁₋₆-Alkyl-C₃₋₇-heterocycloalkyl, CN, NHCO-C₃₋₇-Heterocycloalkyl, CO-C₃₋₇-Heterocycloalkyl und NHCO-C₁₋₆-Alkyl ausgewählt sind, substituiert ist, wobei das C₃₋₇-Heterocycloalkyl gegebenenfalls durch eine oder mehrere C₁₋₆-Alkyl- oder A-Gruppen substituiert ist und das C₁₋₆-Alkyl gegebenenfalls durch eine oder mehrere Halogen- oder NR⁴R⁵-Gruppen substituiert ist.

8. Verbindung nach einem der Ansprüche 1 bis 5, wobei R² aus Phenyl, Pyridin-3-yl, Pyrazol-4-yl, Indazol-5-yl, Indazol-6-yl, Chinolinyl, Chinoxalinyl, Pyrazolopyridinyl, Imidazopyridinyl und Tetrahydroisochinolinyl ausgewählt ist, wobei jede dieser Gruppen gegebenenfalls substituiert sein kann.

9. Verbindung nach einem der Ansprüche 1 bis 5, wobei R² aus:
(i) Phenyl, gegebenenfalls substituiert durch:
Halogen, gegebenenfalls substituiertes C₃₋₇-Heterocycloalkyl, gegebenenfalls substituiertes C₁₋₆-Alkyl, Heteroaryl, C₁₋₆-Alkyl-C₃₋₇-heterocycloalkyl, CN, NHCO-C₃₋₇-Heterocycloalkyl, CO-C₃₋₇-Heterocycloalkyl oder NHCO-C₁₋₆-Alkyl,
wobei das C₃₋₇-Heterocycloalkyl gegebenenfalls durch eine oder mehrere C₁₋₆-Alkyl-, CN-, OH-, Alkoxy-, Halogenalkyl-, COR⁶-Gruppen substituiert ist und
das C₁₋₆-Alkyl gegebenenfalls durch eine oder mehrere Halogen- oder NR⁴R⁵-Gruppen substituiert ist;
(ii) Pyridinyl, das gegebenenfalls durch C₃₋₇-Heterocycloalkyl substituiert ist, wobei das C₃₋₇-Heterocycloalkyl gegebenenfalls ferner durch eine oder mehrere C₁₋₆-Alkylgruppen substituiert ist;
(iii) Pyrazolyl, das durch C₁₋₆-Alkyl, C₁₋₆-Alkyl-C₃₋₇-heterocycloalkyl oder C₃₋₇-Heterocycloalkyl substituiert ist, wobei das C₃₋₇-Heterocycloalkyl gegebenenfalls ferner durch eine oder mehrere C₁₋₆-Alkylgruppen substituiert ist;
(iv) Indazolyl, das gegebenenfalls durch C₁₋₆-Alkyl substituiert ist;
(v) Chinolinyl;
(vi) Chinoxalinyl;
(vii) Pyrazolopyridinyl;
(viii) Imidazopyridinyl und
(ix) tetrahydroisochinolinyl
ausgewählt ist.

10. Verbindung nach einem der vorhergehenden Ansprüche, wobei R² aus:
(i) Phenyl, das gegebenenfalls durch F, N-Morpholinyl, N-Methylpiperazinyl, CH₂-NMe₂, CH₂-Pyrrolidinyl, Oxazolyl, CN, CF₃, NHCO-Pyrrolidinyl, CO-Morpholinyl, NHCOMe, 2-Oxopyrrolidin-1-yl, 1,2,4-Triazol-1-yl, 4-Hydroxy-1-methylpiperidin-4-yl, 1-Methylpiperidin-4-yl, 4-Methoxy-1-methylpiperidin-4-yl, Morpholin-4-ylmethyl, 4-Cyano-methylpiperidin-4-yl, Piperidin-1-ylmethyl oder 1-(2-Fluorethyl)piperidin-4-yl substituiert ist;
(ii) Pyridinyl, das gegebenenfalls durch Morpholinyl oder 4-Methylperhydro-1,4-diazepin-1-yl substituiert ist;
(iii) Pyrazolyl, das gegebenenfalls durch Me, Et, CH₂CH₂-Morpholinyl oder 1-Isopropyl-piperidin-4-yl substituiert ist;
(iv) Indazolyl, das gegebenenfalls durch Me substituiert ist,
ausgewählt ist.

11. Verbindung nach einem der Ansprüche 1 bis 9, wobei R² aus Pyridin-3-yl, 6-(Morpholin-4-yl)pyridin-3-yl, 6-(4-Methylpiperazin-1-yl)pyridin-3-yl, 1-Me-1*H*-Pyrazol-4-yl, 1-(2-Morpholin-4-yl-ethyl)-1*H-*pyrazol-4-yl, 1-Me-1*H*-Indazol-5-yl, 1-Me-1*H-*Indazol-6-yl, 3-Fluorphenyl, 3-Trifluormethylphenyl, 3-Cyanophenyl, 3-Oxazol-5-ylphenyl, 3-Acetylaminophenyl, 4-Dimethylaminomethylphenyl, 4-(4-Methylpiperazin-1-yl)phenyl, 3-Pyrrolidin-1-yl-methylphenyl, 4-(Morpholin-4-yl)phenyl, 4-(Morpholin-4-carbonyl)phenyl, 3-(2-Oxopyrrolidin-1-yl)phenyl, 3-(Pyrrolidin-1-ylcarboxyamino)-phenyl, 4-(2-Oxopyrrolidin-1-yl)phenyl, 1*H-*Indazol-5-yl, 3-(1,2,4-Triazol-1-ylphenyl), 4-(1,2,4-Triazol-1-ylphenyl), Chinoxalin-6-yl, Chinolin-6-yl, Imidazo[1,2-a]pyridin-6-yl, 1-Methyl-1H-pyrazolo[3,4b]pyridin-5-yl, 1-Ethyl-1*H-*pyrazol-4-yl, Piperidin-1-ylmethylphenyl, (1-Isopropylpiperidin-4-yl)-1H-pyrazol-4-yl, 6-(4-Methylperhydro-1,4-diazepin-1-yl)pyridin-3-yl, Morpholin-4-ylmethylphenyl, 2-Methyl-1,2,3,4-tetrahydroisochinolin-7-yl, 4-Oxazol-5-ylphenyl, 4-(1-Methylpiperidin-4-yl)phenyl, 4-(4-Hydroxy-1-methylpiperidin-4-yl)phenyl, 4-(4-Methoxy-1-methylpiperidin-4-yl)phenyl, 4-(4-Cyano-1-methylpiperidin-4-yl)phenyl und 4-(1-(2-Fluorethyl)-piperidin-4-yl)phenyl ausgewählt ist.

12. Verbindung nach einem der vorhergehenden Ansprüche, wobei R³ aus H, Halogen und CN ausgewählt ist.

13. Verbindung nach einem der vorhergehenden Ansprüche, wobei R⁷ aus H und C₁₋₆-Alkyl, weiter bevorzugt H, ausgewählt ist.

14. Verbindung nach einem der vorhergehenden Ansprüche, die aus den folgenden Verbindungen ausgewählt ist:
Cyclobutancarbonsäure{3-[2-(1-methyl-1H-indazol-5-ylamino)pyrrolo[2,3-d]pyrimidin-7-yl]propyl}amid [1] ;
Cyclobutancarbonsäure{3-[2-(1-methyl-1H-indazol-6-ylamino)pyrrolo[2,3-d]pyrimidin-7-yl]propyl}amid [2] ;
Cyclobutancarbonsäure{3-[2-(4-morpholin-4-yl-phenylamino)pyrrolo[2,3-d]pyrimidin-7-yl]propyl}amid [3];
Cyclobutancarbonsäure(3-{2-[4-(4-methyl-piperazin-1-yl)-phenylamino]pyrrolo[2,3-d]pyrimidin-7-yl}-propyl)amid [4];
Cyclobutancarbonsäure{3-[2-(4-dimethylaminomethyl-phenylamino)pyrrolo[2,3-d]pyrimidin-7-yl]propyl}-amid [5];
Cyclobutancarbonsäure{3-[2-(3-pyrrolidin-1-yl-methylphenylamino)pyrrolo[2,3-d]pyrimidin-7-yl]-propyl}amid [6];
Cyclobutancarbonsäure{3-[2-(3-oxazol-5-ylphenyl-amino)pyrrolo[2,3-d]pyrimidin-7-yl]propyl}amid [7];
Cyclobutancarbonsäure{3-[2-(1-methyl-1H-pyrazol-4-ylamino)pyrrolo[2,3-d]pyrimidin-7-yl]propyl}amid [8];
Cyclobutancarbonsäure(3-{2-[1-(2-morpholin-4-yl-ethyl)-1H-pyrazol-4-ylamino]pyrrolo[2,3-d]-pyrimidin-7-yl}propyl)amid [9];
Thiophen-2-carbonsäure{3-[2-(3-fluorphenylamino)-pyrrolo[2,3-d]pyrimidin-7-yl]propyl}amid [10];
Thiophen-2-carbonsäure{3-[2-(3-cyanophenylamino)-pyrrolo[2,3-d]pyrimidin-7-yl]propyl}amid [11];
Thiophen-2-carbonsäure{3-[2-(pyridin-3-ylamino)-pyrrolo[2,3-d]pyrimidin-7-yl]propyl}amid [12];
Thiophen-2-carbonsäure{3-[2-(3-trifluormethyl-phenylamino)pyrrolo[2,3-d]pyrimidin-7-yl]propyl}-amid [13];
Pyrrolidin-1-carbonsäure[3-(7-{3-[(thiophen-2-carbonyl)amino]propyl}-7H-pyrrolo[2,3-d]pyrimidin-2-ylamino)phenyl]amid [14];
Cyclobutancarbonsäure(3-{2-[4-(morpholin-4-carbonyl)phenylamino]pyrrolo[2,3-d]pyrimidin-7-yl}propyl)amid [15];
Cyclopentancarbonsäure{3-[2-(6-morpholin-4-yl-pyridin-3-ylamino)pyrrolo[2,3-d]pyrimidin-7-yl]-propyl}amid [16];
Pyrazin-2-carbonsäure{3-[2-(6-morpholin-4-yl-pyridin-3-ylamino)-pyrrolo[2,3-d]pyrimidin-7-yl]-propyl}amid [17];
2-Cyclopropyl-N-{3-[2-(6-morpholin-4-ylpyridin-3-ylamino)pyrrolo[2,3-d]pyrimidin-7-yl]propyl}acetamid [18];
3-Methyl-N-{3-[2-(6-morpholin-4-ylpyridin-3-yl-amino)pyrrolo[2,3-d]pyrimidin-7-yl]propyl}butyr-amid [19];
Tetrahydrofuran-3-carbonsäure{3-[2-(6-morpholin-4-ylpyridin-3-ylamino)-pyrrolo[2,3-d]pyrimidin-7-yl]propyl}amid [20];
Thiazol-5-carbonsäure{3-[2-(6-morpholin-4-yl-pyridin-3-ylamino)pyrrolo[2,3-d]pyrimidin-7-yl]-propyl}amid [21];
Cyclopropancarbonsäure{3-[2-(6-morpholin-4-yl-pyridin-3-ylamino)pyrrolo[2,3-d]pyrimidin-7-yl]-propyl}amid [22];
N-{3-[2-(6-Morpholin-4-yl-pyridin-3-ylamino)-pyrrolo[2,3-d]pyrimidin-7-yl]propyl}isobutyramid [23];
Cyclohexancarbonsäure{3-[2-(6-morpholin-4-yl-pyridin-3-ylamino)pyrrolo[2,3-d]pyrimidin-7-yl]-propyl}amid [24];
2-Cyclopentyl-N-{3-[2-(6-morpholin-4-ylpyridin-3-ylamino)-pyrrolo[2,3-d]pyrimidin-7-yl]propyl}acetamid [25];
N-{3-[2-(6-Morpholin-4-ylpyridin-3-ylamino)-pyrrolo[2,3-d]pyrimidin-7-yl]propyl}butyramid [26];
Cyclobutancarbonsäure{3-[2-(3-fluorphenylamino)-pyrrolo[2,3-d]pyrimidin-7-yl]propyl}amid [27];
Cyclobutancarbonsäure{3-[2-(3-acetylaminophenyl-amino)pyrrolo[2,3-d]pyrimidin-7-yl]propyl}amid [28] ;
Cyclobutancarbonsäure(3-{2-[3-(2-oxopyrrolidin-1-yl)-phenylamino]-pyrrolo[2,3-d]pyrimidin-7-yl}-propyl)amid [29];
Cyclobutancarbonsäure{3-[2-(6-morpholin-4-yl-pyridin-3-ylamino)pyrrolo[2,3-d]pyrimidin-7-yl]-propyl}amid [30];
Cyclobutancarbonsäure(3-{2-[6-(4-methylpiperazin-1-yl)-pyridin-3-ylamino]pyrrolo[2,3-d]pyrimidin-7-yl}propyl)amid [31];
Cyclopentancarbonsäure{3-[2-(3-fluorphenylamino)-pyrrolo[2,3-d]pyrimidin-7-yl]propyl}amid [32];
Cyclopentancarbonsäure{3-[2-(3-acetylaminophenyl-amino)pyrrolo[2,3-d]pyrimidin-7-yl]propyl}amid [33];
Cyclobutancarbonsäure{3-[5-chlor-2-(3-fluorphenyl-amino)pyrrolo[2,3-d]pyrimidin-7-yl]propyl}amid [34] ;
Cyclobutancarbonsäure{3-[5-chlor-2-(6-morpholin-4-ylpyridin-3-ylamino)pyrrolo[2,3-d]pyrimidin-7-yl]-propyl}amid [35];
Cyclobutancarbonsäure{3-[5-brom-2-(3-fluorphenyl-amino)-pyrrolo[2,3-d]pyrimidin-7-yl]propyl}amid [36];
Cyclobutancarbonsäure{3-[5-cyano-2-(3-fluorphenyl-amino)pyrrolo[2,3-d]pyrimidin-7-yl]propyl}amid [37];
Thiophen-2-carbonsäure{3-[5-chlor-2-(3-fluor-phenylamino)pyrrolo[2,3-d]pyrimidin-7-yl]propyl}-amid [38];
Cyclobutancarbonsäure(3-{2-[4-(2-oxopyrrolidin-1-yl)phenylamino]pyrrolo[2,3-d]pyrimidin-7-yl}propyl)amid [39];
Cyclobutancarbonsäure{3-[2-(1H-indazol-5-ylamino)-pyrrolo[2,3-d]pyrimidin-7-yl]propyl}amid [40];
Cyclobutancarbonsäure{3-[2-(3-1,2,4-triazol-1-yl-phenylamino)pyrrolo[2,3-d]pyrimidin-7-yl]propyl}amid [41];
Cyclobutancarbonsäure{3-[2-(4-1,2,4-triazol-1-yl-phenylamino)pyrrolo[2,3-d]pyrimidin-7-yl]propyl}-amid [42];
Cyclobutancarbonsäure{3-[2-(pyridin-3-ylamino)-pyrrolo[2,3-d]pyrimidin-7-yl]propyl}amid [43];
Cyclobutancarbonsäure{3-[2-(1-methyl-1H-pyrazolo-[3,4-b]pyridin-5-ylamino)pyrrolo[2,3-d]pyrimidin-7-yl]propyl}amid [44];
Cyclobutancarbonsäure{3-[2-(imidazo[1,2-a]pyridin-6-ylamino)pyrrolo[2,3-d]pyrimidin-7-yl]propyl}amid [45];
Cyclobutancarbonsäure{3-[2-(chinoxalin-6-ylamino)-pyrrolo[2,3-d]pyrimidin-7-yl]propyl}amid [46];
Cyclobutancarbonsäure{3-[2-(1-ethyl-1H-pyrazol-4-ylamino)pyrrolo[2,3-d]pyrimidin-7-yl]propyl}amid [47];
Cyclobutancarbonsäure{3-[2-(3-morpholin-4-yl-phenylamino)pyrrolo[2,3-d]pyrimidin-7-yl]propyl}-amid [48];
Cyclobutancarbonsäure{3-[2-(3-piperidin-1-yl-methylphenylamino)pyrrolo[2,3-d]pyrimidin-7-yl]-propyl}amid [49];
Cyclobutancarbonsäure{3-[2-(chinolin-6-ylamino)-pyrrolo[2,3-d]pyrimidin-7-yl]propyl}amid [50];
Cyclobutancarbonsäure(3-{2-[1-(1-isopropyl-piperidin-4-yl)-1H-pyrazol-4-ylamino]pyrrolo[2,3-d]pyrimidin-7-yl}propyl)amid [51];
Cyclobutancarbonsäure{3-[2-(3-morpholin-4-yl-methylphenylamino)pyrrolo[2,3-d]pyrimidin-7-yl]-propyl}amid [52];
Cyclobutancarbonsäure{3-[2-(2-methyl-1,2,3,4-tetrahydroisochinolin-7-ylamino)pyrrolo[2,3-d]-pyrimidin-7-yl]propyl}amid [53];
Cyclobutancarbonsäure{3-[2-(4-oxazol-5-ylphenyl-amino)pyrrolo[2,3-d]pyrimidin-7-yl]propyl}amid [54];
Cyclobutancarbonsäure(3-{2-[6-(4-methylperhydro-1,4-diazepin-1-yl)pyridin-3-ylamino]pyrrolo[2,3-d]pyrimidin-7-yl}propyl)amid [55];
Cyclobutancarbonsäure(3-{2-[4-(1-methylpiperidin-4-yl)phenylamino]pyrrolo[2,3-d]pyrimidin-7-yl}-propyl)amid [56];
Cyclobutancarbonsäure(3-{2-[4-(4-hydroxy-1-methylpiperidin-4-yl)phenylamino]pyrrolo[2,3-d]-pyrimidin-7-yl}propyl)amid [57];
Cyclobutancarbonsäure(3-[2-[4-(4-methoxy-1-methylpiperidin-4-yl)phenylamino]pyrrolo[2,3-d]-pyrimidin-7-yl}propyl)amid [58];
Cyclobutancarbonsäure(3-{2-[4-(4-cyano-l-methylpiperidin-4-yl)phenylamino]pyrrolo[2,3-d]-pyrimidin-7-yl}propyl)amid [59] und
Cyclobutancarbonsäure[3-(2-[4-[1-(2-fluorethyl)-piperidin-4-yl]phenylamino}pyrrolo[2,3-d]-pyrimidin-7-yl)propyl]amid [60].

15. Pharmazeutische Zusammensetzung, umfassend eine Verbindung nach einem der Ansprüche 1 bis 14 und einen pharmazeutisch unbedenklichen Träger, ein pharmazetuisch unbedenkliches Verdünnungsmittel oder einen pharmazeutisch unbedenklichen Exzipienten.

16. Verbindung nach einem der Ansprüche 1 bis 14 zur Verwendung in der Medizin.

17. Verbindung nach einem der Ansprüche 1 bis 14 zur Verwendung bei der Behandlung oder Prävention einer Störung, die aus Krebs, spetischem Schock, primärem Weitwinkelglaukom (POAG), Hyperplasie, rheumatoider Arthritis, Psoriasis, Artherosklerose, Retinopathie, Osteoarthritis, Endometriose, chronischer Entzündung und Alzheimer-Krankheit ausgewählt ist.

18. Verwendung einer Verbindung nach einem der Ansprüche 1 bis 14 bei der Herstellung eines Medikaments zur Behandlung oder Prävention einer Störung, die aus Krebs, septischem Schock, primärem Weitwinkelglaukom (POAG), Hyperplasie, rheumatoider Arthritis, Psoriasis, Artherosklerose, Retinopathie, Osteoarthritis, Endometriose, chronischer Entzündung und Alzheimer-Krankheit ausgewählt ist.

19. Verwendung einer Verbindung nach einem der Ansprüche 1 bis 14 bei der Herstellung eines Medikaments zur Behandlung oder Prävention einer Störung, die durch abnormale Kinaseaktivität verursacht wird, mit abnormaler Kinaseaktivität assoziiert ist oder mit abnormaler Kinaseaktvität einhergeht, wobei es sich bei der Kinase um TBK1 handelt.

20. Verwendung einer Verbindung nach einem der Ansprüche 1 bis 14 bei einem Assay zur Identifizierung weiterer Kandidatenverbindungen, die zur Inhibierung von TBK1 befähigt sind.

21. Verfahren zur Herstellung einer Verbindung der Formel VII, worin R¹¹ und R¹² wie in Anspruch 1 definiert sind, bei dem man: 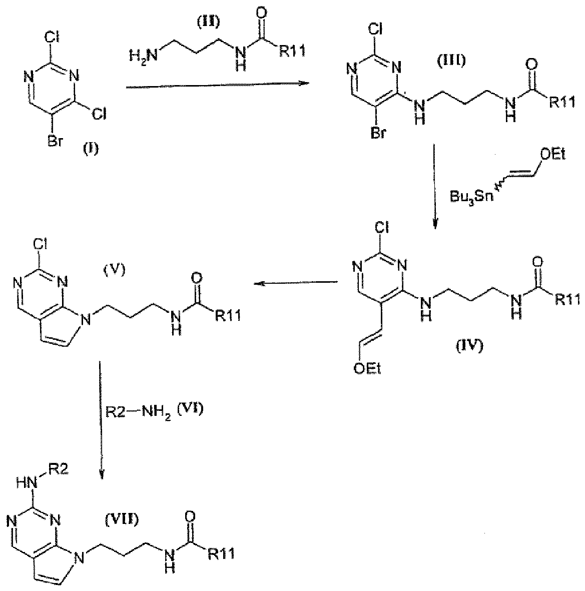
(i) 5-Brom-2,4-dichlorpyrimidin (I) mit einem Amin der Formel II zu einer Verbindung der Formel IIII umsetzt;
(ii) die Verbindung der Formel III mit Ethoxyvinylzinn zu einer Verbindung der Formel IV umsetzt;
(iii) die Verbindung der Formel IV zu einer Verbindung der Formel V cyclisiert;
(iv) die Verbindung der Formel V mit einem Amin der Formel VI zu einer Verbindung der Formel VII umsetzt.

22. Kombination, umfassend eine Verbindung nach einem der Ansprüche 1 bis 14 und ein weiteres Therapeutikum.

23. Pharmazeutische Zusammensetzung nach Anspruch 15, die ferner ein zweites Therapeutikum umfasst.

## Revendications

1. Composé de formule (I), ou un sel ou ester pharmaceutiquement acceptable de celui-ci, 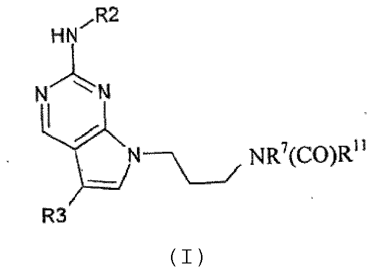 dans lequel :
R² est aryle, hétéroaryle, aryl-(hétérocycloalkyle en C₃₋₆) condensé ou hétéroaryl-(hétérocycloalkyle en C₃₋₆) condensé, dont chacun est facultativement substitué par un ou plusieurs substituants choisis parmi aryle, hétéroaryle, alkyle en C₁₋₆, hétérocycloalkyle en C₃₋₆ et
un groupe A, ledit groupe alkyle en C₁₋₆ étant lui-même facultativement substitué par un ou plusieurs substituants choisis parmi aryle, hétéroaryle,
hétérocycloalkyle en C₃₋₆ et un groupe A, ledit groupe hétéroaryle étant facultativement substitué par un ou
plusieurs groupes R¹⁰; et chaque groupe hétérocycloalkyle en C₃₋₆ étant facultativement substitué par un ou plusieurs groupes choisis parmi alkyle en C₁₋₆, halogénoalkyle en C₁₋₆, et A, et qui contient facultativement un ou plusieurs groupes choisis parmi l'oxygène, le soufre, l'azote et CO ;
R³ est H, halogène, cyano ou alkyle en C₁₋₆ ;
A est choisi parmi halogène, hydroxyle, cyano, trifluorométhyle, -NO₂, -NH₂, -NR⁴R⁵, -OR⁶, -NR⁷(CO)R⁶, -NR⁷(CO)NR⁴R⁵, -NR⁷COOR⁷, -NR⁷(SO₂)R⁶, -CO₂H, -NR⁷(SO₂)NR⁴R⁵, -COOR⁷, -CONR⁴R⁵, COR⁶ et -SO₂CH₃ ;
chaque R⁴ et R⁵ est indépendamment choisi parmi hydrogène, cycloalkyle en C₃₋₇, aryle, hétéroaryle, alkyle en C₁₋₆ et un cycle hétérocycloalkyle en C₃₋₆ contenant facultativement en outre un ou plusieurs groupes choisis parmi l'oxygène, le soufre, l'azote et CO, et facultativement substitué par un ou plusieurs groupes R¹⁰, ledit alkyle en C₁₋₆ étant facultativement substitué par un ou plusieurs substituants choisis parmi halogène, cyano, hydroxyle, aryle, hétéroaryle, -NR⁸R⁹, -NR⁷(CO)R⁶, NR⁷COOR⁶, -NR⁷(SO₂)R⁶, -COOR⁶, -CONR⁸R⁹, OR¹⁰, -SO₂R⁶ et un cycle hétérocycloalkyle en C₃₋₆ contenant facultativement en outre un ou plusieurs groupes choisis parmi l'oxygène, le soufre, l'azote et CO et facultativement substitué par un ou plusieurs groupes R¹⁰ ; ou
R⁴ et R⁵ conjointement avec le N auquel ils sont liés forment un cycle hétérocycloalkyle en C₃₋₆ contenant facultativement en outre un ou plusieurs groupes choisis parmi l'oxygène, le soufre, l'azote et CO, ledit cycle hétérocycloalkyle en C₃₋₆ pouvant être saturé ou insaturé et étant facultativement substitué par un ou plusieurs groupes choisis parmi NR⁸R⁹ et R¹⁰ ;
chaque R⁶ est indépendamment choisi parmi alkyle en C₁₋₆, cycloalkyle en C₃₋₇, hétérocycloalkyle en C₄₋₇, aryle et hétéroaryle, dont chacun peut être facultativement substitué par un ou plusieurs substituants choisis parmi halogène, R¹⁰ et -NR⁸R⁹ ;
chaque R⁷ est choisi parmi hydrogène, alkyle en C₁₋₆ et cycloalkyle en C₃₋₇, ledit alkyle en C₁₋₆ est facultativement substitué par un ou plusieurs halogènes ;
chacun de R⁸ et R⁹ est indépendamment choisi parmi hydrogène et alkyle en C₁₋₆, ledit groupe alkyle en C₁₋₆ étant facultativement substitué par un ou plusieurs halogènes ; ou
R⁸ et R⁹ conjointement avec le N auquel ils sont liés forment un cycle hétérocycloalkyle en C₄₋₆ contenant facultativement en outre un ou plusieurs hétéroatomes choisis parmi l'oxygène et le soufre, ledit cycle hétérocycloalkyle en C₄₋₆ étant facultativement substitué par un ou plusieurs groupes R¹⁰ ; et
chaque R¹⁰ est choisi parmi cycloalkyle en C₃₋₇ et alkyle en C₁₋₆ facultativement substitué par un ou plusieurs halogènes, où, lorsque R¹⁰ est alkyle en C₁₋₆ et deux groupes R¹⁰ ou plus sont liés au même atome de carbone, les groupes R¹⁰ peuvent être liés pour former un groupe spiroalkyle ; et
chaque R¹¹ est indépendamment choisi parmi alkyle en C₁₋₆, cycloalkyle en C₃₋₇, (alkyle en C₁₋₆)-(cycloalkyle en C₃₋₇), hétérocycloalkyle en C₄₋₇, aryle et hétéroaryle, dont chacun peut être facultativement substitué par un ou plusieurs substituants choisis parmi A.

2. Composé selon la revendication 1 dans lequel NR⁷(CO)R¹¹ est choisi parmi NHCO-(alkyle en C₁₋₆), NHCO-(cycloalkyle en C₃₋₇), NHCO-(alkyle en C₁₋₆)-(cycloalkyle en C₃₋₇), NHCO-hétéroaryle, NHCO-(hétérocycloalkyle en C₄₋₇).

3. Composé selon la revendication 1 ou la revendication 2 dans lequel NR⁷(CO)R¹¹ est NHCO-(cycloalkyle en C₃₋₇).

4. Composé selon la revendication 1 ou la revendication 2 dans lequel NR⁷(CO)R¹¹ est choisi parmi NHCO-cyclobutyle, NHCO-thiényle, NHCO-cyclopentyle, NHCO-pyrazinyle, NHCOCH₂-cyclopropyle, NHCO-sec-butyle, NHCO-tétrahydrofuranyle, NHCO-thiazolyle, NHCO-cyclopropyle, NHCO-isopropyle, NHCO-cyclohexyle, NHCOCH₂-cyclopentyle et NHCO-n-propyle.

5. Composé selon la revendication 4 dans lequel NR⁷(CO)R¹¹ est choisi parmi NHCO-cyclobutyle et NHCO-cyclopentyle, NHCO-cyclohexyle et NHCO-thién-2-yle.

6. Composé selon l'une quelconque des revendications précédentes dans lequel R² est aryle ou hétéroaryle, dont chacun est facultativement substitué par un ou plusieurs substituants choisis parmi aryle, hétéroaryle, alkyle en C₁₋₆, hétérocycloalkyle en C₃₋₆ et un groupe A, ledit groupe alkyle en C₁₋₆ étant lui-même facultativement substitué par un ou plusieurs substituants choisis parmi un groupe aryle, hétéroaryle, hétérocycloalkyle en C₃₋₆ et un groupe A, ledit groupe hétéroaryle étant facultativement substitué par un ou plusieurs groupes R¹⁰ ; et ledit groupe hétérocycloalkyle en C₃₋₆ contenant facultativement un ou plusieurs groupes choisis parmi l'oxygène, le soufre, l'azote et CO, et est facultativement substitué par un ou plusieurs groupes alkyle ou A.

7. Composé selon l'une quelconque des revendications précédentes dans lequel R² est choisi parmi aryle ou hétéroaryle, dont chacun est facultativement substitué par un ou plusieurs substituants choisis parmi halogéno, hétérocycloalkyle en C₃₋₇ facultativement substitué, alkyle en C₁₋₆ facultativement substitué, hétéroaryle, (alkyle en C₁₋₆)-(hétérocycloalkyle en C₃₋₇), CN, NHCO-(hétérocycloalkyle en C₃₋₇), CO-(hétérocycloalkyle en C₃₋₇) et NHCO-(alkyle en C₁₋₆), ledit hétérocycloalkyle en C₃₋₇ étant facultativement substitué par un ou plusieurs groupes alkyle en C₁₋₆ ou A, et ledit alkyle en C₁₋₆ étant facultativement substitué par un ou plusieurs groupes halogéno ou NR⁴R⁵.

8. Composé selon l'une quelconque des revendications 1 à 5 dans lequel R² est choisi parmi phényle, pyridin-3-yle, pyrazol-4-yle, indazol-5-yle, indazol-6-yle, quinoléinyle, quinoxalinyle, pyrazolopyridinyle, imidazopyridinyle et tétrahydro-isoquinoléinyle, dont chacun peut être facultativement substitué.

9. Composé selon l'une quelconque des revendications 1 à 5 dans lequel R² est choisi parmi :
(i) phényle facultativement substitué par :
halogéno, hétérocycloalkyle en C₃₋₇ facultativement substitué, alkyle en C₁₋₆ facultativement substitué, hétéroaryle, (alkyle en C₁₋₆)-(hétérocycloalkyle en C₃₋₇), CN, NHCO-(hétérocycloalkyle en C₃₋₇), CO-(hétérocycloalkyle en C₃₋₇) ou NHCO-(alkyle en C₁-₆),
ledit hétérocycloalkyle en C₃₋₇ étant facultativement substitué par un ou plusieurs groupes alkyle en C₁₋₆, CN, OH, alcoxy, halogénoalkyle, COR⁶, et ledit alkyle en C₁₋₆ étant facultativement substitué par un ou plusieurs groupes halogéno ou NR⁴R⁵ ;
(ii) pyridinyle facultativement substitué par hétérocycloalkyle en C₃₋₇, ledit hétérocycloalkyle en C₃₋₇ étant facultativement substitué en outre par un ou plusieurs groupes alkyle en C₁₋₆ ;
(iii) pyrazolyle substitué par alkyle en C₁₋₆, (alkyle en C₁₋₆)-(hétérocycloalkyle en C₃₋₇) ou hétérocycloalkyle en C₃₋₇, ledit hétérocycloalkyle en C₃₋₇ étant facultativement substitué en outre par un ou plusieurs groupes alkyle en C₁₋₆ ;
(iv) indazolyle facultativement substitué par alkyle en C₁₋₆ ;
(v) quinoléinyle ;
(vi) quinoxalinyle ;
(vii) pyrazolopyridinyle ;
(viii) imidazopyridinyle ; et
(ix) tétrahydro-isoquinoléinyle.

10. Composé selon l'une quelconque des revendications précédentes dans lequel R² est choisi parmi :
(i) phényle facultativement substitué par F, N-morpholinyle, N-méthylpipérazinyle, CH₂-NMe₂, CH₂-pyrrolidinyle, oxazolyle, CN, CF₃, NHCO-pyrrolidinyle, CO-morpholinyle, NHCOMe, 2-oxopyrrolidin-1-yle, 1,2,4-triazol-1-yle, 4-hydroxy-1-méthylpipéridin-4-yle, 1-méthyl-pipéridin-4-yle, 4-méthoxy-1-méthylpipéridin-4-yle, morpholin-4-yl-méthyle, 4-cyano-1-méthylpipéridin-4-yle, pipéridin-1-yl-méthyle ou 1-(2-fluoroéthyl)-pipéridin-4-yle ;
(ii) pyridinyle facultativement substitué par morpholinyle ou 4-méthyl-perhydro-1,4-diazépin-1-yle ;
(iii) pyrazolyle facultativement substitué par Me, Et, CH₂CH₂-morpholinyle, ou 1-isopropyl-pipéridin-4-yle ;
(iv) indazolyle facultativement substitué par Me.

11. Composé selon l'une quelconque des revendications 1 à 9 dans lequel R² est choisi parmi :
pyridin-3-yle, 6-(morpholin-4-yl)-pyridin-3-yle, 6-(4-méthylpipérazin-1-yl)-pyridin-3-yle, 1-Me-1*H*-pyrazol-4-yle, 1-(2-morpholin-4-yl-éthyl)-1*H*-pyrazol-4-yle, 1-Me-1*H*-indazol-5-yle, 1-Me-1*H*-indazol-6-yle, 3-fluorophényle, 3-trifluorométhylphényle, 3-cyanophényle, 3-oxazol-5-yl-phényle, 3-acétylaminophényle, 4-diméthylaminométhylphényle, 4-(4-méthyl-pipérazin-1-yl)-phényle, 3-pyrrolidin-1-yl-méthylphényle, 4-(morpholin-4-yl)-phényle, 4-(morpholine-4-carbonyl)-phényle, 3-(2-oxo-pyrrolidin-1-yl)-phényle, 3-(pyrrolidin-1-yl-carboxyamino)-phényle, 4-(2-oxo-pyrrolidin-1-yl)-phényle, 1*H*-indazol-5-yle, 3-(1,2,4-triazol-1-yl-phényle), 4-(1,2,4-triazol-1-yl-phényle), quinoxalin-6-yle, quinoléin-6-yle, imidazo[1,2-a]pyridine-6-yle, 1-méthyl-1*H-*pyrazolo[3,4b]pyridine-5-yle, 1-éthyl-1*H*-pyrazol-4-yle, pipéridin-1-yl-méthylphényle, (1-isopropyl-pipéridin-4-yl)-1H-pyrazol-4-yle, 6-(4-méthyl-perhydro-1,4-diazépin-1-yl)-pyridin-3-yle, morpholin-4-yl-méthyl-phényle, 2-méthyl-1,2,3,4-tétrahydroisoquinoléin-7-yle, 4-oxazol-5-yl-phényle, 4-(1-méthylpipéridin-4-yl)-phényle, 4-(4-hydroxy-1-méthyl-pipéridin-4-yl)-phényle, 4-(4-méthoxy-1-méthyl-pipéridin-4-yl)-phényle, 4-(4-cyano-1-méthyl-pipéridin-4-yl)-phényle et 4-(1-(2-fluoroéthyl)-pipéridin-4-yl)-phényle.

12. Composé selon l'une quelconque des revendications précédentes dans lequel R³ est choisi parmi H, halogéno et CN.

13. Composé selon l'une quelconque des revendications précédentes dans lequel R⁷ est choisi parmi H et alkyle en C₁₋₆, plus préférablement H.

14. Composé selon l'une quelconque des revendications précédentes qui est choisi parmi les suivants :
{3-[2-(1-méthyl-1H-indazol-5-ylamino)-pyrrolo[2,3-d]pyrimidin-7-yl]-propyl}-amide d'acide cyclobutanecarboxylique [1] ;
{3-[2-(1-méthyl-1H-indazol-6-ylamino)-pyrrolo[2,3-d]pyrimidin-7-yl]-propyl}-amide d'acide cyclobutanecarboxylique [2] ;
{3-[2-(4-morpholin-4-yl-phénylamino)-pyrrolo[2,3-d]pyrimidin-7-yl]-propyl}-amide d'acide cyclobutanecarboxylique [3] ;
(3-{2-[4-(4-méthyl-pipérazin-1-yl)-phénylamino]-pyrrolo[2,3-d]pyrimidin-7-yl}-propyl)-amide d'acide cyclobutanecarboxylique [4] ;
{3-[2-(4-diméthylaminométhyl-phenylamino)-pyrrolo[2,3-d]pyrimidin-7-yl]-propyl}-amide d'acide cyclobutanecarboxylique [5] ;
{3-[2-(3-pyrrolidin-1-ylméthyl-phénylamino)-pyrrolo[2,3-d]pyrimidin-7-yl]-propyl}-amide d'acide cyclobutanecarboxylique [6] ;
{3-[2-(3-oxazol-5-yl-phénylamino)-pyrrolo[2,3-d]pyrimidin-7-yl]-propyl}-amide d'acide cyclobutanecarboxylique [7] ;
{3-[2-(1-méthyl-1H-pyrazol-4-ylamino)-pyrrolo[2,3-d]pyrimidin-7-yl]-propyl}-amide d'acide cyclobutanecarboxylique [8] ;
(3-{2-[1-(2-morpholin-4-yl-éthyl)-1H-pyrazol-4-ylamino]-pyrrolo[2,3-d]pyrimidin-7-yl}-propyl)-amide d'acide cyclobutanecarboxylique [9] ;
{3-[2-(3-fluoro-phénylamino)-pyrrolo[2,3-d]pyrimidin-7-yl]-propyl}-amide d'acide thiophène-2-carboxylique [10] ;
{3-[2-(3-cyano-phénylamino)-pyrrolo[2,3-d]pyrimidin-7-yl]-propyl}-amide d'acide thiophène-2-carboxylique [11] ;
{3-[2-(pyridin-3-ylamino)-pyrrolo[2,3-d]pyrimidin-7-yl]-propyl}-amide d'acide thiophène-2-carboxylique [12] ;
{3-[2-(3-trifluorométhyl-phénylamino)-pyrrolo[2,3-d]pyrimidin-7-yl]-propyl}-amide d'acide thiophène-2-carboxylique [13] ;
[3-(7-{3-[(thiophène-2-carbonyl)-amino]-propyl}-7H-pyrrolo[2,3-d]pyrimidin-2-ylamino)-phényl]-amide d'acide pyrrolidine-1-carboxylique [14] ;
(3-{2-[4-(morpholine-4-carbonyl)-phénylamino]-pyrrolo[2,3-d]pyrimidin-7-yl}-propyl)-amide d'acide cyclobutanecarboxylique [15] ;
(3-[2-(6-morpholin-4-yl-pyridin-3-ylamino)-pyrrolo[2,3-d]pyrimidin-7-yl]-propyl}-amide d'acide cyclopentanecarboxylique [16] ;
{3-[2-(6-morpholin-4-yl-pyridin-3-ylamino)-pyrrolo[2,3-d]pyrimidin-7-yl]-propyl}-amide d'acide pyrazine-2-carboxylique [17] ;
2-cyclopropyl-N-{3-[2-(6-morpholin-4-yl-pyridin-3-ylamino)-pyrrolo[2,3-d]pyrimidin-7-yl]-propyl}-acétamide [18] ;
3-méthyl-N-{3-[2-(6-morpholin-4-yl-pyridin-3-ylamino)-pyrrolo[2,3-d]pyrimidin-7-yl]-propyl}-butyramide [19] ;
{3-[2-(6-morpholin-4-yl-pyridin-3-ylamino)-pyrrolo[2,3-d]pyrimidin-7-yl]-propyl}-amide d'acide tétrahydro-furane-3-carboxylique [20] ;
{3-[2-(6-morpholin-4-yl-pyridin-3-ylamino)-pyrrolo[2,3-d]pyrimidin-7-yl]-propyl}-amide d'acide thiazole-5-carboxylique [21] ;
{3-[2-(6-morpholin-4-yl-pyridin-3-ylamino)-pyrrolo[2,3-d]pyrimidin-7-yl]-propyl}-amide d'acide cyclopropanecarboxylique [22] ;
N-{3-[2-(6-morpholin-4-yl-pyridin-3-ylamino)-pyrrolo[2,3-d]pyrimidin-7-yl]-propyl}-isobutyramide [23] ;
{3-[2-(6-morpholin-4-yl-pyridin-3-ylamino)-pyrrolo[2,3-d]pyrimidin-7-yl]-propyl}-amide d'acide cyclohexanecarboxylique [24] ;
2-cyclopentyl-N-{3-[2-(6-morpholin-4-yl-pyridin-3-ylamino)-pyrrolo[2,3-d]pyrimidin-7-yl]-propyl}-acétamide [25] ;
N-{3-[2-(6-morpholin-4-yl-pyridin-3-ylamino)-pyrrolo[2,3-d]pyrimidin-7-yl]-propyl}-butyramide [26] ;
{3-[2-(3-fluoro-phénylamino)-pyrrolo[2,3-d]pyrimidin-7-yl]-propyl}-amide d'acide cyclobutanecarboxylique [27] ;
{3-[2-(3-acétylamino-phénylamino)-pyrrolo[2,3-d]pyrimidin-7-yl]-propyl}-amide d'acide cyclobutanecarboxylique [28] ;
(3-{2-[3-(2-oxo-pyrrolidin-1-yl)-phénylamino]-pyrrolo[2,3-d]pyrimidin-7-yl}-propyl)-amide d'acide cyclobutanecarboxylique [29] ;
{3-[2-(6-morpholin-4-yl-pyridin-3-ylamino)-pyrrolo[2,3-d]pyrimidin-7-yl]-propyl}-amide d'acide cyclobutanecarboxylique [30] ;
(3-{2-[6-(4-méthyl-pipérazin-1-yl)-pyridin-3-ylamino]-pyrrolo[2,3-d]pyrimidin-7-yl}-propyl)-amide d'acide cyclobutanecarboxylique [31] ;
{3-[2-(3-fluoro-phénylamino)-pyrrolo[2,3-d]pyrimidin-7-yl]-propyl}-amide d'acide cyclopentanecarboxylique [32] ;
{3-[2-(3-acétylamino-phénylamino)-pyrrolo[2,3-d]pyrimidin-7-yl]-propyl}-amide d'acide cyclopentanecarboxylique [33] ;
{3-[5-chloro-2-(3-fluoro-phénylamino)-pyrrolo[2,3-d]pyrimidin-7-yl]-propyl}-amide d'acide cyclobutanecarboxylique [34] ;
{3-[5-chloro-2-(6-morpholin-4-yl-pyridin-3-ylamino)-pyrrolo[2,3-d]pyrimidin-7-yl]-propyl}-amide d'acide cyclobutanecarboxylique [35] ;
{3-[5-bromo-2-(3-fluoro-phénylamino)-pyrrolo[2,3-d]pyrimidin-7-yl]-propyl}-amide d'acide cyclobutanecarboxylique [36] ;
{3-[5-cyano-2-(3-fluoro-phénylamino)-pyrrolo[2,3-d]pyrimidin-7-yl]-propyl}-amide d'acide cyclobutanecarboxylique [37] ;
{3-[5-chloro-2-(3-fluoro-phenylamino)-pyrrolo[2,3-d]pyrimidin-7-yl]-propyl}-amide d'acide thiophène-2-carboxylique [38] ;
(3-{2-[4-(2-oxo-pyrrolidin-1-yl)-phénylamino]-pyrrolo[2,3-d]pyrimidin-7-yl}-propyl)-amide d'acide cyclobutanecarboxylique [39] ;
{3-[2-(1H-indazol-5-ylamino)-pyrrolo[2,3-d]pyrimidin-7-yl]-propyl}-amide d'acide cyclobutanecarboxylique [40] ;
{3-[2-(3-1,2,4-triazol-1-yl-phénylamino)-pyrrolo[2,3-d]pyrimidin-7-yl]-propyl}-amide d'acide cyclobutanecarboxylique [41] ;
{3-[2-(4-1,2,4-triazol-1-yl-phénylamino)-pyrrolo[2,3-d]pyrimidin-7-yl]-propyl}-amide d'acide cyclobutanecarboxylique [42] ;
{3-[2-(pyridin-3-ylamino)-pyrrolo[2,3-d]pyrimidin-7-yl]-propyl}-amide d'acide cyclobutanecarboxylique [43] ;
{3-[2-(1-méthyl-1H-pyrazolo[3,4-b]pyridin-5-ylamino)-pyrrolo[2,3-d]pyrimidin-7-yl]-propyl}-amide d'acide cyclobutanecarboxylique [44] ;
{3-[2-(imidazo[1,2-a]pyridin-6-ylamino)-pyrrolo[2,3-d]pyrimidin-7-yl]-propyl}-amide d'acide cyclobutanecarboxylique [45] ;
{3-[2-(quinoxalin-6-ylamino)-pyrrolo[2,3-d]pyrimidin-7-yl]-propyl}-amide d'acide cyclobutanecarboxylique [46] ;
{3-[2-(1-éthyl-1H-pyrazol-4-ylamino)-pyrrolo[2,3-d]pyrimidin-7-yl]-propyl}-amide d'acide cyclobutanecarboxylique [47] ;
{3-[2-(3-morpholin-4-yl-phénylamino)-pyrrolo[2,3-d]pyrimidin-7-yl]-propyl}-amide d'acide cyclobutanecarboxylique [48] ;
{3-[2-(3-pipéridin-1-ylméthyl-phénylamino)-pyrrolo[2,3-d]pyrimidin-7-yl]-propyl}-amide d'acide cyclobutanecarboxylique [49] ;
{3-[2-(quinoléin-6-ylamino)-pyrrolo[2,3-d]pyrimidin-7-yl]-propyl}-amide d'acide cyclobutanecarboxylique [50] ;
(3-{2-[1-(1-isopropyl-pipéridin-4-yl)-1H-pyrazol-4-ylamino]-pyrrolo[2,3-d]pyrimidin-7-yl}-propyl)-amide d'acide cyclobutanecarboxylique [51] ;
{3-[2-(3-morpholin-4-ylméthyl-phénylamino)-pyrrolo[2,3-d]pyrimidin-7-yl]-propyl}-amide d'acide cyclobutanecarboxylique [52] ;
{3-[2-(2-méthyl-1,2,3,4-tétrahydro-isoquinoléin-7-ylamino)-pyrrolo[2,3-d]pyrimidin-7-yl]-propyl}-amide d'acide cyclobutanecarboxylique [53] ;
{3-[2-(4-oxazol-5-yl-phénylamino)-pyrrolo[2,3-d]pyrimidin-7-yl]-propyl}-amide d'acide cyclobutanecarboxylique [54] ;
(3-{2-[6-(4-méthyl-perhydro-1,4-diazépin-1-yl)-pyridin-3-ylamino]-pyrrolo[2,3-d]pyrimidin-7-yl}-propyl)-amide d'acide cyclobutanecarboxylique [55] ;
(3-{2-[4-(1-méthyl-pipéridin-4-yl)-phénylamino]-pyrrolo[2,3-d]pyrimidin-7-yl}-propyl)-amide d'acide cyclobutanecarboxylique [56] ;
(3-{2-[4-(4-hydroxy-1-méthyl-pipéridin-4-yl)-phénylamino]-pyrrolo[2,3-d]pyrimidin-7-yl}-propyl)-amide d'acide cyclobutanecarboxylique [57] ;
(3-{2-[4-(4-méthoxy-1-méthyl-pipéridin-4-yl)-phénylamino]-pyrrolo[2,3-d]pyrimidin-7-yl}-propyl)-amide d'acide cyclobutanecarboxylique [58] ;
(3-{2-[4-(4-cyano-1-méthyl-pipéridin-4-yl)-phénylamino]-pyrrolo[2,3-d]pyrimidin-7-yl}-propyl)-amide d'acide cyclobutanecarboxylique [59] ; et
[3-(2-{4-[1-(2-fluoro-éthyl)-pipéridin-4-yl]-phénylamino}-pyrrolo[2,3-d]pyrimidin-7-yl)-propyl]-amide d'acide cyclobutanecarboxylique [60].

15. Composition pharmaceutique comprenant un composé selon l'une quelconque des revendications 1 à 14 et un véhicule, diluant ou excipient pharmaceutiquement acceptable.

16. Composé selon l'une quelconque des revendications 1 à 14 pour utilisation en médecine.

17. Composé selon l'une quelconque des revendications 1 à 14 pour utilisation dans le traitement ou la prévention d'un trouble choisi parmi le cancer, un choc septique, le glaucome primaire à angle ouvert (GPAO), une hyperplasie, la polyarthrite rhumatoïde, le psoriasis, l'athérosclérose, la rétinopathie, l'arthrose, l'endométriose, une inflammation chronique et la maladie d'Alzheimer.

18. Utilisation d'un composé selon l'une quelconque des revendications 1 à 14 dans la préparation d'un médicament pour traiter ou prévenir un trouble choisi parmi le cancer, un choc septique, le glaucome primaire à angle ouvert (GPAO), une hyperplasie, la polyarthrite rhumatoïde, le psoriasis, l'athérosclérose, la rétinopathie, l'arthrose, l'endométriose, une inflammation chronique et la maladie d'Alzheimer.

19. Utilisation d'un composé selon l'une quelconque des revendications 1 à 14 dans la préparation d'un médicament pour la prévention ou le traitement d'un trouble causé par, associé à ou accompagné d'une activité kinase anormale, la kinase étant TBK1.

20. Utilisation d'un composé selon l'une quelconque des revendications 1 à 14 dans un essai pour identifier de nouveaux composés candidats capables d'inhiber TBK1.

21. Procédé pour préparer un composé de formule VII, R¹¹ et R² étant tels que définis dans la revendication 1, ledit procédé comprenant les étapes de : 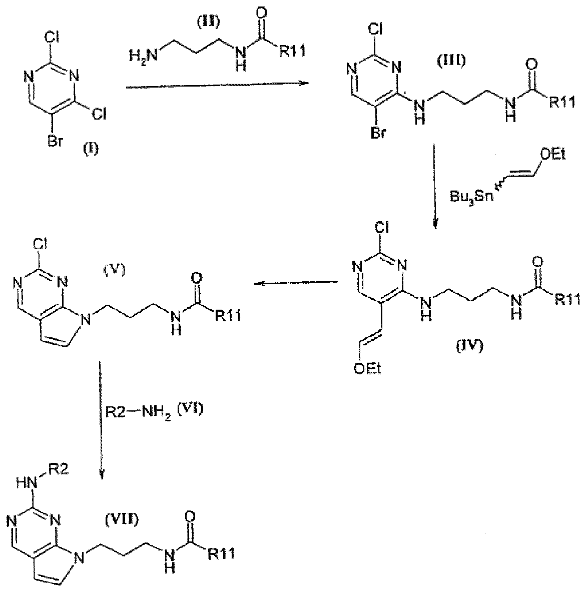
(i) réaction de la 5-bromo-2,4-dichloropyrimidine (I) avec une amine de formule II pour obtenir un composé de formule III ;
(ii) réaction dudit composé de formule III avec de l'éthoxyvinyl-étain pour obtenir un composé de formule IV ;
(iii) cyclisation dudit composé de formule IV pour former un composé de formule V ;
(iv) réaction dudit composé de formule V avec une amine de formule VI pour obtenir un composé de formule VII.

22. Combinaison comprenant un composé selon l'une quelconque des revendications 1 à 14 et un autre agent thérapeutique.

23. Composition pharmaceutique selon la revendication 15 qui comprend en outre un deuxième agent thérapeutique.
